# EUROPÄISCHE PATENTSCHRIFT

(11) **EP 1 720 844 B1**
(45) Veröffentlichungstag und Bekanntmachung des Hinweises auf die Patenterteilung: **29.04.2009**
(21) Anmeldenummer: 04718299.3
(22) Anmeldetag: 08.03.2004
(51) Int. Cl.: C07D 265/32

(54) **PYRROLIDIN-1,2-DICARBONSÄURE-1-(PHENYLAMID)-2-(4-(3-OXO-MORPHOLIN-4-YL)-PHENYLAMID) DERIVATE UND VERWANDTE VERBINDUNGEN ALS INHIBITOREN DES KOAGULATIONSFAKTORS XA ZUR BEHANDLUNG VON THROMBOEMBOLISCHEN ERKRANKUNGEN**
PYRROLIDINO-1,2-DICARBOXY-1-(PHENYLAMIDE)-2-(4-(3-OXO-MORPHOLINO-4-YL)-PHENYLAMIDE) DERIVATIVES AND RELATED COMPOUNDS FOR USE AS INHIBITORS OF COAGULATION FACTOR XA IN THE TREATMENT OF THROMBO-EMBOLIC DISEASES
DERIVES PYRROLIDINO-1,2-DICARBOXY-1-(PHENYLAMIDE)-2-(4-(3-OXO-MORPHOLINO-4-YL9-PHENYLAMIDE) ET COMPOSES SIMILAIRES POUR L'UTILISATION COMME INHIBITEURS DU FACTEUR XA POUR LE TRAITEMENT DE MALADIES THROMBOEMBOLIQUES

(30) Priorität: 03.04.2003 DE 10315377; 30.06.2003 DE 10329295; 02.07.2003 US 483897 P
(43) Veröffentlichungstag der Anmeldung: 15.11.2006
(73) Patentinhaber: Merck Patent GmbH, 64293 Darmstadt (DE)
(72) Erfinder: TSAKLAKIDIS, Christos, 69469 Weinheim (DE); DORSCH, Dieter, 64372 Ober-Ramstadt (DE); MEDERSKI, Werner, 64673 Zwingenberg (DE); CEZANNE, Bertram, 64546 Mörfelden-Walldorf (DE); GLEITZ, Johannes, 64289 Darmstadt (DE)
(86) Internationale Anmeldenummer: PCT/EP2004/002350
(87) Internationale Veröffentlichungsnummer: WO 2004/087646

(56) Entgegenhaltungen:
- WO-A-00/39118
- WO-A-00/71516
- WO-A-01/64642
- WO-A-02/14308
- WO-A-02/22575
- WO-A-02/48099
- WO-A-95/23609
- WO-A-02/057236
- WO-A-02/074735
- WO-A-02/083624
- WO-A-03/045912
- WO-A-03/050088
- GB-A- 1 503 244
- US-A- 5 691 356
- DATABASE BEILSTEIN BEILSTEIN INSTITUTE FOR ORGANIC CHEMISTRY, FRANKFURT-MAIN, DE; 27. Juni 1988 (1988-06-27), XP002282645 Database accession no. BRN 88135 & HAMILTON: J. BIOL. CHEM., Bd. 198, 1952, Seite 587,
- DATABASE BEILSTEIN BEILSTEIN INSTITUTE FOR ORGANIC CHEMISTRY, FRANKFURT-MAIN, DE; 27. Juni 1988 (1988-06-27), XP002282646 Database accession no. BRN 102211 & FISCHER: CHEM. BER., Bd. 34, 1901, Seite 452,
- DATABASE BEILSTEIN BEILSTEIN INSTITUTE FOR ORGANIC CHEMISTRY, FRANKFURT-MAIN, DE; 19. März 1991 (1991-03-19), XP002282647 Database accession no. BRN 3971830 & MORTIMER: J. CHEM. SOC., 1961, Seiten 189-201,
- DATABASE BEILSTEIN BEILSTEIN INSTITUTE FOR ORGANIC CHEMISTRY, FRANKFURT-MAIN, DE; 9. August 1996 (1996-08-09), XP002283422 Database accession no. BRN 7437586 & GLASS ET AL: ARCH. PHARM., Bd. 328, Nr. 10, 1995, Seiten 709-719,

## Beschreibung

Die Erfindung betrifft Verbindungen der Formel I 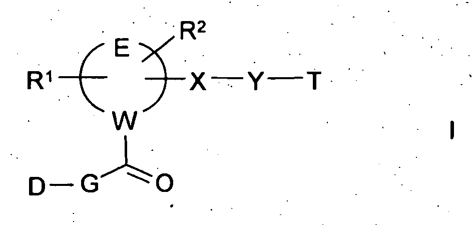 worin
- D: ein- oder zweifach durch Hal substituiertes Phenyl, Pyridyl oder Thienyl,
- R¹: H, =O, COOR³, OH, OA, NH₂, Alkyl mit 1,2, 3, 4, 5 oder 6 C-Atomen, N₃, Ethinyl, Vinyl, Allyloxy, -OCOR³, NHCOA oder NHSO₂A,
- R²: H, =O, OH, OA oder Alkyl mit 1, 2, 3, 4, 5 oder 6 C-Atomen,
- R¹ und R²: zusammen auch einen spirocyclisch gebundenen 3- bis 6-gliedrigen Carbocyclus,
- R³: H oder A,
- R⁴: H oder A, 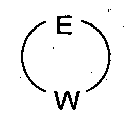 Pyrrolidin-1,2-diyl, Piperidin-1,2-diyl, Oxazolidin-3,4- oder 3,5-diyl, Thiazolidin-3,4-diyl, 2,5-Dihydro-1*H*-pyrrol-1,5-diyl, [1,3]-Dioxolan-4,5-diyl, [1,3]-Oxazinan-3,4-diyl, Piperazin-1,4-diyl, Tetrahydrofuran-3,4-diyl oder Azetidin-1,2-diyl,
- G: (CH₂)ₙ oder (CH₂)ₙNH-,
- X: CONH,
- Y: unsubstituiertes oder ein- oder zweifach durch Methyl, Trifluormethyl, Ethyl, Propyl, Cl oder F substituiertes 1,3-oder 1,4-Phenylen,
- T: ein- oder zweifach durch Carbonylsauerstoff substituiertes Morpholin-4-yl,
- A: unverzweigtes oder verzweigtes Alkyl mit 1-10 C-Atomen und worin 1-7 H-Atome durch F ersetzt sein können,
- Hal: F, Cl, Br oder I,
- n: 0, 1 oder 2,
bedeuten,
sowie ihre pharmazeutisch verwendbaren Solvate, Salze und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren Faktor VIIa, Faktor IXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515, WO 00/71516 oder WO 02/22575 bekannt. Cyclische Guanidine zur Behandlung thromboembolischer Erkrankungen bzw. der kongestiven Herzinsuffizienz sind z.B. in der WO 97/08165 sowie von Glaß et al. in Arch. Pharm. 1995, 328, 709-719, beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.
Andere Carbonsäureamidderivate sind aus WO 02/48099, WO 02/57236, WO 01/64642, WO 95/23609, WO 03/050088 sowie von Emil Fischer in Chem. Ber. 1901, 34, 433-454, bekannt, andere Pyrrolidinderivate sind in WO 02/100830, WO 02/14308 sowie von Paul B. Hamilton in JBC 1952, 198, 587-597, beschrieben.

Weitere heterocyclische Derivate kennt man aus der WO 03/045912, US 5,691,356, WO 00/39118, WO 03/045912 sowie von Lewis & Mortimer in J Chem. Soc. 1961, 189-201.

WO 02/074735 lehrt Biurethanderivate als Inhibitoren des Koagulationsfaktors Xa, die zur Prophylaxe und Therapie von tromboembolischen Erkrankungen und zur Behandlung von Tumoren eingesetzt werden können.
WO 02/083624 offenbart 3,4-disubstituierte Cyclobuten-1,2-Dione für die Behandlung von Erkrankungen, die durch Chemokine vermittelt werden.
Der Stand der Technik beinhaltet auch 1,5-Alkylen-3-Aryl-Hydantoin-Derivate als Herbizide (GB 1503244).

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor VIIa, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in Circulation 1996, 94, 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und anti-thrombotischen Aktivität kann nach üblichen in vitro- oder in vivo-Methoden ermittelt werden. Ein geeignetes Verfahren wird z.B. von J. Hauptmann et al. in Thrombosis and Haemostasis 1990, 63, 220-223 beschrieben.

Die Messung der Inhibierung von Faktor Xa kann z.B. nach der Methode von T. Hara et al. in Thromb. Haemostas. 1994, 71, 314-319 erfolgen.

Der Gerinnungsfaktor VIIa initiiert nach Bindung an Tissue Faktor den extrinsischen Teil der Gerinnungskaskade und trägt zur Aktivierung des Faktors X zu Faktor Xa bei. Eine Inhibierung von Faktor VIIa verhindert somit die Entstehung des Faktors Xa und damit eine nachfolgende Thrombinbildung.
Die Inhibierung des Faktors VIIa durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIa wird z.B. von H. F. Ronning et al. in Thrombosis Research 1996, 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in Journal of Biological Chemistry 1998, 273, 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.
Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo*, oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel I nach den Ansprüchen 1-16 sowie ihrer pharmazeutisch verwendbaren Solvate, Salze und Stereoisomere, **dadurch gekennzeichnet, daß** man
a) zur Herstellung von Verbindungen der Formel I, worin
   - W: N und
   - G: NH bedeuten,
eine Verbindung der Formel II 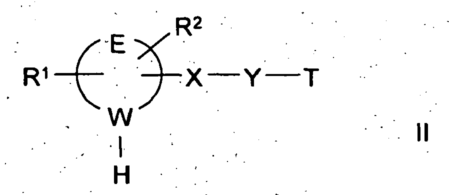 worin
R¹, R², E, X, Y und T die in Anspruch 1 angegebene Bedeutung haben, und W N bedeutet,
mit einer Verbindung der Formel III

D-N=C=O III

worin
D die in Anspruch 1 angegebene Bedeutung hat,
umsetzt,
und/oder
eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.
Vor- und nachstehend haben die Reste bzw. Parameter D, E, G, W, X, Y, T, H¹ und R² die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1- , 2- , 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cyclopentyl, Cyclohexyl oder Cycloheptyl.
Alkylen bedeutet vorzugsweise Methylen, Ethylen, Propylen, Butylen, Pentylen oder Hexylen, ferner verzweigtes Alkylen.

R¹ bedeutet bevorzugt H, =O, COOR³, wie z.B. COOA, OH, OA, NH₂, Alkyl mit 1, 2, 3, 4, 5 oder 6 C-Atomen, N₃, Ethinyl, Vinyl, Allyloxy, -OCOR³, wie z.B. Methylcarbonyloxy, NHCOA, wie z.B. Acetamino, oder NHSO₂A, wie z.B. Methylsulfonylamino; OCH₂COOA wie z.B. OCH₂COOCH₃; oder OCH₂COOH
R² bedeutet vorzugsweise H, =O, OH, OA, wie z.B. Methoxy, oder Alkyl mit 1, 2, 3, 4, 5 oder 6 C-Atomen.

R¹ und R² bedeuten zusammen insbesondere einen an das 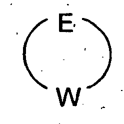 -Ringsystem spirocyclisch gebundenen
3- bis 6-gliedrigen Carbocyclus. Dabei bedeutet der 3- bis 6-gliedrige Carbocyclus bevorzugt Cyclopropyl, Cyclobutyl, Cyclopentyl oder Cyclohexyl.
R³ bedeutet vorzugsweise H oder A, ferner auch Phenyl, Benzyl oder [C(R⁴)₂]ₙCOOA, wie z.B. CH₂COOCH₃.
R⁴ bedeutet vorzugsweise H oder A, ganz besonders bevorzugt H. COR², COR³ bzw. COR⁴ bedeutet z.B. CHO oder -COA.
-COA (Acyl) bedeutet vorzugsweise Acetyl, Propionyl, ferner auch Butyryl, Pentanoyl, Hexanoyl oder z.B. Benzoyl.
Hal bedeutet vorzugsweise F, Cl oder Br, aber auch I.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p- (N-Methylaminocarbonyl)-phehyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Phenoxyphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5-oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise z.B. unsubstituiertes oder ein-, zwei- oder dreifach durch Hal, A, OR², OR³, SO₂A, COOR² oder CN substituiertes Phenyl.

Ar bedeutet insbesondere bevorzugt z.B. unsubstituiertes oder ein- oder zweifach durch Hal, A, OA, Phenoxy, SO₂A, SO₂NH₂, COOR² oder CN substituiertes Phenyl, wie z.B. Phenyl, 2-Methylsulfonylphenyl, 2-Aminosulfonylphenyl, Phenoxyphenyl, 2-, 3- oder 4-Chlorphenyl, 3,4-Dichlorphenyl, 4-Methylphenyl, 4-Bromphenyl, 3-Fluor-4-methoxyphenyl, 4-Trifluormethoxyphenyl, 4-Ethoxyphenyl, 2-Methoxyphenyl, 3-Cyanphenyl, 4-Ethoxycarbonylphenyl, Methoxycarbonylphenyl, Carboxyphenyl oder Aminocarbonylphenyl.
Ganz besonders bevorzugt bedeutet Ar unsubstituiertes Phenyl, 4-Chlorphenyl oder 2-Methylsulfonylphenyl.

G bedeutet besonders bevorzugt (CH₂)ₙ, (CH₂)ₙNH-, -CH=CH- oder -CH=CH-CH=CH-.

X bedeutet besonders bevorzugt -CONH- oder -CON(CH₂COOA)-.

Y bedeutet vorzugsweise Cycloalkylen, Het-diyl oder Ar-diyl, besonders bevorzugt unsubstituiertes oder ein- oder zweifach durch A, OA, Cl, F, COOCH₃, COOH, Phenoxy oder Aminocarbonyl substituiertes 1,4-Phenylen, ferner auch Pyridin-diyl, vorzugsweise Pyridin-2,5-diyl; Piperidin-diyl oder Cyclohexylen.
Y bedeutet insbesondere Pyridin-diyl, Piperidin-diyl, Cyclohexylen oder unsubstituiertes oder ein- oder zweifach durch A, OA, Cl, F, COOCH₃, COOH, Phenoxy oder Aminocarbonyl substituiertes Phenylen.

Het bedeutet z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5- pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het' bedeutet vorzugsweise z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1-3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het' kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5- pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3-oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-; -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

T bedeutet besonders bevorzugt ein- oder zweifach durch =O substituiertes Morpholin-4-yl, wobei die Reste auch ein- oder zweifach durch Hal, A und/oder OA substituiert sein können;
ganz besonders bevorzugt ist 3-Oxo-morpholin-4-yl.

D bedeutet vorzugsweise ein- oder zweifach durch Hal substituiertes Phenyl, Thienyl, Pyridyl, Furyl, Thiazolyl, Pyrrolyl oder Imidazolyl, besonders bevorzugt ein- oder zweifach durch Hal substituiertes Phenyl, Pyridyl, Thienyl, Furyl oder Imidazolyl.

Der Rest 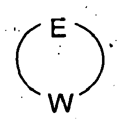 bedeutet vorzugsweise Pyrrolidin-1,2-diyl, Piperidin-1,2-diyl, Piperidin-1,3-diyl, Oxazolidin-3,4- oder 3,5-diyl, Thiazolidin-3,4-diyl, 2,5-Dihydro-1H-pyrrol-1,5-diyl, [1,3]-Dioxolan-4,5-diyl, [1,3]-Oxazinan-3,4-diyl, Piperazin-1 ,4-diyl, Tetrahydrofuran-3,4-diyl oder Azetidin-1,2-diyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgende Teilformel ausgedrückt werden, die der Formel I entspricht und worin die nicht näher bezeichneten Reste die bei der Formel I angegebene Bedeutung haben, worin jedoch
- D: ein- oder zweifach durch Hal substituiertes Phenyl, Pyridyl oder Thienyl,
- R¹: H, =O, COOR³, OH, OA, NH₂, Alkyl mit 1, 2, 3, 4, 5 oder 6 C-Atomen, N3, Ethinyl, Vinyl, Allyloxy, -OCOR³, NHCOA oder NHSO₂A,
- R²: H, =O, OH, OA oder Alkyl mit 1, 2, 3, 4, 5 oder 6 C-Atomen,
- R¹ und R²: zusammen auch einen spirocyclisch gebundenen 3- bis 6-gliedrigen Carbocyclus,
- R³: H oder A,
- R⁴: H oder A, 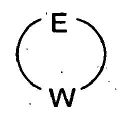 Pyrrolidin-1,2-diyl, Piperidin-1,2-diyl, Oxazolidin-3,4- oder 3,5-diyl, Thiazolidin-3,4-diyl, 2,5-Dihydro-1*H*-pyrrol-1,5-diyl, [1,3]-Dioxolan-4,5-diyl, [1,3]-Oxazinan-3,4-diyl, Piperazin-1,4-diyl, Tetrahydrofuran-3,4-diyl oder Azetidin-1,2-diyl,
- G: (CH₂)ₙ oder (CH₂)ₙNH-,
- X: CONH,
- Y: unsubstituiertes oder ein- oder zweifach durch Methyl, Trifluormethyl, Ethyl, Propyl, Cl oder F substituiertes 1,3-oder 1,4-Phenylen,
- T: ein- oder zweifach durch Carbonylsauerstoff substituiertes Morpholin-4-yl,
- A: unverzweigtes oder verzweigtes Alkyl min 1-10 C-Atomen und worin 1-7 H-Atome durch F ersetzt sein können,
- Hal: F, Cl, Br oder I,
- n: 0, 1 oder 2,
bedeuten,
sowie ihre pharmazeutisch verwendbaren Solvate, Salze und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II, III, sind in der Regel bekannt. Sind sie neu, so können sie aber nach an sich bekannten Methoden hergestellt werden.

Verbindungen der Formel I können vorzugsweise erhalten werden, indem man Verbindungen der Formel II mit Verbindungen der Formel III umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Phenolkomponente der Formel II bzw. des Alkylierungsderivates der Formel III kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können weiterhin vorzugsweise erhalten werden, indem man eine Verbindung der Formel D-NH₂, worin D die in Anspruch 1 angegebene Bedeutung hat, mit einem Chloroformiatderivat, z.B. 4-Nitrophenylchlorformiat zu einem intermediären Carbamat umsetzt, und dieses anschließend mit einer Verbindung der Formel II umsetzt. Dies geschieht unter Bedingungen wie oben beschrieben.

Verbindungen der Formel I können ferner erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-Iodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppe ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden; die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem träger wie Kohle) abgespalten werden. Als Lösungsmittel eignen sich dabei die oben angegebene, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Gegenstand der Erfindung sind insbesondere auch die Zwischenverbindungen
ausgewählt aus der Gruppe (S)-Pyrrolidin-2-carbonsäure-*N*-[4-(3-oxo-morpholin-4-yl)-phenyl]-amid,
(R)-Pyrrolidin-2-carbonsäure-*N*-[4-(3-oxo-morpholin-4-yl)-phenyl]-amid,
(2R,4R)-4-Hydroxy-pyrrolidin-2-carbonsäure-*N*-[4-(3-oxo-morpholin-4-yl)-phenyl]-amid,
4-Hydroxy-pyrrolidin-2-carbonsäure-*N*-[4-(3-oxo-morpholin-4-yl)-phenyl]-amid,
(R)-4,4-Dimethoxy-pyrrolidin-2-carbonsäure-*N*-[4-(3-oxo-morpholin-4-yl)-phenyl]-amid,
(2R,4R)-4-Methoxy-pyrrolidin-2-carbonsäure-*N*-[4-(3-oxo-morpholin-4-yl)-phenyl]-amid,
sowie deren Isomere und Salze.
Die Herstellung ist beschrieben z.B. in Beispiel 1 und 7.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I, und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen verwendet werden.

Dabei werden die efindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I, und/oder ihre pharmazeutisch verwendbaren Solvate, Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I, und/oder ihrer pharmazeutisch verwendbaren Solvate, Salze und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I, und/oder ihrer pharmazeutisch verwendbaren Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

Gegenstand der Erfindung ist ferner die Verwendung von Verbindungen der Formel I, und/oder ihrer pharmazeutisch verwendbaren Solvate, Salze und Stereoisomere, einschließlich deren Mischungen in allen Verhältnissen, zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit mindestens einem weiteren Arzneimittelwirkstoff.

Gegenstand der Erfindung ist ferner ein Arzneimittel enthaltend (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} und/oder seine pharmazeutisch verwendbaren Solvate, Salze und Stereoisomere, einschließlich deren Mischungen in allen Verhältnissen, und Aspirin.

Gegenstand der Erfindung ist ferner die Verwendung von (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} und/oder seine pharmazeutisch verwendbaren Solvate, Salze und Stereoisomere, einschließlich deren Mischungen in allen Verhältnissen, zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit Aspirin.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): EI (Elektronenstoß-lonisation) M⁺
FAB (Fast Atom Bombardment) (M+H)⁺
ESI (Electrospray lonization) (M+H)⁺ (wenn nichts anderes angegeben)

### Beispiel 1

Die Herstellung von (R)-Pyrrolidin-1,2-dicarbonsäure-1-[(4-chlor-phenyl)-amid]-2-{[4-(3-oxo-morpholin-4-yl)-plenyl]-amid} ("A1") erfolgt analog nachstehendem Schema: 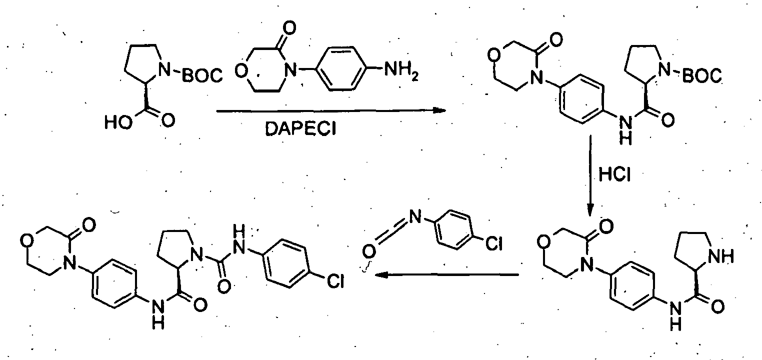
1.1 Eine Lösung von 1.0 g (5.2 mMol) 4-(4-Amino-phenyl)-morpholin-3-on in 25 ml Dimethylformamid wird nacheinander mit 0.8 g (5.2 mMol) 1-Hydroxybenzotriazolhydrat, 1.12 g (5.2 mmol) D-Boc-Prolin, 2 g (10.4 mmol) N-(3-Dimethylaminopropyl)-N'-ethylcarbodiimidhydrochlorid (DAPECI) und 1.26 ml N-Methylmorpholin versetzt und die so erhaltene Lösung 12 Stunden bei Raumtemperatur gerührt. Anschließend wird die Reaktionslösung im Vakuum zu Trockne eingedampft, der Rückstand in 10 ml 5%ige Natriumhydrogencarbonatlösung aufgenommen und die Natriumhydrogebcarbonatlösung zwei mal mit je 10 ml Essigsäureethylester extrahiert. Nach dem Trocknen der vereinigten organischen Phasen über Natriumsulfat und Abziehen des Lösungsmittels wird der feste Rückstand mit 20 ml Diethylether verrieben. Man erhält so 1.4 g 2-[4-(3-Oxo-morpholin-4-yl)-phenylcarbamoyl]-pyrrolidin-1-carbonsäure-*tert*-butylester als weißes Pulver; ESI 390.
1.2 Eine Lösung von 1.4 g (3.60 mMol) 2-[4-(3-Oxo-morpholin-4-yl)-phenylcarbamoyl]-pyrrolidin-1-carbonsäure-*tert*-butylester in 20 ml Dioxan wird mit 40 ml 4N Salzsäure in Dioxan versetzt und 12 Stunden bei Raumtemperatur gerührt. Anschließend wird der ausgefallene Niederschlag abgesaugt und nacheinander mit je 10 ml Dioxan und Diethylether gewaschen und im Vakuum getrocknet. Man erhält so 1.1 g Pyrrolidin-2-carbonsäure-*N*-[4-(3-oxo-morpholin-4-yl)-phenyl]-amid-Hydrochlorid als weißes Pulver; ESI 290.
1.3 Eine Lösung von 200 mg (0.61 mMol) Pyrrolidin-2-carbonsäure-*N*-[4-(3-oxo-morpholin-4-yl)-phenyl]-amid-Hydrochlorid und 1 ml Triethylamin in 5 ml Methylenchlorid wird mit 95 mg (0.61 mmol) 4-Chlorphenylisocyanat versetzt und die Reaktionslösung zwei Stunden bei Raumtemperatur gerührt. Anschließend wird die Reaktionslösung mit je 5 ml 1N Salzsäure und Wasser gewaschen und die Methylenchloridlösung über Natriumsulfat getrocknet. Nach Abziehen des Lösungsmittels im Vakuum wird das Rohprodukt aus Ethanol/Diethylether umkristallisiert. Man erhält so 120 mg der Titelverbindung ("A1") als weißes Pulver; ESI 443; F. 227.6°.

Analog erhält man die nachstehenden Verbindungen
(R)-Pyrrolidin-1,2-dicarbonsäure-1-[(4-chlor-phenyl)-amid]-2-{[3-methyl-4-(3-oxo-morpholin-4-yl)-phenyl]-amid}, ESI 457, F. 147° (Zersetzung);
(R)-Pyrrolidin-1,2-dicarbonsäure-1-[(4-chlor-phenyl)-amid]-2-{[3-fluor-4-(3-oxo-morpholin-4-yl)-phenyl]-amid}, ESI 461, F. 155°;
(R)-Pyrrolidin-1,2-dicarbonsäure-1-[(4-chlor-phenyl)-amid]-2-{[2-fluor-4-(3-oxo-morpholin-4-yl)-phenyl]-amid}, ESI 461;
(R)-Pyrrolidin-1,2-dicarbonsäure-1-[(4-chlor-phenyl)-amid]-2-{[3-trifluormethyl-4-(3-oxo-morpholin-4-yl)-phenyl]-amid}, ESI 511, F. 147°;
(R)-Piperidin-1,2-dicarbonsäure-1-[(4-chlor-phenyl)-amid]-2-{[3-methyl-4-(3-oxo-morpholin-4-yl)-phenyl]-amid}, ESI 471, F. 140°;
(S)-Pyrrolidin-1,2-dicarbonsäure-1-[(4-chlor-phenyl)-amid]-2-{[3-methyl-4-(3-oxo-morpholin-4-yl)-phenyl]-amid}, ESI 457; F. 174°.

### Beispiel 1a

### (R)-1-(5-Chlor-thiophen-2-carbonyl)-pyrrolidin-2-carbonsäure-N-[4-(3-oxo-morpholin-4-yl)-phenyl]-amid ("AB1")

Eine Lösung von 1.35 g (4.66 mmol) Pyrrolidin-2-carbonsäure-[4-(3-oxo-morpholin-4-yl)-phenyl]-amid in 30 ml Dimethylformamid wird nacheinander mit 0.71 g (4.66 mmol) 1-Hydroxybenzotriazolhydrat, 0.76 g (4.66 mmol) 5-Chlor-thiophencarbonsäure, 1.79 g (9.33 mmol) N-(3-Dimethylaminopropyl)-N'-ethylcarbodiimidhydrochlorid (DAPECI) und 1.13 ml N-Methylmorpholin versetzt und die so erhaltene Lösung 12 Stunden bei Raumtemperatur gerührt. Anschließend wird die Reaktionslösung im Vakuum zu Trockne eingedampft, der Rückstand in 10 ml 5%ige Natriumhydrogencarbonatlösung aufgenommen und die Natriumhydrogencarbonatlösung zweimal mit je 10 ml Essigsäureethylester extrahiert. Nach dem Trocknen der vereinigten organischen Phasen über Natriumsulfat und Abziehen des Lösungsmittels wird der feste Rückstand mit 20 ml Diethylether verrieben. Man erhält so 1.2 g (59.4%) "AB1", ESI 434; F. 195°.

Analog erhält man die Verbindung
(R)-1-(5-Chlor-thiophen-2-carbonyl)-pyrrolidin-2-carbonsäure-*N*-[3-methyl-4-(3-oxo-morpholin-4-yl)-phenyl]-amid, ESI 448; F. 113° (Zersetzung).

### Beispiel 1b

Die Herstellung von (R)-2,5-Dihydro-pyrrol-1,2-dicarbonsäure-1-[(4-chlorphenyl)-amid]-2-{[4-(3-oxo-morpholin-4-yl)-phenyl]-amid} erfolgt wie folgt 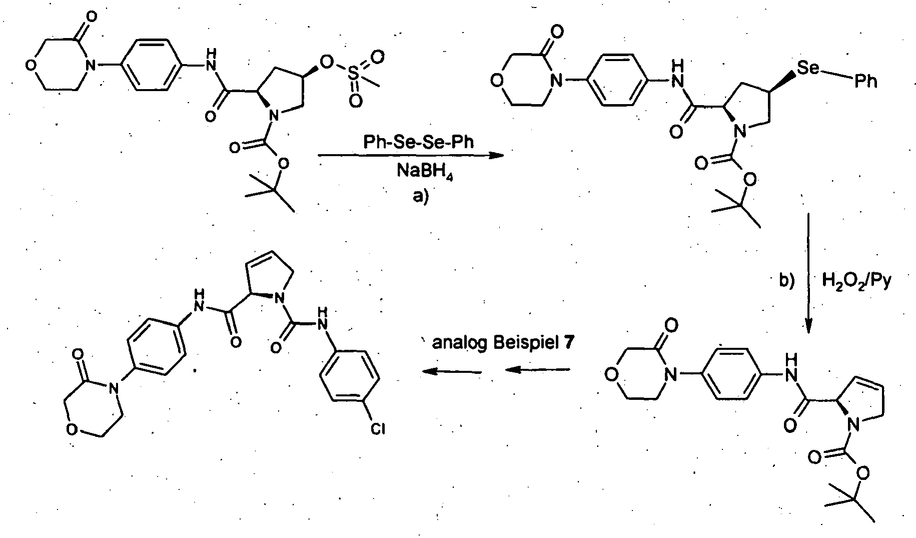
a) Die Suspension von 0.82 g (2.63 mmol) Diphenyldiselenid in 12 ml tert.-Butanol wird unter Stickstoff mit 0,19 g (5.1 mMol) Natriumborhydrid (NaBH₄) versetzt und die Reaktionsmischung ca. eine Stunde am Rückfluß erhitzt, bis die gelbe Reaktionslösung farblos wird. Anschliessend tropft man bei dieser Temperatur die Lösung von1.99 g (4.11 mMol) (2R,4R)-4-Methansulfonyloxy-2-[4-(3-oxo-morpholin-4-yl)-phenylcarbamoyl]-pyrrolidin-1-carbonsäure-*tert*-butylester (s. Beispiel 9.1) in 12 ml tert.-Butanol zu und lässt dann die Reaktionsmischung 12 Stunden am Rückfluß unter Rühren kochen. Nach dem Abkühlen der Reaktionsmischung wird das Lösungsmittel im Vakuum abgezogen, der Rückstand in 20 ml Essigsäureethylester aufgenommen und die so erhaltene Lösung mit 20 ml Wasser gewaschen. Nach dem Trocknen der Essigsäureethylester-Phase über Natriumsulfat und Abziehen des Lösungsmittels erhält man 1.82 g (81.3%) (1 R,4R)-2-[4-(3-Oxo-morpholin-4-yl)-phenylcarbamoyl]-4-phenylselanyl-pyrrolidin-1-carbonsäure-*tert*-butylester, ESI 545.
b) Die Lösung von 1,72 g (3.16 mMol) der unter a) hergestellten Selenverbindung und 0.4 ml Pyridin in 25 ml Methylenchlorid wird bei 0°C mit 1 ml 30%iges Wasserstoffperoxid (H₂O₂) tropfenweise versetzt. Anschliessend lässt man die Reaktionsmischung innerhalb von zwei Stunden auf Raumtemperatur kommen, versetzt sie dann mit 10 ml 5%iger Kaliumhydrogensulfatlösung, trennt die Phasen ab und wäscht die organische Phase mit 10 ml gesättigter Natriumhydrogencarbonatlösung. Nach dem Trocknen der organischen Phase über Natriumsulfat und Abziehen des Lösungsmittels wird der Rückstand an Kieselgel chromatographiert. Man erhält man so 0.73 g (59.7%) (R)-2-[4-(3-Oxo-morpholin-4-yl)-phenylcarbamoyl]-2,5-dihydro-pyrrol-1-carbonsäure-*tert*.-butylester, ESI 388.

Die weitere Umsetzung erfolgt analog Beispiel 7.
Man erhält (R)-2,5-Dihydro-pyrrol-1,2-dicarbonsäure-1-[(4-chlor-phenyl)-amid]-2-{[4-(3-oxo-morpholin-4-yl)-phenyl]-amid}, ESI 441, F. 245°.

Analog erhält man die nachstehenden Verbindungen
(R)-2,5-Dihydro-pyrrol-1,2-dicarbonsäure-1-[(4-chlor-phenyl)-amid]-2-{[3-fluor-4-(3-oxo-morpholin-4-yl)-phenyl]-amid},
(R)-2,5-Dihydro-pyrrol-1,2-dicarbonsäure-1-[(4-chlor-phenyl)-amid]-2-{[2-fluor-4-(3-oxo-morpholin-4-yl)-phenyl]-amid}.

### Beispiel 2

Die Herstellung von (R)-Oxazolidin-3,4-dicarbonsäure-3-[(4-chlorphenyl)-amid]-4-{[4-(3-oxo-morpholin-4-yl)-phenyl]-amid} ("A2") erfolgt analog nachstehendem Schema: 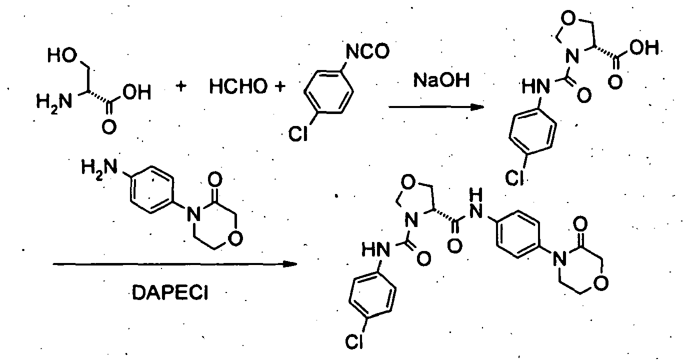
2.1 Eine Lösung von 2.10 g (20.0 mmol) D-Serin in 10 ml 1N wässriger Natronlauge wird mit 1.49 ml (20.0 mmol) 37%iger wässriger Formaldehydlösung versetzt. Die entstandene Lösung wird 18 Stunden bei 5 °C belassen. Die Lösung wird auf 80 °C erhitzt, 6.14 g (40 mmol) 4-Chlorphenylisocyanat zugegeben und eine Stunde bei dieser Temperatur gerührt. Man lässt abkühlen und filtriert den entstandenen Niederschlag ab. Das Filtrat wird mit 1 N HCl angesäuert und der entstandene Niederschlag abfiltriert und getrocknet: (R)-3-(4-Chlorphenylcarbamoyl)-oxazolidin-4-carbonsäure als farbloser Feststoff; ESI 271.
2.2 Eine Lösung von 541 mg (2.00 mmol) (R)-3-(4-Chlorphenylcarbamoyl)-oxazolidin-4-carbonsäure und 384 mg (2.00 mmol) 4-(4-Amino-phenyl)-morpholin-3-on in 4 ml Dimethylformamid (DMF) wird mit 498 mg (2.60 mmol) *N*-(3-Dimethylaminopropyl)-*N'*-ethylcarbodiimidhydrochlorid (DAPECI) versetzt und 18 Stunden bei Raumtemperatur gerührt. Das Reaktionsgemisch wird auf gesättigte Natriumhydrogencarbonatlösung gegeben und der entstandene Niederschlag abfiltriert: (R)-Oxazolidin-3,4-dicarbonsäure-3-[(4-chlor-phenyl)-amid]-4-{[4-(3-oxo-morpholin-4-yl)-phenyl]-amid} ("A2") als farbloser Feststoff; ESI 461.

Analog erhält man die nachstehenden Verbindungen
(R)-Oxazolidin-3,4-dicarbonsäure-3-[(4-chlor-phenyl)-amid]-4-{[3-methyl-4-(3-oxo-morpholin-4-yl)-phenyl]-amid}, ESI 459;
(4R,5S)- 5-Methyl-oxazolidin-3,4-dicarbonsäure-3-[(4-chlor-phenyl)-amid]-4-{[4-(3-oxo-morpholin-4-yl)-phenyl]-amid}, ESI 459;
(4R,5S)-5-Methyl-oxazolidin-3,4-dicarbonsäure-3-[(4-chlor-phenyl)-amid-4-{[3-methyl-4-(3-oxo-morpholin-4-yl)-phenyl]-amid}, ESI 473;
(4R,5S)-5-Methyl-oxazolidin-3,4-dicarbonsäure-3-[(4-chlor-phenyl)-amid]-4-{[3-fluor-4-(3-oxo-morpholin-4-yl)-phenyl]-amid}, ESI 477;
(4R,5S)-5-Methyl-oxazolidin-3,4-dicarbonsäure-3-[(4-chlor-phenyl)-amid]-4-{[3-chlor-4-(3-oxo-morpholin-4-yl)-phenyl]-amid}, ESI 477;
(4R,5R)-5-Methyl-oxazolidin-3,4-dicarbonsäure-3-[(4-chlor-phenyl)-amid]-4-{[3-methyl-4-(3-oxo-morpholin-4-yl)-phenyl]-amid}, ESI 473.

### Beispiel 2a

Analog Beispiel 2 erhält man, ausgehend von (R)-Cleonin 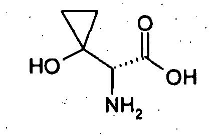 die nachstehende Verbindung
4-Oxa-6-aza-spiro[2.4]heptan-6,7-dicarbonsäure-6-[(4-chlorphenyl)-amid]-7-{[4-(3-oxo-morpholin-4-yl)-phenyl]-amid}

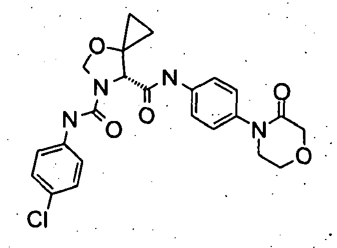

### Beispiel 3

Die Herstellung von (S)-Thiazolidin-3,4-dicarbonsäure-3-[(4-chlorphenyl)-amid]-4-{[4-(3-oxo-morpholin-4-yl)-phenyl]-amid} ("A3") und (S)-1,1-Dioxo-1λ⁶-thiazolidin-3,4-dicarbonsäure-3-[(4-chlor-phenyl)-amid] 4-{[4-(3-oxo-morpholin-4-yl)-phenyl]-amid} ("A4") erfolgt analog nachstehendem Schema: 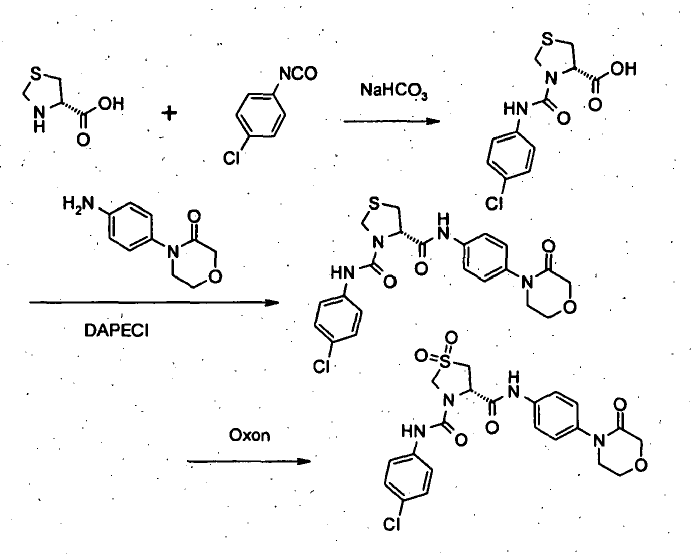
3.1 Eine Lösung von 4.54 g (54.0 mmol) Natriumhydrogencarbonat und 3.60 g (27.0 mmol) 2-(S)-Thiazolidin-4-carbonsäure in 50 ml Wasser wird auf 80° C erhitzt und 8.46 g (54.0 mmol) 4-Chlorphenylisocyanat zugegeben. Das Reaktionsgemisch wird 1 Stunde bei dieser Temperatur gerührt. Man lässt abkühlen und filtriert den entstandenen Niederschlag ab. Das Filtrat wird mit 1 N HCl angesäuert und der entstandene Niederschlag abfiltriert und getrocknet: (S)-3-(4-Chlorphenylcarbamoyl)-thiazolidin-4-carbonsäure als farbloser Feststoff; ESI 287.
3.2 Eine Lösung von 573 mg (2.00 mmol) (S)-3-(4-Chlorphenylcarbamoyl)-thiazolidin-4-carbonsäure und 384 mg (2.00 mmol) 4-(4-Amino-phenyl)-morpholin-3-on in 4 ml Dimethylformamid (DMF) wird mit 498 mg (2.60 mmol) *N*-(3-Dimethylaminopropyl)-*N'*-ethylcarbodiimidhydrochlorid (DAPECI) versetzt und 18 Stunden bei Raumtemperatur gerührt. Das Reaktionsgemisch wird auf gesättigte Natriumhydrogencarbonatlösung gegeben und der entstandene Niederschlag abfiltriert: (S)-Thiazolidin-3,4-dicarbonsäure-3-[(4-chlorphenyl)-amid]-4-{[4-(3-oxo-morpholin-4-yl)-phenyl]-amid} ("A3") als farbloser Feststoff; ESI 461.
3.3 Eine Suspension von 450 mg (0.976 mmol) "A3" in 50 ml Methanol wird mit einer Lösung von 1.9 g Oxon in 30 ml Wasser versetzt und das Reaktionsgemisch 24 Stunden bei Raumtemperatur gerührt. Das Reaktionsgemisch wird auf Wasser gegeben und der entstandene Niederschlag abfiltriert und getrocknet: (S)-1,1-Dioxo-1λ⁶-thiazolidin-3,4-dicarbonsäure-3-[(4-chlorphenyl)-amid]-4-{[4-(3-oxo-morpholin-4-yl)-phenyl]-amid} ("A4") als farbloser Feststoff; ESI 493.

Analog erhält man die nachstehenden Verbindungen
(S)-Thiazolidin-3,4-dicarbonsäure-3-[(4-chlor-phenyl)-amid]-4-{[3-methyl-4-(3-oxo-morpholin-4-yl)-phenyl]-amid}, ESI 475;
(S)-1,1-Dioxo-1λ⁶-thiazolidin-3,4-dicarbonsäure-3-[(4-chlor-phenyl)-amid] 4-{[3-methyl-4-(3-oxo-morpholin-4-yl)-phenyl]-amid}, ESI 507.

### Beispiel 4

Die Herstellung von 3-(5-Chlorthiophen-2-carbonyl)-oxazolidin-5-carbonsäure-[4-(3-oxo-morpholin-4-yl)-phenyl]-amid ("A5") erfolgt analog nachstehendem Schema: 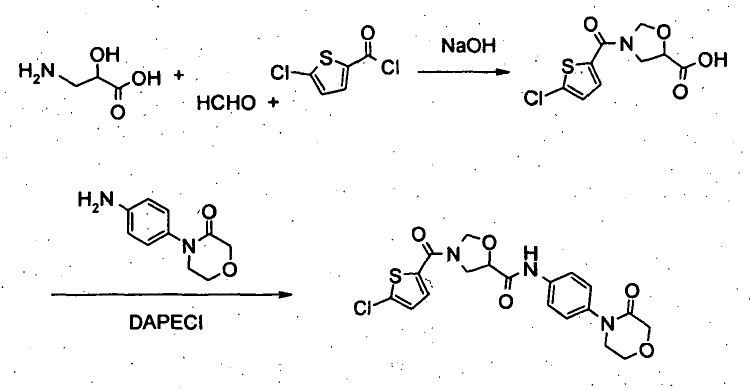
4.1 Eine Lösung von 2.00 g (19.0 mmol) DL-Isoserin in 10 ml 1N wässriger Natronlauge wird mit 1.48 ml (19.9 mmol) 37%iger wässriger Formaldehydlösung versetzt. Die entstandene Lösung wird 18 Stunden bei 5 °C belassen. Zu dieser Lösung wird bei einer Innentemperatur von 0 - 5 °C eine Lösung von 3.46 g (19.1 mmol) 5-Chlorthiophencarbonylchlorid in 10 ml Aceton zugetropft. Während des Zutropfens wird durch Zugabe von festem Natriumhydrogencarbonat der pH auf einen Wert über 7 gehalten. Nach beendeter Zugabe lässt man auf Raumtemperatur erwärmen, gibt Wasser zu und extrahiert mit tert.-Butylmethylether. Die wässrige Phase wird mit 1N HCl angesäuert und mit tert.-Butylmethylether extrahiert. Diese organische Phase wird über Natriumsulfat getrocknet und eingedampft: 3-(5-Chlorthiophen-2-carbonyl)-oxazolidin-5-carbonsäure als farbloser Feststoff; ESI 262.
4.2 Eine Lösung von 500 mg (1.91 mmol) 3-(5-Chlorthiophen-2-carbonyl)-oxazolidin-5-carbonsäure und 367 mg (1.91 mmol) 4-(4-Amino-phenyl)-morpholin₋3-on in 5 ml Dimethylformamid (DMF) wird mit 479 mg (2.50 mmol) *N*-(3-Dimethylaminopropyl)-*N'*-ethylcarbodiimidhydrochlorid (DAPECI) versetzt und 18 Stunden bei Raumtemperatur gerührt. Das Reaktionsgemisch wird auf gesättigte Natriumhydrogencarbonatlösung gegeben und der entstandene Niederschlag abfiltriert: 3-(5-Chlorthiophen-2-carbonyl)-oxazolidin-5-carbonsäure-[4-(3-oxo-morpholin-4-yl)-phenyl]-amid ("A5") als farbloser Feststoff; ESI 436.

Analog erhält man die nachstehenden Verbindungen
3-(5-Chlorthiophen-2-carbonyl)-oxazolidin-5-carbonsäure-[3-methyl-4-(3-oxo-morpholin-4-yl)-phenyl]-amid, ESI 450.

### Beispiel 5

Die Herstellung von (2R,4R)-4-Hydroxypyrrolidin-1,2-dicarbonsäure-1-[(5-chlorpyridin-2-yl)-amid]-2-{[4-(2-oxo-2*H*-pyridin-1-yl)-phenyl]-amid} ("A6") erfolgt analog nachstehendem Schema:

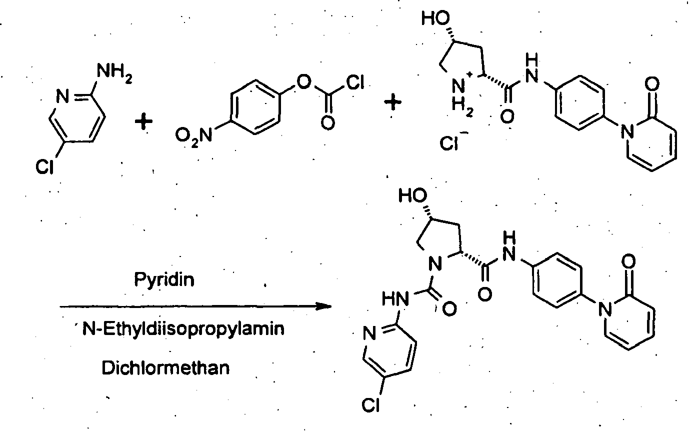

Zu einer Lösung von 570 mg (4.43 mmol) 2-Amino-5-chlorpyridin und 0.73 ml (9.0 mmol) Pyridin in 50 ml Dichlormethan werden 894 mg (4.43 mmol) 4-Nitrophenylchlorformiat gegeben und 1 Stunde bei Raumtemperatur gerührt. Zu der enstandenen Suspension werden 1.49 g (4.43 mmol) (2R,4R)-4-Hydroxy-2-[4₋(2-oxo-2*H*-pyridin-1-yl)-phenylcarbamoyl]-pyrrolidinium-chlorid und 1.5 ml (9.0 mmol) N-Ethyldiisopropylamin gegeben und das Reaktionsgemisch 18 Stunden bei Raumtemperatur gerührt. Das Reaktionsgemisch wird eingedampft und der Rückstand an einer Kieselgelsäule mit Dichlormethan/Methanol 95:5 als Laufmittel chromatographiert: (2R,4R)-4-Hydroxypyrrolidin-1,2-dicarbonsäure-1-[(5-chlorpyridin-2-yl)-amid]-2-{[4-(2-oxo-2*H*-pyridin-1-yl)-phenyl]-amid} ("A6") als farbloser Feststoff, ESI 454.

Analog werden die nachstehenden Verbindungen erhalten
(2R,4R)-4-Hydroxypyrrolidin-1,2-dicarbonsäure-1-[(5-chlorpyridin-2-yl)-amid]-2-{[4-(3-oxo-morpholin-4-yl)-phenyl]-amid}, ESI 460;
(R)-4,4-Dimethoxypyrrolidin-1,2-dicarbonsäure-1-[(5-chlorpyridin-2-yl)-amid]-2-{[4-(3-oxo-morpholin-4-yl)-phenyl]-amid}, ESI 504.

### Beispiel 7

Die Herstellung von (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} ("A8") erfolgt analog nachstehendem Schema: 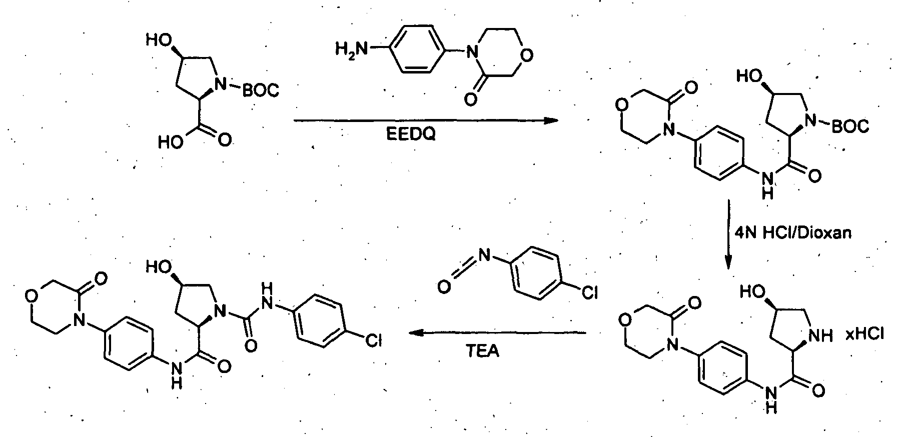
7.1 Eine Suspension von 15 g (64.86 mmol) cis-N-BOC-4-hydroxy-D-Prolin und 12.47 g (64.86 mmol 1-(4-Amino-phenyl)-1*H*-pyridin-2-on in 250 ml Toluol wird mit 16 g (12.86 mmol) Ethyl-2-ethoxy-1,2-dihydrochinolin-1-carboxylat (EEDQ) versetzt und 18 Stunden bei Raumtemperatur gerührt. Anschliessend wird das ausgefallene Produkt abfiltriert, nacheinander mit je 50 ml Toluol und Diethylether gewaschen und im Exsikkator getrocknet. Man erhält so 24.5 g (93.2%) (2R,4R)-4-Hydroxy-2-[4-(3-oxo-morpholin-4-yl)-phenylcarbamoyl]-pyrrolidin-1-carbonsäure-*ter.t*-butylester als grau-weisses Pulver. ESI 406.
7.2 Eine Lösung von 15 g (37 mmol) (2R,4R)-4-Hydroxy-2-[4-(3-oxo-morpholin-4-yl)-phenylcarbamoyl]-pyrrolidin-1-carbonsäure-*tert*.-butylester in 200 ml Dioxan wird mit 300 ml 4N Salzsäure in Dioxan versetzt und 12 Stunden bei Raumtemperatur gerührt. Anschliessend wird der ausgefallene Niederschlag abfiltriert, mit je 50 ml Dioxan und Diethylether gewaschen und im Exsikkator getrocknet. Man erhält so 12.64 g (100%) (2R,4R)-4-Hydroxy-pyrrolidin-2-carbonsäure [4-(3-oxo-morpholin-4-yl)-phenyl]-amid-Hydrochlorid als weißes Pulver. ESI 306.
7.3 12.64 g (36.98 mmol) (2R,4R)-4-Hydroxy-pyrrolidin-2-carbonsäure-[4-(3-oxo-morpholin-4-yl)-phenyl]-amid-Hydrochlorid werden in 1200 ml Dichlomethan suspendiert und unter Eisbadkühlung mit 5.4 ml Triethylamin versetzt. Zu der Mischung tropft man anschließend die Lösung von 5.96 g (38.83 mmol) 4-Chlorphenylisocyanat in 100 ml Dichlometrhan bei 2 °C innerhalb 1.5 Stunden zu, und läßt dann die Reaktionslösung noch weitere 30 min unter Eiskühlung rühren. Danach wird die Dichlormethanlösung nacheinander mit je 100 ml 1N Salzsäure und Wasser gewaschen und über Natriumsulfat getrocknet. Nach Abfiltrieren des Trockenmittels und Einengen der Methylenchloridlösung auf 1/3 des ursprünglichen Volumens am Rotationsverdampfer wird das ausgefallene Produkt abfiltriert, mit 50 ml Petrolether gewaschen und im Exsikkator getrocknet. Man erhält so 14.6 g (86%) (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} ("A8") als weisses Pulver, ESI 459; F. 216°.

Analog werden die nachstehenden Verbindungen erhalten
(2R,4R)-4-Hydroxy-pyrrolidin-1,2-dicarbonsäure-1-[(4-chlorphenyl)-amid]-2-{[3-methyl-4-(3-oxo-morpholin-4-yl)-phenyl]-amid}, ESI 473; F. 250°;
(2R,4R)-4-Hydroxy-pyrrolidin-1,2-dicarbonsäure-1-[(4-chlorphenyl)-amid]-2-{[2-fluor-4-(3-oxo-morpholin-4-yl)-phenyl]-amid}, ESI 477; F. 235°;
(2R,3R)-3-Hydroxy-pyrrolidin-1,2-dicarbonsäure-1-[(4-chlorphenyl)-amid]-2-{[3-fluor-4-(3-oxo-morpholin-4-yl)-phenyl]-amid},
(2R,3S)-3-Hydroxy-pyrrolidin-1,2-dicarbonsäure-1-[(4-chlorphenyl)-amid]-2-{[3-fluor-4-(3-oxo-morpholin-4-yl)-phenyl]-amid},
(2R,4R)-4-Hydroxy-pyrrolidin-1,2-dicarbonsäure-1-[(4-chlorphenyl)-amid]-2-{[3-fluor-4-(3-oxo-morpholin-4-yl)-phenyl]-amid},
(2S,3S)-3-Hydroxy-pyrrolidin-1,2-dicarbonsäure-1-[(4-chlorphenyl)-amid]-2-{[4-(3-oxo-morpholin-4-yl)-phenyl]-amid}, ESI 459;
(2S,4S)-4-Hydroxy-pyrrolidin-1,2-dicarbonsäure-1-[(4-chlorphenyl)-amid]-2-{[4-(3-oxo-morpholin-4-yl)-phenyl]-amid}, ESI 459 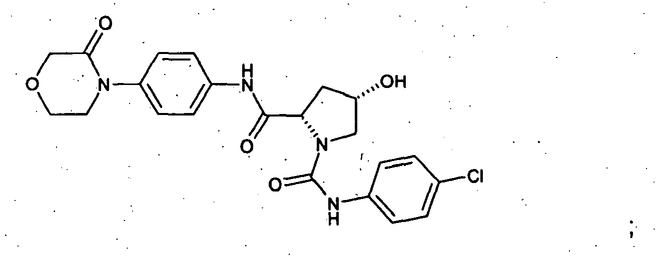 (2R,4R)-3-Hydroxy-pyrrolidin-1,2-dicarbonsäure-1-[(4-chlorphenyl)-amid]-2-{[2-methoxycarbonyl-4-(3-oxo-morpholin-4-yl)-phenyl]-amid}, ESI 517, F. 119; und daraus durch Hydrolyse
(2R,4R)-3-Hydroxy-pyrrolidin-1,2-dicarbonsäure-1-[(4-chlorphenyl)-amid]-2-{[2-carboxy-4-(3-oxo-morpholin-4-yl)-phenyl]-amid}, ESI 503,F.145°,
(2R,4R)-4-Hydroxy-pyrrolidin-1,2-dicarbonsäure-1-[(4-chlorphenyl)-amid]-2-{[2-methoxycarbonyl-4-(3-oxo-morpholin-4-yl)-phenyl]-amid}, und daraus durch Hydrolyse
(2R,4R)-4-Hydroxy-pyrrolidin-1,2-dicarbonsäure-1-[(4-chlorphenyl)-amid]-2-{[2-carboxy-4-(3-oxo-morpholin-4-yl)-phenyl]-amid}.

### Beispiel 8

Die Herstellung von (2R,4S)-4-Hydroxy-pyrrolidin-1,2-dicarbonsäure-1-[(4-chlor-phenyl)-amid]-2-{[4-(3-oxo-morpholin-4-yl)-phenyl]-amid} erfolgt analog nachstehendem Schema: 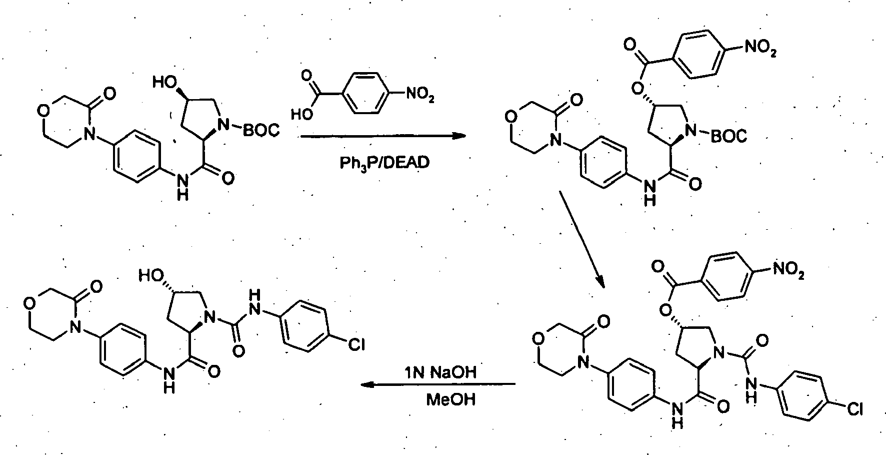
8.1 Zu einer Lösung von 7.0 g (7.26 mmol) (2R,4R)-4-Hydroxy-2-[4-(3-oxo-morpholin-4-yl)-phenylcarbamoyl]-pyrrolidin-1-carbonsäure-*tert*.-butylester, 5.77 g (34.5 mmol) p-Nitrobenzoesäure und 9.18 g (35 mmol) Triphenylphosphin in 350 ml Tetrahydrofuran tropft man bei 0 °C unter Stickstoff 5.51 ml (35 mmol) Azodicarbonsäurediethylester (DEAD) zu. Anschliessend lässt man die Reaktionsmischung 12 Stunden bei Raumtemperatur rühren, dampft sie im Vakuum zur Trockne ein, versetzt den Rückstand mit 20 ml Methylenchlorid, wäscht die Methylenchloridlösung nacheinander mit je 10 ml gesättigter Kochsalzlösung und Wasser und trocknet sie über Natriumsulfat. Nach Abfiltrieren des Trockenmittels und Abziehen des Lösungsmittels am Rotationsverdampfer wird der Rückstand mit 30 ml Diethylether verrieben. Man erhält so 8.5 g (88.8%) (2R,4S)-4-(4-Nitro-benzoyloxy)-2-[4-(3-oxo-morpholin-4-yl)-phenylcarbamoyl]-pyrrolidin-1-carbonsäure-*ter.t*-butylester als leicht gelbe Kristalle, ESI 555.
8.2 Analog zu Beispiel 7 erhält man aus (2R,4S)-4-(4-Nitro-benzoyloxy)-2-[4-(3-oxo-morpholin-4-yl)-phenylcarbamoyl]-pyrrolidin-1-carbonsäure-*tert*.-butylester die Verbindung 4-Nitro-benzoesäure (3S,5R)-1-(4-chlor-phenylcarbamoyl)-5-[4-(3-oxo-morpholin-4-yl)-phenylcarbamoyl]-pyrrolidin-3-ylester als gelbliche Kristalle, ESI 608.
8.3 Die Lösung von 50 mg (0.082 mmol) 4-Nitro-benzoesäure (3S,5R)-1-(4-chlor-phenylcarbamoyl)-5-[4-(3-oxo-morpholin-4-yl)-phenylcarbamoyl]-pyrrolidin-3-ylester in 2 ml Methanol wird unter Eiskühlung mit 0.075 ml 1N Natronlauge versetzt und die Reaktionsmischung 15 min. gerührt. Der ausgefallene Niederschlag wird abfiltriert und mit 2 ml Methanol gewaschen und getrocknet. Man erhält so 35 mg (93%) (2R,4S)-4-Hydroxy-pyrrolidin-1,2-dicarbonsäure-1-[(4-chlor-phenyl)-amid]-2-{[4-(3-oxo-morpholin-4-yl)-phenyl]-amid} als farblose. Kristalle, ESI 459, F. 243° (Zersetzung).

Analog erhält man
(2R, 3S, 4R)-3,4-Dihydroxy-pyrrolidin-1,2-dicarbonsäure-1-[(4-chlor-phenyl)-amid]-2-{[4-(3-oxo-morpholin-4-yl)-phenyl]-amid}, ESI 475, F. 247;
(2S,4R)-4-Hydroxy-pyrrolidin-1,2-dicarbonsäure-1-[(4-chlor-phenyl)-amid]-2-{[4-(3-oxo-morpholin-4-yl)-phenyl]-amid}, ESI 459; F. 253°;
3,4-Dihydroxy-pyrrolidin-1,2-dicarbonsäure-1-[(4-chlor-phenyl)-amid]-2-{[4-(3-oxo-morpholin-4-yl)-phenyl]-amid}.

### Beispiel 8a

Die Herstellung von (2R,4S)-4-Ethinyl-4-hydroxy-pyrrolidin-1,2-dicarbonsäure-1-[(4-chlor-phenyl)-amid]-2-{[4-(3-oxo-morpholin-4-yl)-phenyl]-amid}, ESI 483 erfolgt analog nachstehendem Schema:

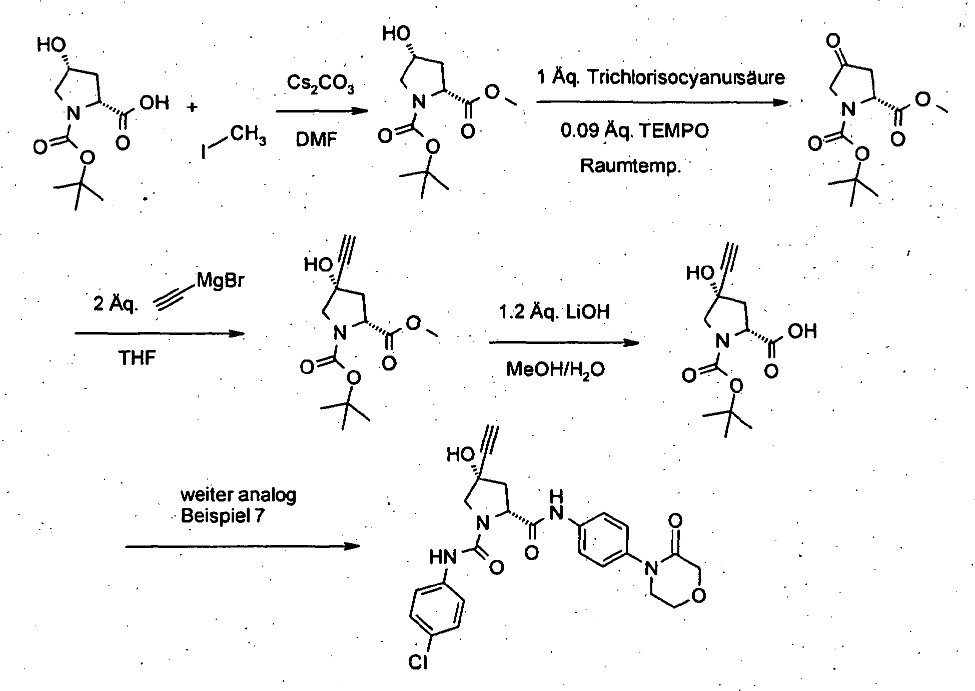

### Beispiel 9

Die Herstellung von (2R,4S)-4-Azido-pyrrolidin-1,2-dicarbonsäure-1-[(4-chlor-phenyl)-amid]-2-{[4-(3-oxo-morpholin-4-yl)-phenyl]-amid} ("A9") und (2R,4S)-4-Amino-pyrrolidin-1,2-dicarbonsäüre-1-[(4-chlor-phenyl)-amid]-2-{[4-(3-oxo-moorpholin-4-yl)-phenyl]-amid} ("A10") erfolgt analog nachstehendem Schema: 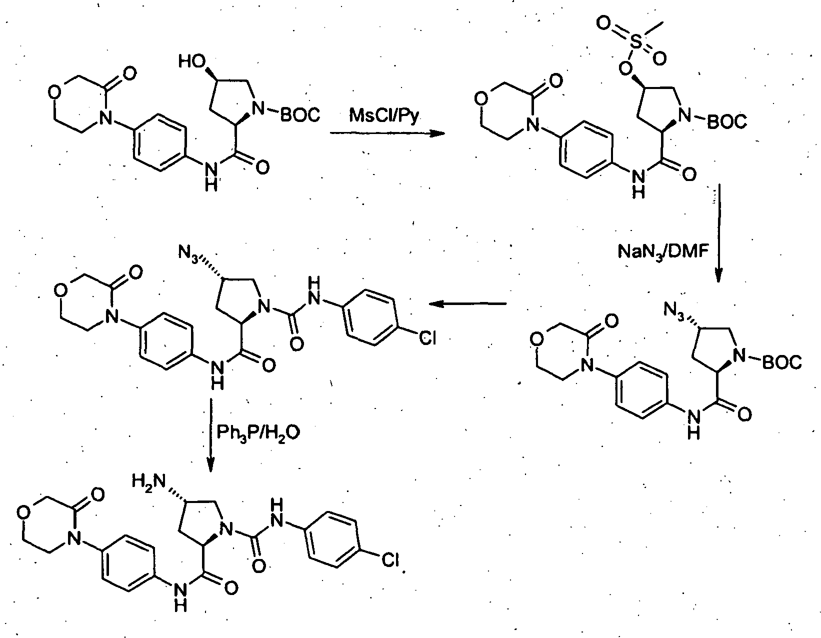
9.1 Eine Lösung von 4.5 g (11.1 mmol) (2R,4R)-4-Hydroxy-2-[4-(3-oxo-morpholin-4-yl)-phenylcarbamoyl]-pyrrolidin-1-carbonsäure-*tert*.-butylester in 20 ml Pyridin wird unter Eiskühlung mit 1.3 ml (16.65 mmol) Methansulfonsäurechlorid tropfenweise versetzt und die Reaktionslösung bei Raumtemperatur 12 Stunden gerührt. Anschließend wird das Pyridin im Vakuum abgezogen, der Rückstand mit 10 ml gesättigter Citronen säurelösung versetzt und die saure Lösung zweimal mit je 10 ml Methylenchlorid extrahiert. Die vereinigten organischen Phasen werden nun mit 10 ml gesättigter Kochsalzlösung gewaschen und über Natriumsulfat getrocknet. Nach Abfiltrieren des Trockenmittels und Abziehen des Lösungsmittels erhält man 5.4 g (100%) (2R,4R)-4-Methansulfonyloxy-2-[4-(3-oxo-morpholin-4-yl)-phenylcarbamoyl]-pyrrolidin-1-carbonsäure-*tert*.-butylester als gelbes Öl, ESI 484.
9.2 Eine Mischung von 5.4 g (11.7 mmol) (2R,4R)-4-Methansulfonyloxy-2-[4-(3-oxo-morpholin-4-yl)-phenylcarbamoyl]-pyrrolidin-1-carbonsäure-*tert*.-butylester und 3.69 g (56.8 mmol) Natriumazid in 50 ml Dimethylformamid (DMF) wird 12 Stunden bei 60 °C gerührt. Anschließend wird von Unlöslichem abfiltriert und das Filtrat im Vakuum zu Trockne eingedampft. Der Rückstand wird dann mit 20 ml Wasser gelöst und die wässrige Lösung zweimal mit je 10 ml Methylenchlorid extrahiert. Die vereinigten Methylenchloridextrakte werden schließlich einmal mit 10 ml gesättigter-Kochsalzlösung gewaschen und über Natriumsulfat getrocknet. Nach Abfiltrieren des Trockenmittels und Abziehen des Lösungsmittels erhält man 4.8 g (100%) (2R,4S)-4-Azido-2-[4-(3-oxo-morpholin-4-yl)-phenylcarbamoyl]-pyrrolidin-1-carbonsäure-*tert*.-butylester als leicht gelbe Kristalle, ESI 431.
9.3 Analog zu Beispiel 7 erhält man aus (2R,4S)-4-Azido-2-[4-(3-oxo-morpholin-4-yl)-phenylcarbamoyl]-pyrrolidin-1-carbonsäure-*tert*.-butylester die Verbindung (2R,4S)-4-Azido-pyrrolidin-1,2-dicarbonsäure-1-[(4-chlorphenyl)-amid] 2-{[4-(3-oxo-morpholin-4-yl)-phenyl]-amid} ("A9") als weißes Pulver, ESI 459, F. 145°.
9.4 Eine Lösung von 25 mg (0.052 mmol) "A9" und 20.46 mg (0.08mmol) Triphenylphosphin in einer Mischung aus 0.5 ml Tetrahydrofuran und 0.5 ml Wasser wird 12 Stunden bei Raumtemperatur gerührt. Anschließend wird nach Abfiltrieren des ausgefallenen Triphenylphosphinoxids das Filtrat zu Trockne eingeengt und der Rückstand mittels präparativer HPLC (Acetonitril/Wasser/0.15 Trifluoressigsäure) gereinigt. Man erhält so 12 mg (40%) (2R,4S)-4-Amino-pyrrolidin-1,2-dicarbonsäure-1-[(4-chlor-phenyl)-amid]-2-{[4-(3-oxo-morpholin-4-yl)-phenyl]-amid} ("10") als farblose Kristalle, ESI 458.

Analog erhält man die Verbindungen
(2R,4R)-4-Azido-pyrrolidin-1,2-dicarbonsäure- 1-[(4-chlor-phenyl)-amid]-2-{[4-(3-oxo-morpholin-4-yl)-phenyl]-amid}, ESI 484, F. 125°;
(2R,4R)-4-Amino-pyrrolidin-1,2-dicarbonsäure-1-[(4-chlor-phenyl)-amid]-2-{[4-(3-oxo-morpholin-4-yl)-phenyl]-amid}, ESI 458, F. 110°;
(2R,4S)-4-Amino-pyrrolidin-1,2-dicarbonsäure-1-[(4-chlor-phenyl)-amid]-2-{[3-methyl-4-(3-oxo-morpholin-4-yl)-phenyl]-amid}, ESI 472, F. 218°.

Aus den 4-Aminoverbindungen erhält man durch
a) Umsetzung mit Acetylchlorid die Verbindungen
   (2R,4S)-4-Acetamino-pyrrolidin-1,2-dicarbonsäure-1-[(4-chlor-phenyl)-amid]-2-{[4-(3-oxo-morpholin-4-yl)-phenyl]-amid},
   (2R,4R)-4-Acetamino-pyrrolidin-1,2-dicarbonsäure-1-[(4-chlorphenyl)-amid]-2-{[4-(3-oxo-morpholin-4-yl)-phenyl]-amid}, ESI 458; und analog
   (2R,4S)-4-Acetamino-pyrrolidin-1,2-dicarbonsäure-1-[(4-chlorphenyl)-amid]-2-{[3-methyl-4-(3-oxo-morpholin-4-yl)-phenyl]-amid}, ESI 514, F. 170°;
b) Umsetzung mit Mesylchlorid die Verbindungen
   (2R,4S)-4-Methylsulfonylamino-pyrrolidin-1,2-dicarbonsäure-1-[(4-chlor-phenyl)-amid]-2-{[4-(3-oxo-morpholin-4-yl)-phenyl]-amid} und
   (2R,4R)-4-Methylsulfonylamino-pyrrolidin-1,2-dicarbonsäüre-1-[(4-chlor-phenyl)-amid]-2-{[4-(3-oxo-morpholin-4-yl)-phenyl]-amid};
c) Umsetzung mit Butylsulfonylchlorid die Verbindungen
   (2R,4R)-4-Butylsulfonylamino-pyrrolidin-1,2-dicarbonsäure-1-[(4-chlor-phenyl)-amid]-2-{[4-(3-oxo-morpholin-4-yl)-phenyl]-amid},
   (2R,4S)-4-Butylsulfonylamino-pyrrolidin-1,2-dicarbonsäure-1-[(4-chlor-phenyl)-amid]-2-{[4-(3-oxo-morpholin-4-yl)-phenyl]-amid}, ESI 592;
d) Umsetzung mit Isobuttersäurechlorid die Verbindungen
   (2R,45)-4-(2-Methylpropanoyl-amino)-pyrrolidin-1,2-dicarbonsäure-1-[(4-chlor-phenyl)-amid]-2-{[4-(3-oxo-morpholin-4-yl)-phenyl]-amid}, ESI 542; F.169.

### Beispiel 10

Die Herstellung von (2,R,4R)-4-Methoxy-pyrrolidin-1,2-dicarbonsäure-1-[(4-chlor-phenyl)-amid]-2-{[4-(3-oxo-morpholin-4-yl)-phenyl]-amid} ("A11") erfolgt analog nachstehendem Schema: 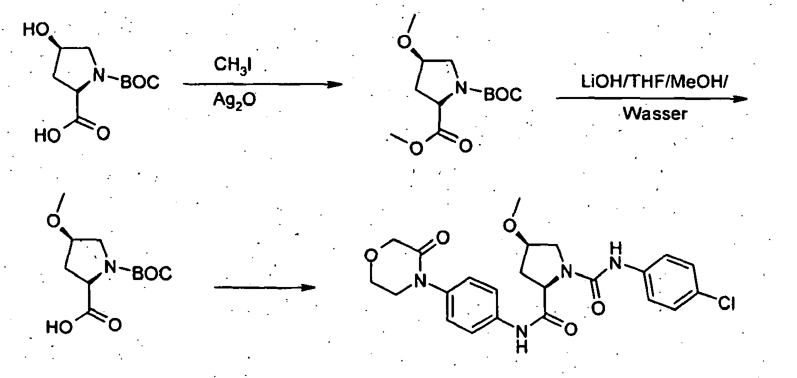
10.1 Eine Mischung von 1 g (4.32 mmol) cis-N-BOC-4-Hydroxy-D-prolin und 3.31 g (14.27 mmol) Silberoxid in 15 ml Aceton wird unter Stickstoff mit 0.94 ml (15.1 mmol) Methyliodid versetzt und die Reaktionsmischung 48 Stunden bei Raumtemperarur gerührt. Anschliessend wird der Niederschlag abfiltriert und das Filtrat im Vakuum zur Trockne eingeengt. Man erhält so 1 g (89.2%) cis-N-BOC-4-Methoxy-D-prolin-methylester als farbloses Öl, das ohne weitere Reinigung weiter umgesetzt wird, ESI 260.
10.2 Eine Lösung von 1 g (3.85 mmol) cis-N-BOC-4-Methoxy-D-prolin-methylester in 75 ml Tetrahydrofuran (THF) wird mit 25 ml Methanol, 25 ml Wasser und 0.28 g (11.57 mmol) Lithiumhydroxid versetzt und die Reaktionslösung 5 Stunden bei Raumtemperatur gerührt. Anschließend wird das Methanol und das THF am Rotationsverdampfer abgezogen und die wässrige Lösung nach einmaligem Ausschütteln mit 10 ml Methylenchlorid mittels gesättigter Citronensäurelösung auf pH 2 angesäuert und die saure Lösung zweimal mit je 10 ml Ethylenchlorid extrahiert. NachTrocknen der vereinigten organischen Phasen über Natriumsulfat und Abziehen des Lösungsmittels erhält man 0.5 g (53%) cis-N-BOC-4-Methoxy-D-prolin als helles Öl, das allmählich kristallisiert, ESI 246.
10.3 Analog zu Beispiel 7 erhält man aus cis-N-BOC-4-Methoxy-D-prolin die Verbindung (2R,4R)-4-Methoxy-pyrrolidin-1,2-dicarbonsäure-1-[(4-chlor-phenyl)-amid]-2-{[4-(3-oxo-morpholin-4-yl)-phenyl]-amid} ("A11") als weisses Pulver, ESI 473, F. 133°.

Analog erhalt man die nachstehenden Verbindungen
(2R,4R)-4-Allyloxy-pyrrolidin-1,2-dicarbonsäure-1-[(4-chlor-phenyl)-amid]-2-{[2-fluor-4-(3-oxo-morpholin-4-yl)-phenyl]-amid}, ESI 517, F. 106° 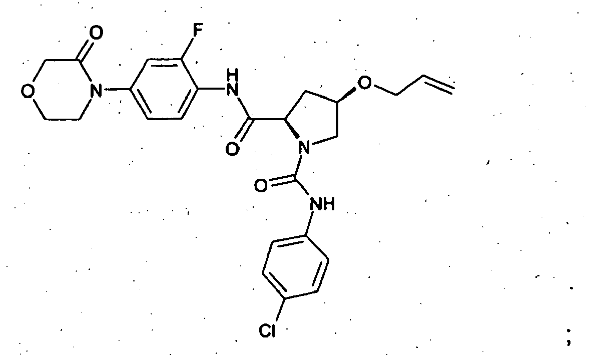 (2R,4R)-4-Ethoxy-pyrrolidin-1,2-dicarbonsäure-1-[(4-chlor-phenyl)-amid]-2-{[4-(3-oxo-morpholin-4-yl)-phenyl]-amid}, ESI 487, F. 136°;
(2R,4R)-4-Propoxy-pyrrolidin-1,2-dicarbonsäure-1-[(4-chlorphenyl)-amid]-2-{[4-(3-oxo-morpholin-4-yl)-phenyl]-amid}, ESI 501, F. 106;
(2R,4R)-4-Allyloxy-pyrrolidin-1,2-dicarbonsäure-1-[(4-chlor-phenyl)-amid]-2-{[4-(3-oxo-morpholin-4-yl)-phenyl]-amid}, ESI 499, F. 100° und als Nebenprodukt
4-Hydroxy-pyrrolidin-1,2-dicarbonsäure-2-{allyl-[4-(3-oxo-morpholin-4-yl)-phenyl]-amid}-1-[(4-chlor-phenyl)-amid], ESI 499;
(2R,4R)-4-Methoxy-pyrrolidin-1,2-dicarbonsäure-1-[(4-chlor-phenyl)-amid]-2-{[3-methyl-4-(3-oxo-morpholin-4-yl)-phenyl]-amid}, ESI 487, F. 140°;
(2R,4R)-4-Methoxy-pyrrolidin-1,2-dicarbonsäure-1-[(4-chlorphenyl)-amid]-2-{[2-fluor-4-(3-oxo-morpholin-4-yl)-phenyl]-amid}, ESI 491, F. 109°;
(2R,4R)-4-Methoxy-pyrrolidin-1,2-dicarbonsäure-1-[(4-chlor-phenyl)-amid]-2-{[3-fluor-4-(3-oxo-morpholin-4-yl)-phenyl]-amid}, ESI 491, F. 99°;
(2R,4R)-4-Ethoxy-pyrrolidin-1,2-dicarbonsäure-1-[(4-chlor-phenyl)-amid]-2-{[3-fluor-4-(3-oxo-morpholin-4-yl)-phenyl]-amid}, ESI 505, F. 131°;
(2R,4R)-4-(Prop-2-in-yloxy)-pyrrolidin-1,2-dicarbonsäure-1-[(4-chlor-phenyl)-amid]-2-{[4-(3-oxo-morpholin-4-yl)-phenyl]-amid}, ESI 497, F. 120°;
(2R,4R)-4-(But-2-in-yloxy)-pyrrolidin-1,2-dicarbonsäure-1-[(4-chlor-phehyl)-amid]-2-{[4-(3-oxo-morpholin-4-yl)-phenyl]-amid},
(2R,4R)-4-(Prop-2-in-yloxy)-pyrrolidin-1,2-dicarbonsäure-1-[(4-chlor-phenyl)-amid]-2-{[2-fluor-4-(3-oxo-morpholin-4-yl)-phenyl]-amid}, ESI 515, F. 108°;
(2R,4S)-4-(Prop-2-in-yloxy)-pyrrolidin-1,2-dicarbonsäure-1-[(4-chlor-phenyl)-amid]-2-{[2-fluor-4-(3-oxo-morpholin-4-yl)-phenyl]-amid}, ESI 515, F. 92°;
(2R,4R)-4-(Methoxycarbonylmethoxy)-pyrrolidin-1,2-dicarbonsäure-1-[(4-chlor-phenyl)-amid]-2-{[4-(3-oxo-morpholin-4-yl)-phenyl]-amid}, ESI 531, F. 106°; und daraus durch Hydrolyse
(2R,4R)-4-(Carboxymethoxy)-pyrrolidin-1,2-dicarbonsäure-1-[(4-chlor-phenyl)-amid]-2-{[4-(3-oxo-morpholin-4-yl)-phenyl]-amid}, ESI 517, F. 134°;
(2R,4R)-4-Methoxy-pyrrolidin-1,2-dicarbonsäure-1-[(4-bromphenyl)-amid]-2-{[2-fluor-4-(3-oxo-moroholin-4-yl)-phenyl]-amid}, ESI 536, F. 103°.

### Beispiel 11

Die Herstellung von Isobuttersäure-(3R,5R)-1-(4-chlor-phenyl- carbamoyl)-5-[4-(3-oxo-morpholin-4-yl)-phenylcarbamoyl]-pyrrolidin-3-yl-ester ("A12") erfolgt analog nachstehendem Schema:

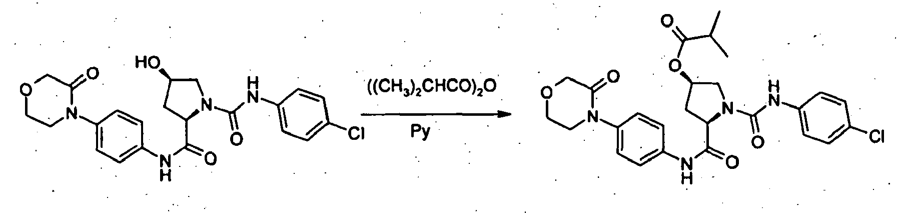

Eine Lösung von 0.2 g (0.44 mmol) "A8" und 0.146 ml Isobuttersäureanhydrid in 1 ml Pyridin wird 12 Stunden bei Raumtemperatur gerührt. Anschließend wird die Reaktionsmischung mit 10 ml Essigsäureethylester versetzt und die Essigsäureethylester-Lösung nacheinander mit je 5 ml 1N Salzsäure und gesättigter Kochsalzlösung gewaschen und über Natriumsulfat getrocknet. Nach Abfiltrieren des Trockenmittels und Abziehen des Lösungsmittels erhält man 183 mg (79.3%) Isobuttersäure-(3R,5R)-1-(4-chlor-phenyl-carbamoyl)-5-[4-(3-oxo-morpholin-4-yl)-phenylcarbamoyl]-pyrrolidin-3-yl-ester ("12") als weisse Kristalle, ESI 529, F. 129°.

Analog erhält man die nachstehenden Verbindungen
Propionsäure-(3R,5R)-1-(4-chlor-phenyl-carbamoyl)-5-[4-(3-oxo-morpholin-4-yl)-phenylcarbamoyl]-pyrrolidin-3-yl-ester, ESI 515;
Essigsäure-(3R,5R)-1-(4-chlor-phenyl-carbamoyl)-5-[4-(3-oxo-morpholin-4-yl)-phenylcarbamoyl]-pyrrolidin-3-yl-ester, ESI 501, F. 148°.

### Beispiel 12

Die Herstellung von [1,3]Dioxolan-4,5-dicarbonsäure-4-[(4-chlorphenyl)-amid]-5-{[4-(3-oxo-morpholin-4-yl)-phenyl]-amid} erfolgt analog nachstehendem Schema:

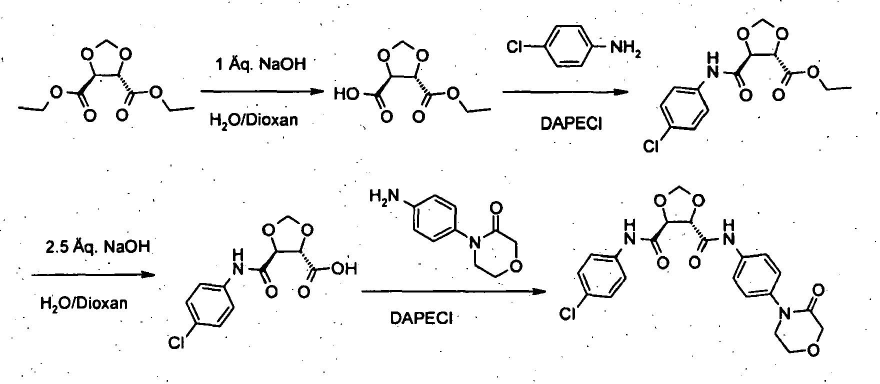

Analog erhält man die nachstehenden Verbindungen
[1,3]Dioxolan-4,5-dicarbonsäure-4-[(4-chlorphenyl)-amid]-5-{[3-methyl-4-(3-oxo-morpholin-4-yl)-phenyl]-amid},
[1,3]Dioxolan-2,2-dimethyl-4,5-dicarbonsäure-4-[(4-chlorphenyl)-amid]-5-{[4-(3-oxo-morpholin-4-yl)-phenyl]-amid}, ESI 474;
[1,3]Dioxolan-2,2-dimethyl-4,5-dicarbonsäure-4-[(4-chlorphenyl)-amid]-5-{[3-methyl-4-(3-oxo-morpholin-4-yl)-phenyl]-amid}, ESI 488.

### Beispiel 13

Analog Beispiel 7 erhält man durch Umsetzung von 1-BOC-piperazin-2-carbonsäure-[4-(3-oxo-morpholin-4-yl)-phenyl]-amid mit 4-Chlorphenyl-isocyanat die Verbindung
1-BOC-piperazin-1,2-dicarbonsäure-1-[4-chlorphenyl)-amid]-2-{[4-(3-oxo-morpholin-4-yl)-phenyl]-amid}

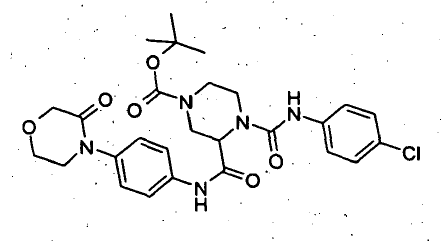

Durch Abspaltung der BOC-Gruppe erhält man
Piperazin-1,2-dicarbonsäure-1-[4-chlorphenyl)-amid]-2-{[4-(3-oxo-morpholin-4-yl)-phenyl]-amid}.

Analog erhält man durch Umsetzung von 4-Chlorphenylisocyanat mit [1,3]Oxazinan-4-carbonsäure-[4-(3-oxo-morpholin-4-yl)-phenyl]-amid die Verbindung
[1,3]Oxazinan-3,4-dicarbonsäure-1-[4-chlorphenyl)-amid]-2-{[4-(3-oxo-morpholin-4-yl)-phenyl]-amid}.

### Beispiel 13 - 1

Die Herstellung von (R)-4-Oxo-pyrrolidin-1,2-dicarbonsäüre-1-[(4-chlorphenyl)-amid]-2-{[4-(3-oxo-morpholin-4-yl)-phenyl]-amid} erfolgt analog nachstehendem Schema:

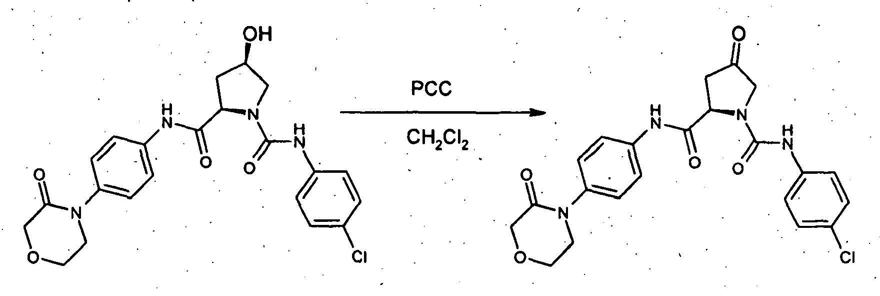

Die Lösung von 0.3 g (0.65 mmol) (1R,4R)-4-Hydroxy-pyrrolidin-1,2-dicarbonsäure-1-[(4-chlor-phenyl)-amid]-2-{[4-(3-oxo-morpholin-4-yl)-phenyl]-amid} (Beispiel 7) in 15 ml Methylenchlorid wird mit 0.21 g (0.98 mmol) Pyridiniumchlorochromat (PCC) versetzt und die Reaktionsmischung 48 Stunden bei Raumtemperatur gerührt. Anschliessend wird oder ausgefallene Niederschlag abfiltriert, das Filtrat dreimal mit je 20 ml Wasser gewaschen und über Natriumsulfat getrocket. Nach Abziehen des Lösungsmittels wird der Rückstand mittels präparativer HPLC aufgereinigt. Man erhält so 140 mg (47%) (R)-4-Oxo-pyrrolidin-1,2-dicarbonsäure-1-[(4-chloro-phenyl)-amid]-2-{[4-(3-oxo-morpholin-4-yl)-phenyl]-amid} als weisses Pulver, ESI 457, F. 154 °.

### Beispiel 13 - 2

Die Herstellung von (2R,4R)-1-[2-(4-Chlor-phenyl)-acetyl]-4-hydroxy-pyrrolidin-2-carbonsäure-[4-(3-oxo-morpholin-4-yl)-phenyl]-amid erfolgt analog nachstehendem Schema:

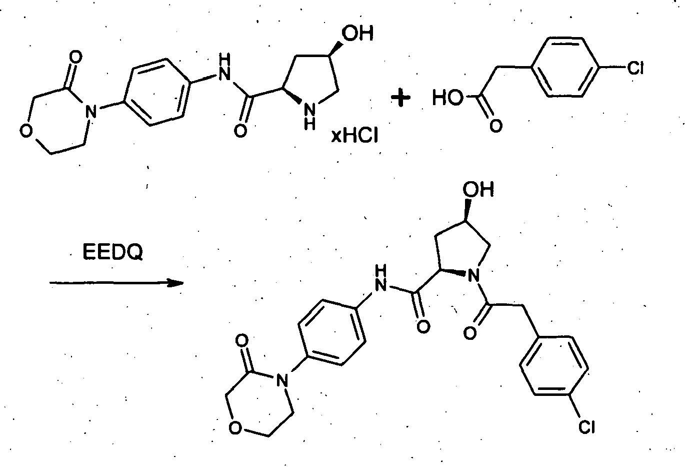

Eine Lösung aus 0.5 g (1.46 mmol) (2R,4R)-4-Hydroxy-pyrrolidin-2-carbonsäure-[4-(3-oxo-morpholin-4-yl)-phenyl]-amid (Beispiel 7.2) und 0.2 ml Triethylamin in 20 ml Toluol wird bei Raumtemperatur nacheinander mit 0.25 g (1.46 mMol) 4-Chlorphenylessigsäure und 0.36 g (1.46 mmol) Ethyl-2-ethoxy-1,2-dihydrochinolin-1-carboxylat (EEDQ) versetzt. Die so erhaltene Reaktionsmischung lässt man anschliessend 12 Stunden bei Raumtemperatur rühren, wäscht sie dann nacheinander mit je 10 ml 1N Salzsäure und 10 ml gesättigter Natriumhydrogencarbonat-lösung und trocknet die organische Phase über Natriumsulfat. Nach Abziehen des Lösungsmittels wird das Rohprodukt mittels präparativer HPLC gereinigt. Man erhält so 0.31g (46.4%) (2R,4R)-1-[2-(4-Chlor-phenyl)-acetyl]-4-hydroxy-pyrrolidin-2-carbonsäure-[4-(3-oxo-morpholin-4-yl)-phenyl]-amid als weisses Pulver, ESI 458, F. 141°.

Analog erhält man die nachstehenden Verbindungen
(2R,4R)-1-(4-Chlor-benzoyl)-4-hydroxy-pyrrolidin-2-carbonsäure-[4-(3-oxo-morpholin-4-yl)-phenyl]-amid, ESI 444, F. 216°;
(2R,4R)-1-(1-1*H*-Indol-3-yl-methanoyl)-4-hydroxy-pyrrolidin-2-carbonsäure-[4-(3-oxo-morpholin-4-yl)-phenyl]-amid, ESI 449, F. 283°;
(2R,4R)-1-(1-1*H*-Indol-6-yl-methanoyl)-4-hydroxy-pyrrolidin-2-carbonsäure-[4-(3-oxo-morpholin-4-yl)-phenyl]-amid, ES1.44.9, F. 148°.

### Beispiel 13 - 3

Die Herstellung von (2R,4R)-4-Ethoxy-pyrrolidin-1,2-dicarbonsäure-1-[(4-chlor-phenyl)-amid]-2-{[3-methyl-4-(3-oxo-morpholin-4-yl)-phenyl]-amid} erfolgt analog nachstehendem Schema:

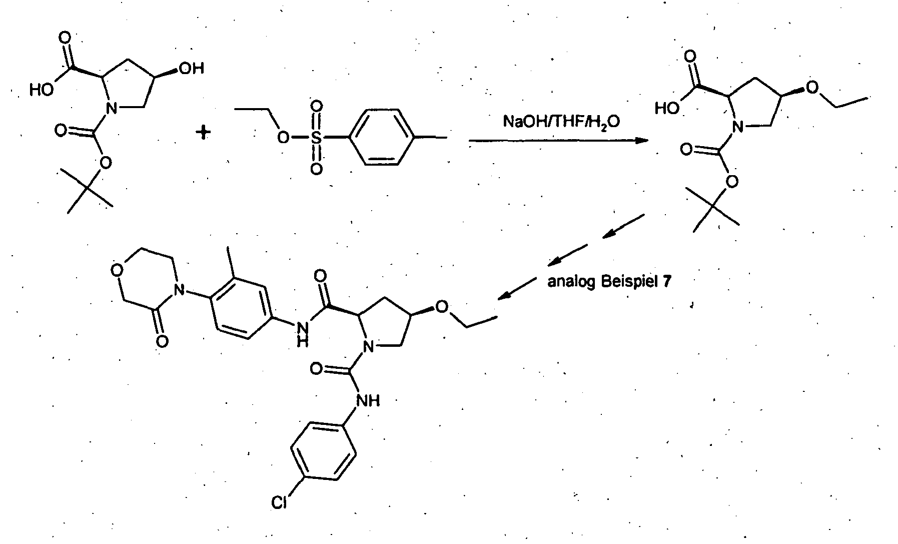

Eine Suspension von 5 g (21.62 mMol) Cis-N-Boc-4-Hydroxy-D-prolin und 8.66 g (43.24 mMol) Ethyl-4-toluolsulfonat in 5 ml Tetrahydrofuran (THF) wird mit einer Lösung von 2.94 g (73.5 mmol) Natriumhydroxid in 5 ml Wasser zugegeben. Nun wird die Reaktionsmischung 12 Stunden bei 40 °C gerührt, ahschliessend am Rotationsverdampfer eingeengt und der Rückstand mit 10 ml Wasser aufgenommen. Die wässrige Lösung wird dann zweimal mit je 10 ml Methylenchlorid gewaschen und mit 2N Salzsäure angesäuert. Die so erhaltene saure Lösung wird dreimal mit je 20 ml Methylenchlorid extrahiert. Nach dem Trocknen der vereinigten Methylenchlorid-Extrakte über Natriumsulfat und Abziehen des Lösungsmittels erhält man 4.87 g (86.9%) Cis-N-Boc-4-Ethoxy-D-prolin als farbloses Öl. ESI: 232.
Analog Beispiel 7 erhält man aus Cis-N-Boc-4-Ethoxy-D-prolin die Verbindung
(2R,4R)-4-Ethoxy-pyrrolidin-1,2-dicarbonsäure-1-[(4-chlor-phenyl)-amid]-2-{[3-methyl-4-(3-oxo-morpholin-4-yl)-phenyl]-amid}, ESI 501, F. 117°.

Analog erhält, man die Verbindung
(2R,4R)-4-Ethoxy-pyrrolidin-1,2-dicarbonsäure-1-[(4-chlor-phenyl)-amid]-2-{[2-fluor-4-(3-oxo₋morpholin-4-yl)-phenyl]-amid}, ESI 505, F. 187°.

### Beispiel 13 - 4

Die Herstellung von (R)-Pyrrolidin-1,2-dicarbonsäure-2-[(4-chlorphenyl)-amide]-1-{[4-(3-oxo-morpholin-4-yl)-phenyl]-amid} erfolgt analog nachstehendem Schema:

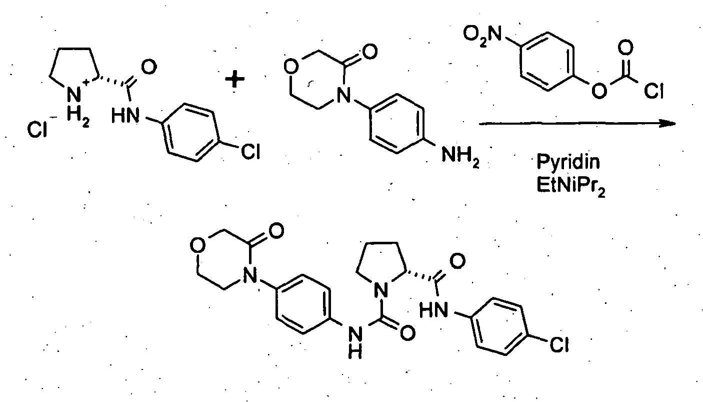

Eine Lösung von 961 mg (5.00 mmol) 4-(4-Amino-phenyl)-morpholin-3-on in 10 ml Dichlormethan wird mit 1.01 g (5.00 mmol) 4-Nitrophenylchlorformiat und 0.404 ml (5.00 mmol) Pyridin versetzt und 1 Stunde bei Raumtemperatur gerührt. Zu der Suspension werden 1.31 g (5.00 mmol) (R)-2-(4-Chlorphenylcarbamoyl)-pyrrolidinium-chlorid und 2.55 ml (15.0 mmol) N-Ethyldiisopropylamin gegeben. Das Reaktionsgemisch wird 12 Stunden bei Raumtemperatur gerührt, anschließend eingedampft und der Rückstand an einer Kieselgelsäule chromatographiert: (R)₋Pyrrolidin-1,2-dicarbonsäure-2-[(4-chlorphenyl)-amide]-1-{[4-(3-oxo-morpholin-4-yl)-phenyl]-amid} als gelblicher Feststoff, ESI 443.

Analog erhält man
(S)-Pyrrolidin-1,2-dicarbonsäure-2-[(4-chlorphenyl)-amide]-1-{[4-(3-oxo-morpholin-4-yl)-phenyl]-amid}, ESI 443.

### Beispiel 13 - 5

Die Herstellung von (R)-1-{2-[4-(3-Oxo-morpholin-4-yl)-phenyl]-acetyl}-pyrrolidin-2-carbonsäure-(4-chlorphenyl)-amid erfolgt analog nachstehendem Schema:

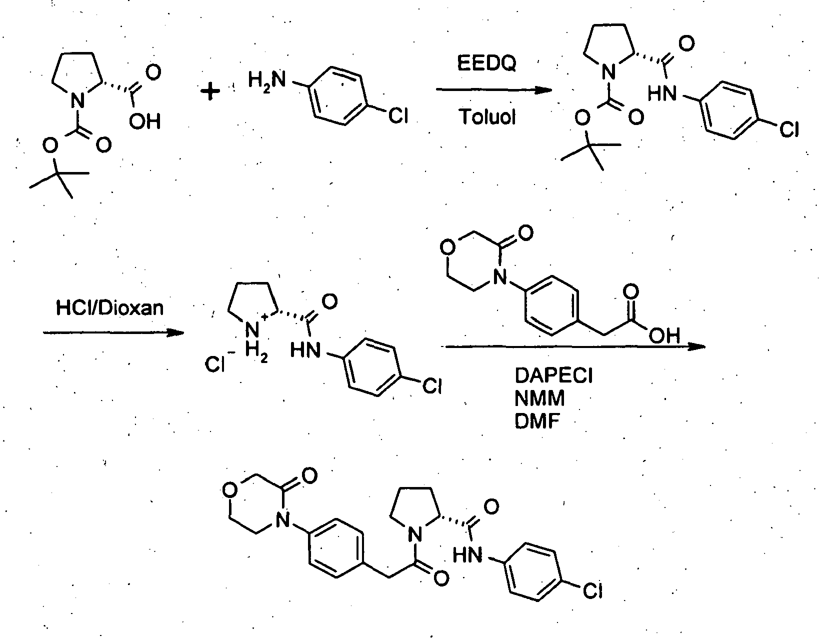

Eine Suspension von 2.80 g (13.0 mmol) N-Boc-D-Prolin und 1.66 g (13.0 mmol) 4-Chloranilin in 50 ml Toluol wird mit 4.82 g (19.5 mmol) Ethyl-2-ethoxy-1,2-dihydrochinolin-1-carboxylat (EEDQ) versetzt und 3 Stunden bei Raumtemperatur gerührt Das Reaktionsgemisch wird filtriert und das Filtrat mit Petrolether versetzt. Der entstandene Niederschlag wird abfiltriert und getrocknet: (R)-2-(4-Chlorphenylcarbamoyl)-pyrrolidin-1-carbonsäure-tert.-butylester als farblose Kristalle; ESI 325.

4.00 g (12.3 mmol) (R)-2-(4-Chlorphenylcarbamoyl)-pyrrolidin-1-carbonsäure-tert.-butylester wird in 20 ml 4 N HCl in Dioxan gelöst und 2 Stunden bei Raumtemperatur belassen. Das Reaktionsgemisch wird eingedampft und getrocknet: (R)-2-(4-Chlorphenylcarbamoyl)-pyrrolidinium-chlorid als leicht bräunlicher Feststoff; ESI 225.

Eine Lösung von 261 mg (1.00 mmol) (R)-2-(4-Chlorphenylcarbamoyl)-pyrrolidinium-chlorid und 235 mg (1.00mmol) 4-(3-Oxomorpholin-4-yl)-phenylessigsäure in 2 ml DMF wird mit 0.26 ml (2.4 mmol) 4-Methylmorpholin und 230 mg (1.2 mmol) N-(3-Dimethylaminopropyl)-N'-ethylcarbodiimidhydrochlorid (DAPECI) versetzt und 18 Stunden bei Raumtemperatur gerührt. Das Reaktionsgemisch wird auf Wasser gegeben und der entstandene Niederschlag abfiltriert: (R)-1-{2-[4-(3-Oxo-morpholin-4-yl)-phenyl]-acetyl}-pyrrolidin-2-carbonsäure-(4-chiorphenyl)-amid als leicht bräunlicher Feststoff; ESI 442.

Analog erhält man (S)-1-{2-[4-(3-Oxo-morpholin-4-yl)-phenyl]-acetyl}-pyrrolidin-2-carbonsäure-(4-chlorphenyl)-amid, ESI 442.

### Herstellung des Carbonsäurebausteins

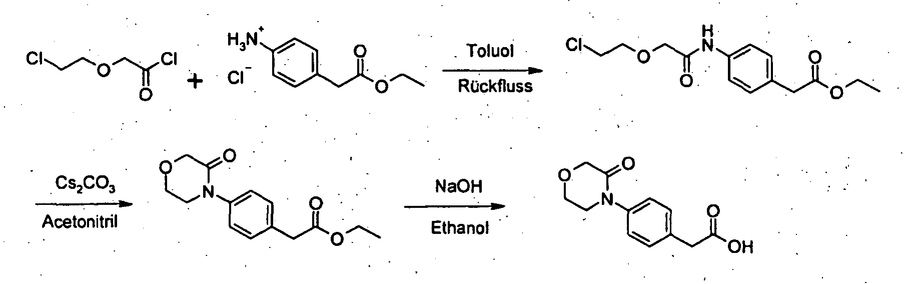

Eine Suspension von 20.0 g (92.7 mmol) 4-Aminophenylessigsäureethylester Hydrochlorid in 25 ml Toluol wird mit 14.6 g (92.7 mmol) (2-Chlorethoxy)-acetylchlorid versetzt und 24 Stunden zum Sieden erhitzt. Das Reaktionsgemisch wird eingedampft und getrocknet: {4-[2-(2-Chlorethoxy)-acetylamino]-phenyl}-essigsäureethylester als gelblicher Feststoff; ESI 300.

Eine Lösung von 26.6 g (88.8 mmol) {4-[2-(2-Chlorethoxy)-acetylamino]-phenyl}-essigsäureethylester in 100 ml Acetonitril wird mit 43.4 g (133 mmol) Caesiumcarbonat versetzt und 18 Stunden bei Raumtemperatur gerührt. Das Reaktionsgemisch wird filtriert und das Filtrat eingedampft: [4-(3-Oxo-morpholin-4-yl)-phenyl]-essigsäureethylester als gelbliches Öl; ESI 264.

In einer Lösung von 3.37 g Natriumhydroxid in 40 ml Ethanol werden 20.2 g (76.8 mmol) [4-(3-Oxomorpholin-4-yl)-phenyl]-essigsäureethylester gelöst und die Reaktionslösung 18 Stunden bei Raumtemperatur gerührt. Das Reaktionsgemisch wird eingeengt, der Rückstand in Wasser gelöst und mit 1 N Salzsäure auf einen pH-Wert von 3 angesäuert. Es wird mit Ethylacetat extrahiert und die organische Phase über Natriumsulfat getrocknet und eingedampft: 4-(3-Oxomorpholin-4-yl)-phenylessigsäure als gelblicher Feststoff; ESI 236.

Analog Beispiel 13-5 erhält man die nachstehenden Verbindungen
(2R,4R)-1-{2-[4-(3-Oxo-morpholin-4-yl)-phenyl]-acetyl}-4-methoxy-pyrrolidin-2-carbonsäure-(4-chlorphenyl)-amid,
(2R,4S)-1-{2-[4-(3-Oxo-morpholin-4-yl)-phenyl]-acetyl}-4-methoxy-pyrrolidin-2-carbonsäure-(4-chlorphenyl)-amid,
(2S,4R)-1-{2-[4-(3-Oxo-morpholin-4-yl)-phenyl]-acetyl}-4-methoxy-pyrrolidin-2-carbonsäure-(4-chlorphenyl)-amid.

### Beispiel 13 - 6

Die Herstellung von (2R,4R)-4-(2,3-Dihydroxypropoxy)-pyrrolidin-1,2-dicarbonsäure-1-[(4-chlor-phenyl)-amid]-2-{[4-(3-oxo-morpholin-4-yl)-phenyl]-amid}, erfolgt analog nachstehendem Schema:

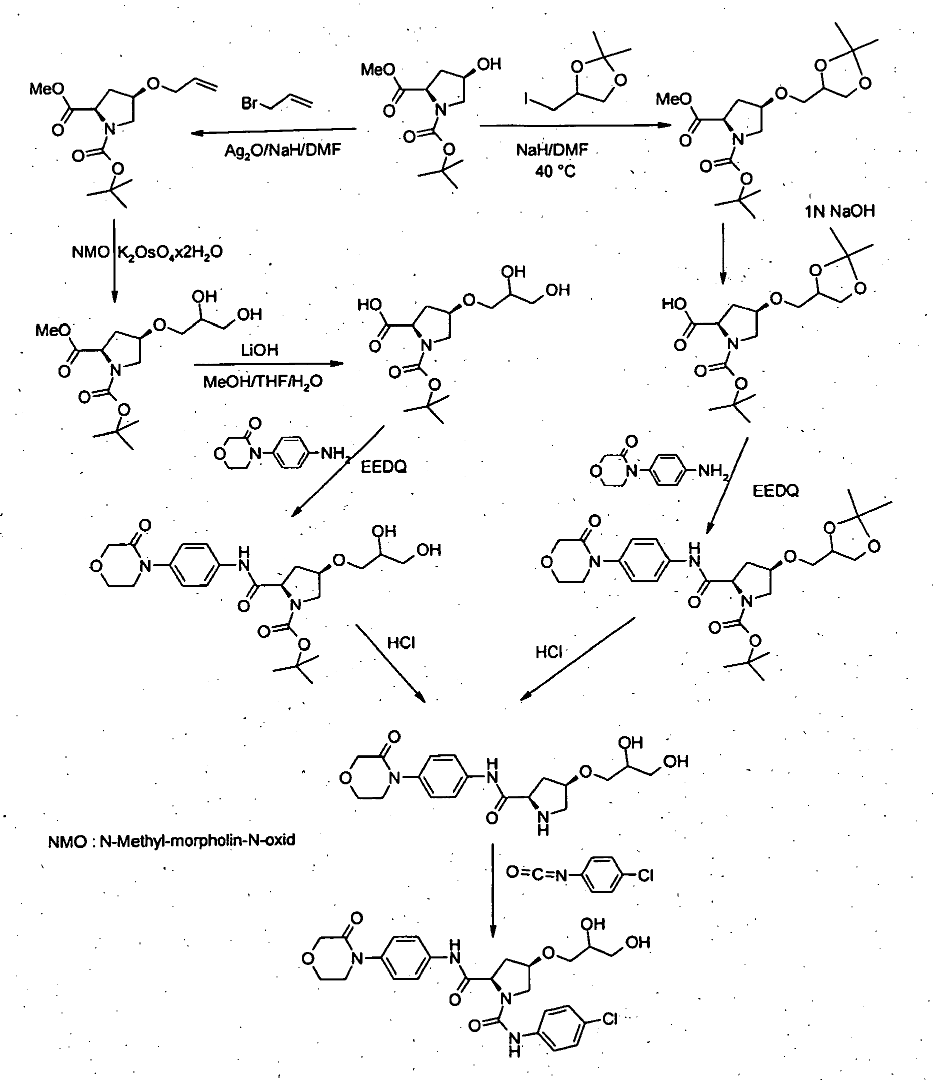

Die Lösung von 10.3 g (42 mmol) (2R,4R)-4-Hydroxy-pyrrolidin-1,2-dicarbonsäure-1-tert-butylester-2-methylester und 36.34 ml (420 mmol) 3-Brom-1-propen in 100 ml Dimethylformamid (DMF) wird unter Stickstoff portionsweise mit 1.55 g (38.6 mmol) Natriumhydrid versetzt und anschließend 15 min. bei Raumtemperatur gerührt. Man fügt dann zu der Reaktionsmischung 9.73 g (42 mmol) Silberoxid portionsweise hinzu und lässt das Reaktionsgemisch noch 12 Stunden bei Raumtemperatur rühren. Danach wird die Reaktionsmischung abfiltriert, das Filtrat im Vakuum zu Trockne eingedampft und der Rückstand in 20 ml gesättigter Zitronensäurelösung aufgenommen. Nach Abfiltrieren des ausgefallenen Niederschlags wird das Filtrat zweimal mit je 20 ml Essigsäureethylester extrahiert. Nach Trocknen der vereinigten organischen Phasen über Natriumsulfat und Abziehen des Lösungsmittels erhält man 11.6 g (2R,4R)-4-Allyloxy-pyrrolidin-1,2-dicarbonsäure-1-*tert*-butylester-2-methylester als rotbraunes Öl; ESI 286.

Die Lösung von 5 g (17.52 mmol) (2R,4R)-4-Allyloxy-pyrrolidin-1,2-dicarbonsäure-1-*tert*-butylester-2-methylester in 60 ml Wasser, 25 ml Aceton und 10 ml tert.Butanol wird bei Raumtemperatur nacheinander mit 6.16 g (52.6 mmol) N-Methylmorpholin-N-oxid (NMO) und 193,7 mg Kaliumosmat-Dihydrat versetzt und 48 Stunden gerührt. Anschließend wird die Reaktionsmischung mit 6.6 g (52.6 mmol) Natriumsulfit versetzt und noch eine Stunde bei Raumtemperatur gerührt. Danach wird das Reaktionsgemisch im Vakuum eingeengt, der Rückstand in 50 ml Wasser aufgenommen und die wässrige Lösung zweimal mit je 20 ml Essigsäureethylester extrahiert. Nach dem Trocknen der vereinigten organischen Phasen über Natriumsulfat und Abziehen des Lösungsmittels erhält man 4.7 g (2R,4R)-4-(2,3-Dihydroxy-propoxy)-pyrrolidin-1,2-dicarbonsäure-1-*tert*-butylester-2-methylester als gelbliches Öl; ESI 320:
Die Lösung von 4.6 g dieses Methylesters werden in 40 ml

Tetrahydrofuran, 10 ml Methanol und 10 ml Wasser mit 1.06 g Lithiumhydroxid versetzt und die Reaktionsmischung wird 12 Stunden bei Raumtemperatur gerührt. Anschließend wird die Reaktionsmischung im Vakuum eingengt, die zurückgebliebene wässrige Lösung mit 10 ml gesättigter Citronensäurelösung versetzt und dreimal mit je 20 ml Essigsäureethylester extrahiert. Nach dem Trocknen der vereinigten organischen Phasen über Natriumsulfat und Abziehen des Lösungsmittels erhält man 4.3 g (2R,4R)-4-(2,3-Dihydroxy-propoxy)-pyrrolidin-1,2-dicarbonsäure-*tert*-butylester als gelbes Pulver; ESI 306. Aus dieser Säure erhält man analog zum Beispiel 7 die Verbindung (2R,4R)-4-(2,3-Dihydroxy-propoxy)-pyrrolidin-1,2-dicarbonsäure-1-[(4-chlor-phenyl)-amid] 2-{[4-(3-oxo-morpholin-4-yl)-phenyl]-amid}; ESI 533.

Analog erhält man
(2R,4R)-4-(2,3-Dihydroxy-propoxy)-pyrrolidin-1,2-dicarbonsäure-1-[(4-chlor-phenyl)-amid] 2-{[2-fluor-4-(3-oxo-morpholin-4-yl)-phenyl]-amid}; ESI 551.

### Beispiel 13 - 7

Die Herstellung von
(2R,4R)-4-(2-Hydroxy-3-pyrrolidin-1-ylpropoxy))-pyrrolidin-1,2-dicarbonsäure-1-[(4-chlor-phenyl)-amid]-2-{[4-(3-oxo-morpholin-4-yl)-phenyl]-amid},
(2R,4R)-4-(2-Oxo-oxazolidin-5-ylmethoxy)-pyrrolidin-1,2-dicarbonsäure-1-[(4-chlor-phenyl)-amid]-2-{[4-(3-oxo-morpholin-4-yl)-phenyl]-amid} und
(2R,4R)-4-(3-Amino-2-hydroxy-propoxy)-pyrrolidin-1,2-dicarbonsäure-1-[(4-chlor-phenyl)-amid]-2-{[4-(3-oxo-morpholin-4-yl)-phenyl]-amid}, ESI 532, F. 115;
erfolgt analog nachstehendem Schema:

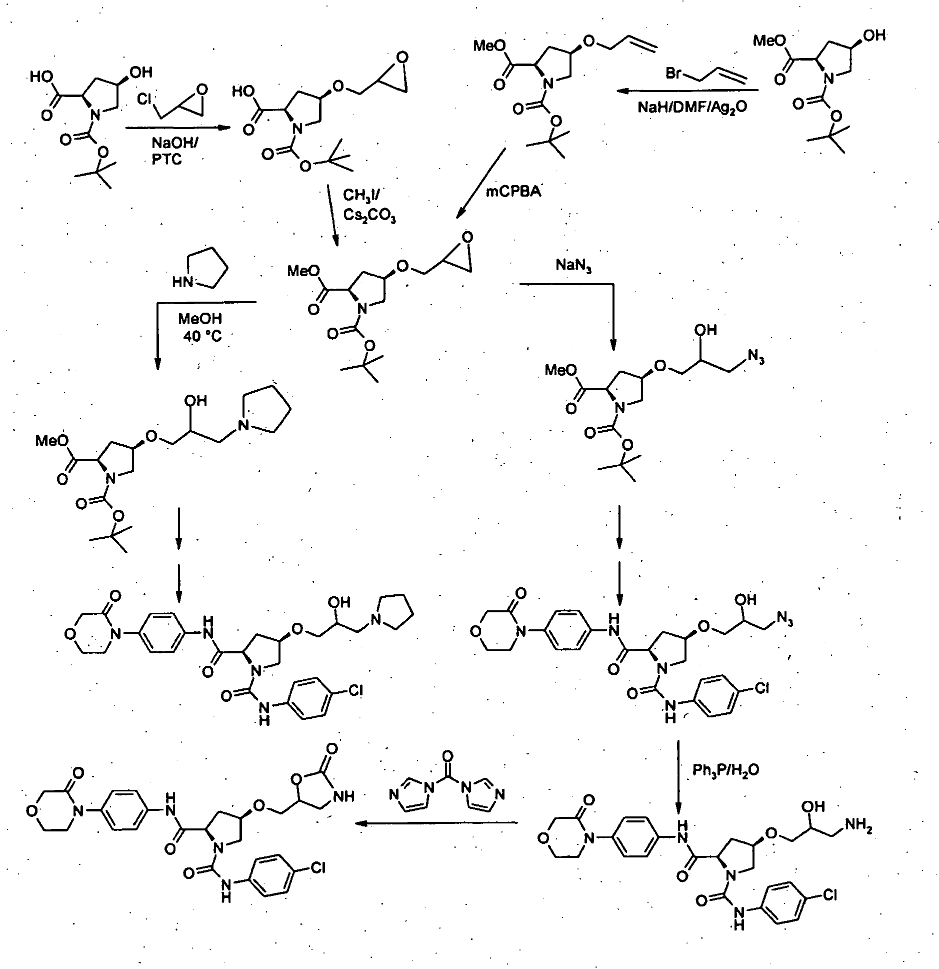

### Beispiel 13 - 8

Die Herstellung von (2R,4R)-4-Methoxy-pyrrolidin-1,2-dicarbonsäure-1-[(4-chlor-phenyl)-amid]-2-{N-methoxycarbonylmethyl-N-[4-(3-oxo-morpholin-4-yl)-phenyl]-amid} erfolgt analog nachstehendem Schema:

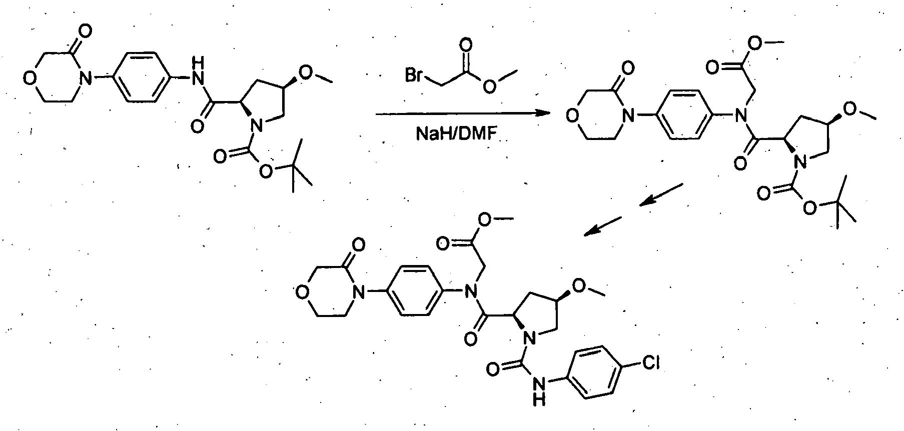

Die Lösung von 1g (2.31 mmol) (2R,4R)-4-Methoxy-2-[4-(3-oxo-morpholin-4-yl)-phenylcarbamoyl]-pyrrolidin-1-carbonsäure-*tert*.-butylester (hergestellt analog zum Beispiel 7.1) in 20 ml Dimethylformamid wird mit 61 mg (2.54 mmol) Natriumhydrid versetzt und 30 min bei Raumtemperatur gerührt. Anschließend fügt man der Reaktionsmischung noch 0.22 mg (2.31 mmol) Bromessigsäuremethylester hinzu und lässt sie dann 12 Stunden bei Raumtemperatur rühren. Danach wird das Reaktionsgemisch im Vakuum eingeengt, der Rückstand in 20 ml Wasser aufgenommen und die wässrige Lösung dreimal mit je 20 ml Methylenchlorid extrahiert. Nach Trocknen der vereinigten organischen Phasen über Natriumsulfat und Abziehen des Lösungsmittels erhält man 1.1 g (2R,4R)-4-Methoxy-2-{methoxycarbonylmethyl-[4-(3-oxo-morpholin-4-yl)-phenyl]-carbamoyl}-pyrrolidin-1-carbonsäure-*tert*.-butylester als gelbes Öl; ESI (M-BOC) 392.

Durch Abspaltung der BOC-Gruppe erhält man daraus (2R,4R)-4-Methoxy-pyrrolidin-1,2-dicarbonsäure-1-[(4-chlor-phenyl)-amid]-2-{N-methoxycarbonylmethyl-N-[4-(3-oxo-morpholin-4-yl)-phenyl]-amid}, ESI 545, F. 106°.

Analog erhält man die Verbindung
(2R,4R)-4-Methoxy-pyrrolidin-1,2-dicarbonsäure-1-[(4-chlor-phenyl)-amid]-2-{N-methoxycarbonylmethyl-N-[2-fluor-4-(3-oxo-morpholin-4-yl)-phenyl]-amid},
ESI 563, F. 100°.

### Beispiel 13 - 9

Die Herstellung von (2R,4R)-4-Hydroxy-pyrrolidin-1,2-dicarbonsäure-1-[(4-chlor-phenyl)-amid]-2-{[4-(3-oxo-morpholin-4-yl)-cyclohexan-1-yl]-amid} erfolgt analog nachstehendem Schema 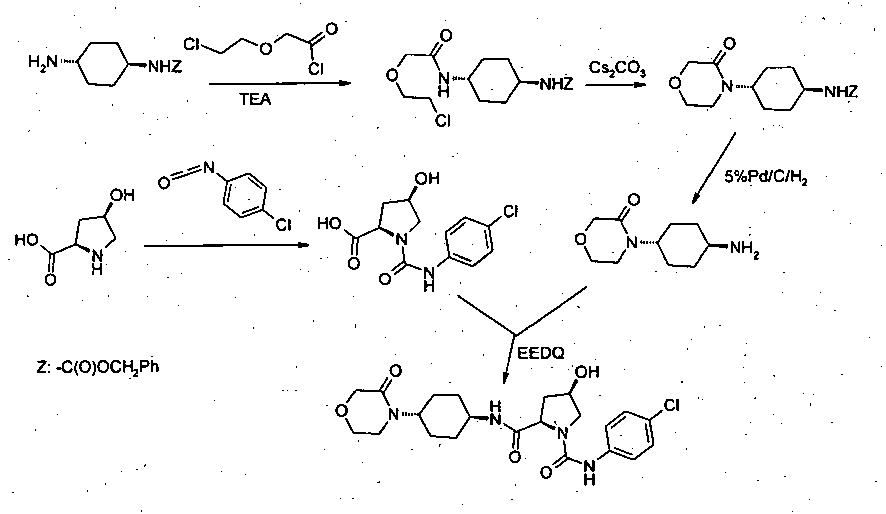
13-9.1 Die Lösung von 10 g (40.3 mmol) (4-Amino-cyclohexyl)-carbaminsäure-benzylester und 6.2 ml Triethylamin (TEA) in 300 ml Tetrahydrofuran wird mit 6.32 g (40.3 mmol) (2-Chlor-ethoxy)-acetylchlorid versetzt und anschließend 20 Stunden bei Raumtemperatur gerührt. Danach engt man die Reaktionsmischung im Vakuum ein, nimmt den Rückstand in 20 ml Wasser auf und extrahiert die wässrige Lösung dreimal,mit je 20 ml Essigsäureethylester. Nach dem Trocknen der vereinigten organischen Phasen über Natriumsulfat und Abziehen des Lösungsmittels wird der Rückstand in 20 ml Acetonitril aufgenommen und die entstandene Lösung mit 2.3 g Cäsiumcarbonat versetzt. Man lässt nun die Reaktionsmischung 48 Stunden bei Raumtemperatur rühren, engt dann im Vakuum ein; nimmt den Rückstand in 20 ml Wasser auf und extrahiert die wässrige Lösung viermal mit je 20 ml Essigsäureethylester. Nach dem Trocknen der vereinigten organischen Phasen über Natriumsulfat und Abziehen des Lösungsmittels nimmt man den Rückstand in 50 ml Tetrahydrofuran auf, versetzt die so entstandene Lösung mit 0.3 g 5%igem Palladium/Kohle und hydriert bis zum Aufhören der Wasserstoffaufnahme. Anschließend wird der Katalysator abfiltriert und das Filtrat zur Trockne im Vakuum eingeengt. Man erhält so 1.5 g 4-(4-Amino-cyclohexyl)-morpholin-3-on als farbloses Öl; ESI 199.
13-9.3 Analog zum Beispiel 7.1 erhält man aus dem Amin 13-9.1 und der Säure 13-9.2 die Verbindung (2R,4R)-4-Hydroxy-pyrrolidin-1,2-dicarbonsäure-1-[(4-chlor-phenyl)-amid]-2-{[4-(3-oxo-morpholin-4-yl)-cyclohexan-1-yl]-amid}, ESI 465; F. 245°.

### Beispiel 13 - 11

Analog Beispiel 7 erhält man die nachstehenden Verbindungen
(2R,4R)-4-Hydroxy-pyrrolidin-1,2-dicarbonsäure-1-[(4-chlor-phenyl)-amid]-2-{[2-aminocarbonyl-4-(3-oxo-morpholin-4-yl)-phenyl]-amid}, ESI 502;
(2R,4R)-4-Hydroxy-2-methyl-pyrrolidin-1,2-dicarbonsäure-1-[(4-chlorphenyl)-amid]-2-{[4-(3-oxo-morpholin-4-yl)-phenyl]-amid}, ESI 457.

### Beispiel 13 -12

Die Herstellung von (2R,4R)-1-[(E)-3-(5-Chlor-thiophen-2-yl)-acryloyl]-4-hydroxy-pyrrolidin-2-carbonsäure-[4-(3-oxo-morpholin-4-yl)-phenyl]-amid erfolgt analog nachstehendem Schema:

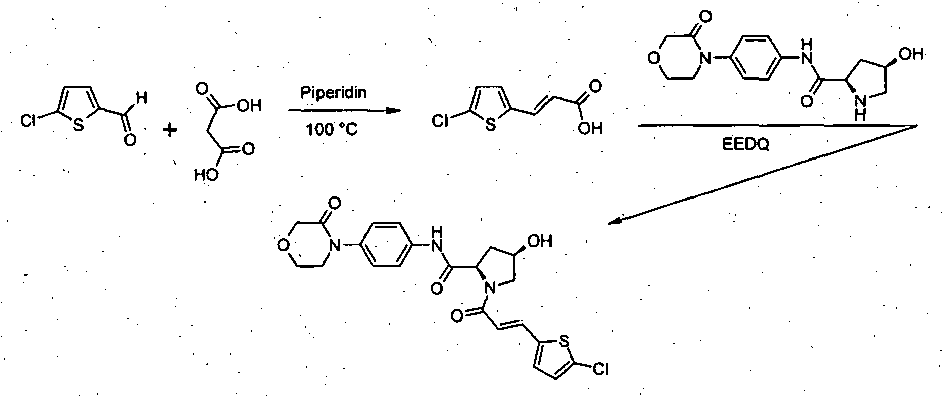

Die Lösung von 1 g (6.62 mmol) 5-Chlor-2-thiophencarboxaldehyd und 1.38 g (13.23 mmol) Malonsäure in 0.07 ml Piperidin und 5 ml Pyridin wird 2 Stunden am Rückfluß erhitzt. Anschließend lässt man die Reaktionslösung abkühlen, giesst sie dann auf 20 ml Wasser und säuert sie mit 2N Salzsäure auf pH 1 an. Das dabei ausgefallene Produkt wird abgesaugt und bei 80° C im Trockenschrank getrocknet. Man erhält so 1.02 g (E)-3-(5-Chlor-thiophen-2-yl)-acrylsäure als braune Kristalle, ESI 189.
Analog zum Beispiel 7.1 erhält man bei der Reaktion zwischen der Verbindung des Beispiel 7.2 und (E)-3-(5-Chlor-thiophen-2-yl)-acrylsäure die Verbindung (2R,4R)-1-[(E)-3-(5-Chlor-thiophen-2-yl)-acryloyl]-4-hydroxy-pyrrolidin-2-carbonsäure-[4-(3-oxo-morpholin-4-yl)-phenyl]-amid als farblose Kristalle, ESI 476, F. 151°.

Analog werden die nachstehenden Verbindungen erhalten
(2R,4R)-1-[(E)-3-Thiophen-3-yl-acryloyl]-4-hydroxy-pyrrolidin-2-carbonsäure-[4-(3-oxo-morpholin-4-yl)-phenyl]-amid, ESI 442, F. 137°;
(2R,4R)-1-[(2E,4E)-5-Phenyl-penta-2,4-dienyloyl]-4-hydroxy-pyrrolidin-2-carbonsäure-[4-(3-oxo-morpholin-4-yl)-phenyl]-amid, ESI 462, F. 127°;
(2R,4R)-1-[(E)-3-(5-Methyl-furan-2-yl)-acryloyl]-4-hydroxy-pyrrolidin-2-carbonsäure-[4-(3-oxo-morpholin-4-yl)-phenyl]-amid, ESI 440, F. 133°;
(2R,4R)-1-[(E)-3-Thiophen-2-yl-acryloyl]-4-hydroxy-pyrrolidin-2-carbonsäure-[4-(3-oxo-morpholin-4-yl)-phenyl]-amid, ESI 442;
(2R,4R)-1-[(E)-3-(5-Chlor-thiophen-2-yl)-acryloyl]-4-methoxy-pyrrolidin-2-carbonsäure-[2-fluor-4-(3-oxo-morpholin-4-yl)-phenyl]-amid, ESI 508;
(2R,4R)-1-[(E)-3-(5-Chlor-thiophen-2-yl)-acryloyl]-4-hydroxy-pyrrolidin-2-carbonsäure-[2-fluor-4-(3-oxo-morpholin-4-yl)-phenyl]-amid, ESI 494, F. 111°;
(2R,4R)-1-[(E)-3-(4-Chlor-phenyl)-acryloyl]-4-hydroxy-pyrrolidin-2-carbonsäure-[4-(3-oxo-morpholin-4-yl)-phenyl]-amid, ESI 470;
(2R,4R)-1-[(E)-3-(3,4-Dichlor-phenyl)-acryloyl]-4-hydroxy-pyrrolidin-2-carbonsäure-[4-(3-oxo-morpholin-4-yl)-phenyl]-amid, ESI 504;
(2R,4R)-1-[(E)-3-(4-Chlor-phenyl)-acryloyl]-4-methoxy-pyrrolidin-2-carbonsäure-[4-(3-oxo-morpholin-4-yl)-phenyl]-amid, ESI 484;
(2R,4R)-1-[(E)-3-(3,4-Dichlor-phenyl)-acryloyl]-4-methoxy-pyrrolidin-2-carbonsäure-[4-(3-oxo-morpholin-4-yl)-phenyl]-amid, ESI 518;
(2R,4R)-1-[(E)-3-1*H*-Imidazol-4-yl-acryloyl]-4-hydroxy-pyrrolidin-2-carbonsäure-[4-(3-oxo-morpholin-4-yl)-phenyl]-amid, ESI 426;
(2R,4R)-1-[(E)-3-(5-Chlor-thiophen-2-yl)-acryloyl]-4-methoxy-pyrrolidin-2-carbonsäure-[4-(3-oxo-morpholin-4-yl)-phenyl]-amid, ESI 490;
(2R,4R)-1-[(E)-3-(5-Chlor-furan-2-yl)-acryloyl]-4-hydroxy-pyrrolidin-2-carbonsäure-[4-(3-oxo-morpholin-4-yl)-phenyl]-amid, ESI 460;
(2R,4R)-1-[(E)-3-(5-Chlor-furan-2-yl)-acryloyl]-4-methoxy-pyrrolidin-2-carbonsäure-[4-(3-oxo-morpholin-4-yl)-phenyl]-amid, ESI 474;
(2R,4R)-1-[(E)-3-(4-Chlor-phenyl)-acryloyl]-4-ethoxy-pyrrolidin-2-carbonsäure-[4-(3-oxo-morpholin-4-yl)-phenyl]-amid, ESI 498;
(2R,4R)-1-[(E)-3-(3,4-Dichlor-phenyl)-acryloyl]-4-ethoxy-pyrrolidin-2-carbonsäure-[4-(3-oxo-morpholin-4-yl)-phenyl]-amid, ESI 532;
(2R,4R)-1-[(E)-3-(5-Chlor-furan-2-yl)-acryloyl]-4-ethoxy-pyrrolidin-2-carbonsäure-[4-(3-oxo-morpholin-4-yl)-phenyl]-amid, ESI 488;
(2R,4R)-1-[(E)-3-(5-Chlor-thiophen-2-yl)-acryloyl]-4-ethoxy-pyrrolidin-2-carbonsäure-[4-(3-oxo-morpholin-4-yl)-phenyl]-amid, ESI 504;
(2R,4R)-1-[(E)-3-(4-Chlor-phenyl)-acryloyl]-4-hydroxy-pyrrolidin-2-carbonsäure-[2-fluor-4-(3-oxo-morpholin-4-yl)-phenyl]-amid, ESI 488;
(2R,4R)-1-[(E)-3-(3,4-Dichlor-phenyl)-acryloyl]-4-hydroxy-pyrrolidin-2-carbonsäure-[2-fluor-4-(3-oxo-morpholin-4-yl)-phenyl]-amid, ESI 522;
(2R,4R)-1-[(E)-3-(5-Chlor-furan-2-yl)-acryloyl]-4-hydroxy-pyrrolidin-2-carbonsäure-[2-fluor-4-(3-oxo-morpholin-4-yl)-phenyl]-amid, ESI 478;
(2R,4R)-1-[(E)-3-(5-Chlor-furan-2-yl)-acryloyl]-4-methoxy-pyrrolidin-2-carbonsäure-[2-fluor-4-(3-oxo-morpholin-4-yl)-phenyl]-amid ESI 492;
(2R,4R)-1-[(E)-3-(4-Chlor-phenyl)-acryloyl]-4-methoxy-pyrrolidin-2-carbonsäure-[2-fluor-4-(3-oxo-morpholin-4-yl)-phenyl]-amid, ESI 502;
(2R,4R)-1-[(E)-3-(3,4-Dichlor-phenyl)-acryloyl]-4-methoxy-pyrrolidin-2-carbonsäure-[2-fluor-4-(3-oxo-morpholin-4-yl)-phenyl]-amid, ESI 536;
(2R,4R)-1-[(E)-3-(4-Chlor-phenyl)-acryloyl]-4-ethoxy-pyrrolidin-2-carbonsäure-[2-fluor-4-(3-oxo-morpholin-4-yl)-phenyl]-amid, ESI 516;
(2R,4R)-1-[(E)-3-(3,4-Dichlor-phenyl)-acryloyl]-4-ethoxy-pyrrolidin-2-carbonsäure-[2-fluor-4-(3-oxo-morpholin-4-yl)-phenyl]-amid, ESI 550;
(2R,4R)-1-[(E)-3-(5-Chlor-furan-2-yl)-acryloyl]-4-ethoxy-pyrrolidin-2-carbonsäure-[2-fluor-4-(3-oxo-morpholin-4-yl)-phenyl]-amid, ESI 506;
(2R,4R)-1-[(E)-3-(5-Chlor-thiophen-2-yl)-acryloyl]-4-ethoxy-pyrrolidin-2-carbonsäure-[2-fluor-4-(3-oxo-morpholin-4-yl)-phenyl]-amid, ESI 522;
(2R,4R)-1-[(E)-3-1*H*-Imidazol-4-yl-acryloyl]-4-ethoxy-pyrrolidin-2-carbonsäure-[4-(3₋oxo-morpholin-4-yl)-phenyl]-amid, ESI 454;
(2R,4R)-1-[(E)-3-1*H*-Imidazol-4-yl-acryloyl]-4-hydroxy-pyrrolidin-2-carbonsäure-[2-fluor-4-(3-oxo-morpholin-4-yl)-phenyl]-amid, ESI 444;
(2R,4R)-1-[(E)-3-1*H*-Imidazol-4-yl-acryloyl]-4-methoxy-pyrrolidin-2-carbonsäure-[2-fluor-4-(3-oxo-morpholin-4-yl)-phenyl]-amid, ESI 458;
(2R,4R)-1-[(E)-3-1*H*-Imidazol-4-yl-acryloyl]-4-ethoxy-pyrrolidin-2-carbonsäure-[2-fluor-4-(3-oxo-morpholin-4-yl)-phenyl]-amid, ESI 472;
(2R,4R)-1-[(E)-3-Pyridin-3-yl-acryloyl]-4-hydroxy-pyrrolidin-2-carbonsäure-[2-fluor-4-(3-oxo-morpholin-4-yl)-phenyl]-amid, ESI 455;
(2R,4R)-1-[(E)-3-Pyridin-3-yl-acryloyl]-4-ethoxy-pyrrolidin-2-carbonsäure-[4-(3-oxo-morpholin-4-yl)-phenyl]-amid, ESI 465;
(2R,4R)-1-[(E)-3-Pyridin-3-yl-acryloyl]-4-methoxy-pyrrolidin-2-carbonsäure-[2-fluor-4-(3-oxo-morpholin-4-yl)-phenyl]-amid, ESI 469;
(2R,4R)-1-[(E)-3-Pyridin-3-yl-acryloyl]-4-ethoxy-pyrrolidin-2-carbonsäure-[2-fluor-4-(3-oxo₋morpholin-4-yl)-phenyl]-amid, ESI 483;
(2R,4R)-1-[(E)-3-Pyridin-3-yl-acryloyl]-4-hydroxy-pyrrolidin-2-carbonsäure-[4-(3-oxo-morpholin-4-yl)-phenyl]-amid, ESI 437;
(2R,4R)-1-[(E)-3-Pyridin-3-yl-acryloyl]-4-methoxy-pyrrolidin-2-carbonsäure-[4-(3-oxo-morpholin-4-yl)-phenyl]-amid, ESI 451;
(2R,4R)-1-[(E)-3-Pyridin-4-yl-acryloyl]-4-hydroxy-pyrrolidin-2-carbonsäure-[4-(3-oxo-morpholin-4-yl)-phenyl]-amid, ESI 437;
(2R,4R)-1-[(E)-3-Pyridin-4-yl-acryloyl]-4-ethoxy-pyrrolidin-2-carbonsäure-[4-(3-oxo-morpholin-4-yl)-phenyl]-amid, ESI 465;
(2R,4R)-1-[(E)-3-1*H*-Imidazol-4-yl-acryloyl]-4-methoxy-pyrrolidin-2-carbonsäure-[4-(3-oxo-morpholin-4-yl)-phenyl]-amid, ESI 440;
(2R,4R)-1-[(E)-3-(4-Brom-thiophen-2-yl)-acryloyl]-4-hydroxy-pyrrolidin-2-carbonsäure-[4-(3-oxo-morpholin-4-yl)-phenyl]-amid, ESI 521;
(2R,4R)-1-[(E)-3-(4-Brom-thiophen-2-yl)-acryloyl]-4-ethoxy-pyrrolidin-2-carbonsäure-[4-(3-oxo-morpholin-4-yl)-phenyl]-amid, ESI 549;
(2R,4R)-1-[(E)-3-(5-Brom-thiophen-2-yl)-acryloyl]-4-hydroxy-pyrrolidin-2-carbonsäure-[4-(3-oxo-morpholin-4-yl)-phenyl]-amid, ESI 521;
(2R,4R)-1-[(E)-3-(5-Brom-thiophen-2-yl)-acryloyl]-4-ethoxy-pyrrolidin-2-carbonsäure-[4-(3-oxo-morpholin-4-yl)-phenyl]-amid, ESI 549.

### Beispiel 13 - 13

Analog Beispiel 7 werden die folgenden Verbindungen erhalten
(2R,4R)-4-Hydroxy-pyrrolidin-1,2-dicarbonsäure-1-[(4-chlor-phenyl)-amid]-2-{[4-((S)-2-methyl-3-oxo-morpholin-4-yl)-phenyl]-amid}, ESI 473;
(2R)-Pyrrolidin-1,2-dicarbonsäure-1-[(4-chlor-phenyl)-amid]-2₋{[4-((S)-2-methyl-3-oxo-morpholin-4-yl)-phenyl]-amid}, ESI 457;
(2R,4R)-4-Hydroxy-pyrrolidin-1,2-dicarbonsäure-1-[(4-chlor-phenyl)-amid]-2-{[4-((R)-2-methyl-3-oxo-morpholin-4-yl)-phenyl]-amid}, ESI 473;
(2R)-Pyrrolidin-1,2-dicarbonsäure-1-[(4-chlor-phenyl)-amid]-2-{[4-((R)-2-methyl-3-oxo-morpholin-4-yl)-phenyl]-amid}, ESI 457;
(2R)-Pyrrolidin-1,2-dicarbonsäure-1-[(4-chlor-phenyl)₋amid]-2-{[4-(3-oxo-morpholin-4-yl)-2-phenoxy-phenyl]-amid}, ESI 535;
(2R,4R)-4-Hydroxy-pyrrolidin-1,2-dicarbonsäure-1-[(4-chlor-phenyl)-amid]-2-{[2-fluor-4-((R)-2-methyl-3-oxo-morpholin-4-yl)-phenyl]-amid}, ESI 491;
Piperidin-1,3-dicarbonsäure-1-[(4-chlor-phenyl)-amid]-3-{[4-(3-oxo-morpholin-4-yl)-phenyl]-amid}, ESI 457;
Piperidin-1,3-dicarbonsäure-1-[(4-chlor-phenyl)-amid]-3-{[3-methyl-4-(3-oxo-morpholin-4-yl)-phenyl]-amid}, ESI 471;
(2R,4R)-4-Hydroxy-pyrrolidin-1,2-dicarbonsäure-1-[(4-chlor-phenyl)-amid]-2-{[2-methyl-4-(3-oxo-morpholin-4-yl)-phenyl]-amid}, ESI 473;
(2R,4R)-4-Hydroxy-pyrrolidin-1,2-dicarbonsäure-1-[(4-chlor-phenyl)-amid]-2-{[4-(3-oxo-morpholin-4-yl)-phenyl]-amid}, ESI 459;
(2R,4R)-4-Hydroxy-pyrrolidin-1,2-dicarbonsäure-1-[(4-chlor-phenyl)-amid]-2-{[2-(3-oxo-morpholin-4-yl)-phenyl]-amid}, ESI 459.

### Beispiel 13 - 15

Die Herstellung von (2R,4R)-4-(2-Methoxy-ethoxy)-pyrrolidin-1,2-dicarbonsäure-1-[(4-chlor-phenyl)-amid]-2-{[4-(3-oxo-morpholin-4-yl)-phenyl]-amid}, ESI 517, erfolgt wie nachstehend beschrieben

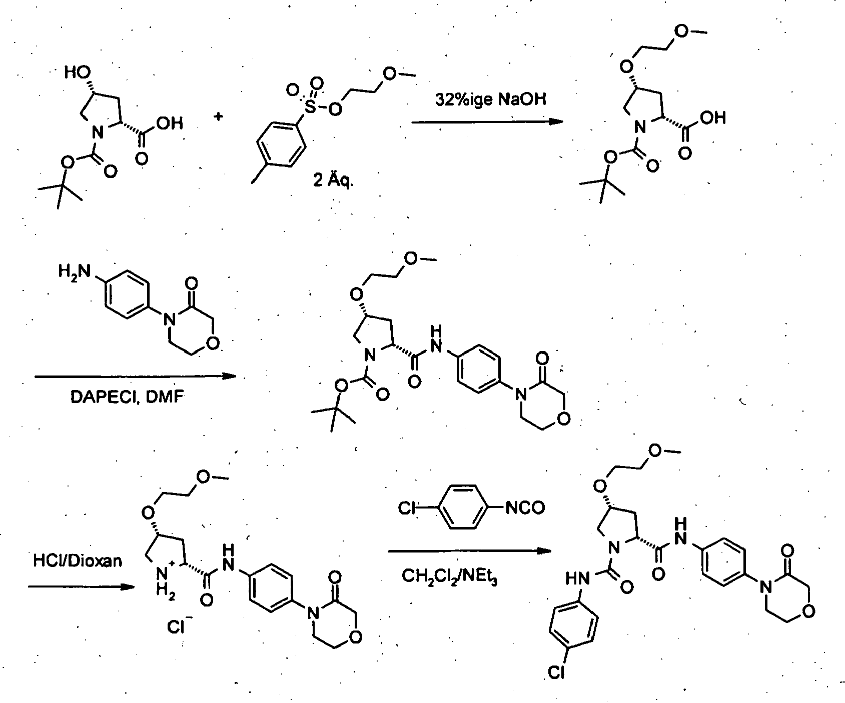

### 14. Beispiele zur Herstellung von Zwischenverbindungen

14.1 Nach folgendem Schema lassen sich **alle** Verbindungen der folgenden Formel VI (mit R = H oder Methyl; n = 3, 4 oder 5) synthetisieren. 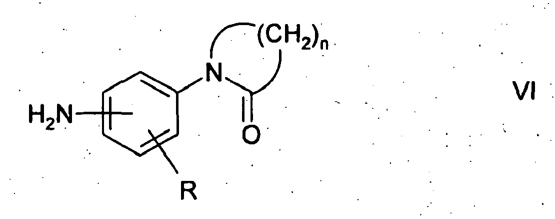 Z.B. Synthese von 1-(4-Amino-2-methylphenyl)-piperidin-2-on: 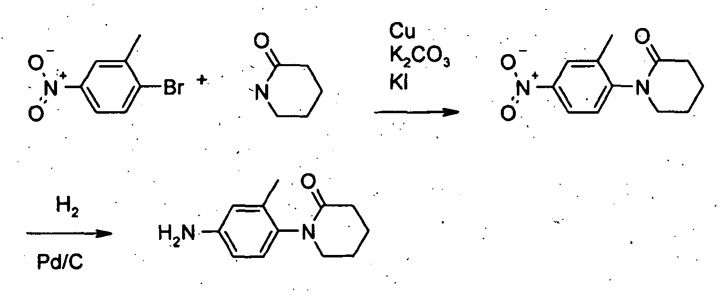
14.2 Synthese des Phenylpiperidonbausteins ohne Methylgruppe: 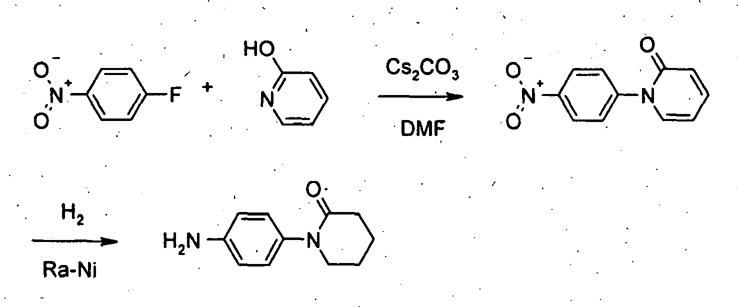
   Die Herstellung von 1-(4-Amino-2-methyl-phenyl)-piperidin-2-on erfogt z.B. wie nachfolgend angegeben: 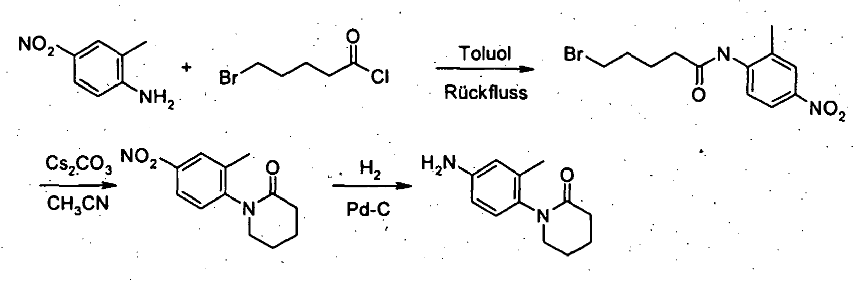
14.3 1-(4-Amino-phenyl)-1*H*-pyrazin-2-on 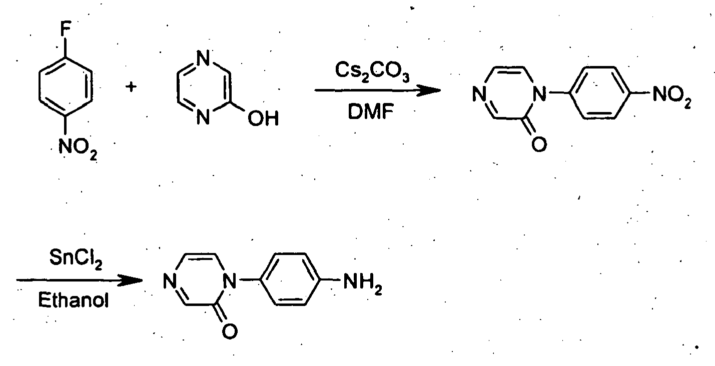
14.4 1-(4-Amino-2,5-dimethyl-phenyl)-piperidin-2-on 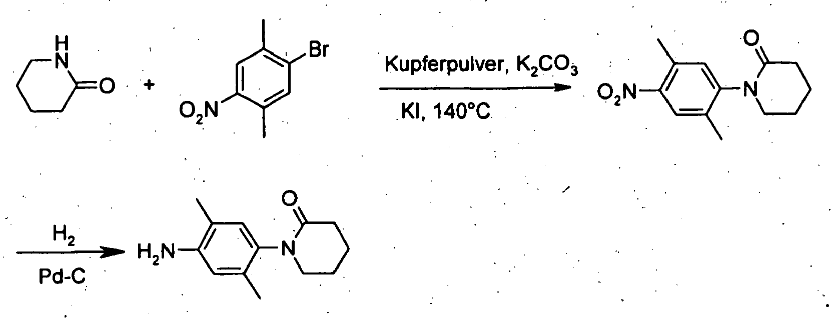
14.5 1-(4-Amino-3-methyl-phenyl)-piperidin-2-on 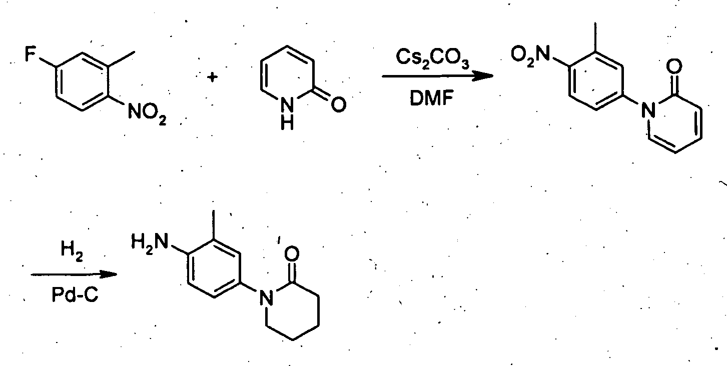
14.6 1-(5-Amino-pyridin-2-yl)-piperidin-2-on 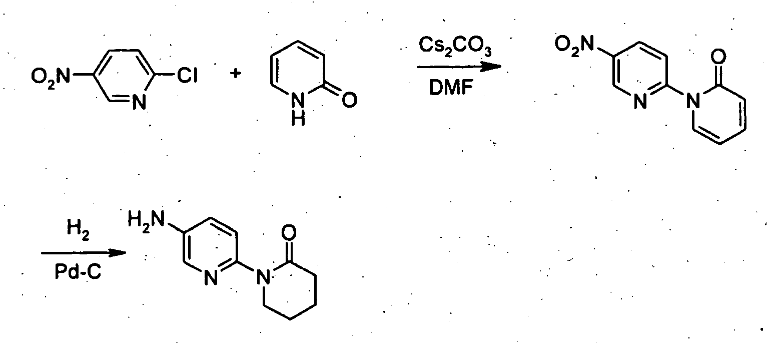
14.7 1-(4-Aminomethyl-phenyl)-piperidin-2-on 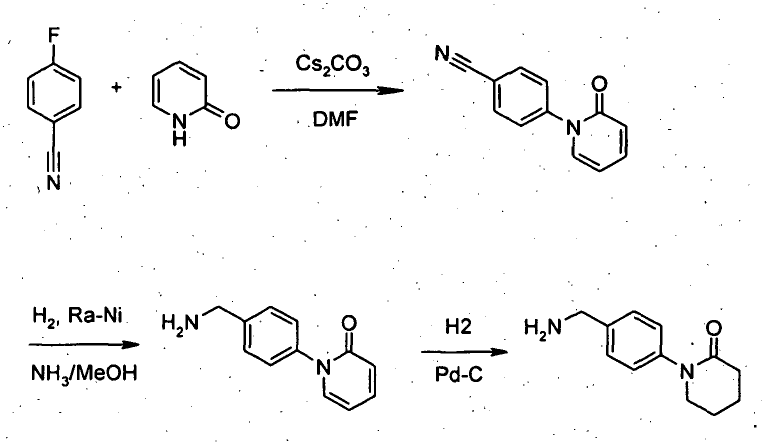
14.8 2-(4-Amino-phenyl)-2-aza-bicyclo[2.2.2]octan-3-on 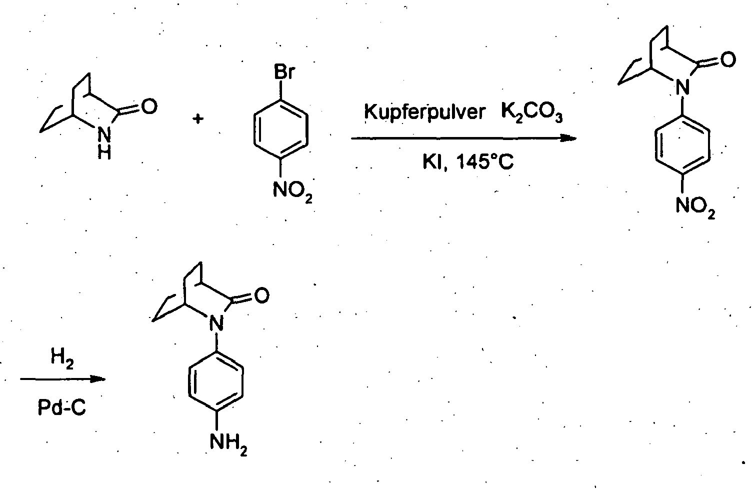
14.9 1-(3-Amino-6-ethyl-phenyl)-pyrrolidin-2-on 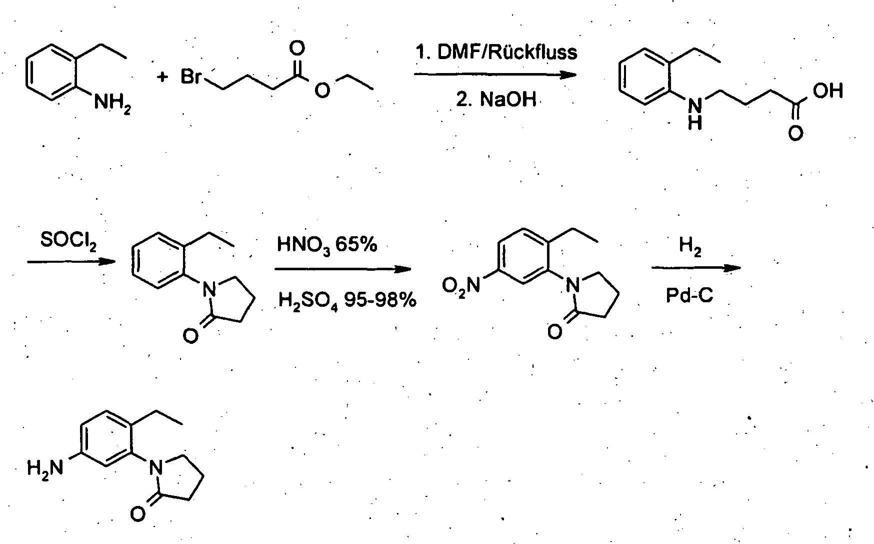
14.10 2-(4-Amino-2-trifluormethyl-phenyl)-2-aza-bicydo[2.2.2]octan-3-on 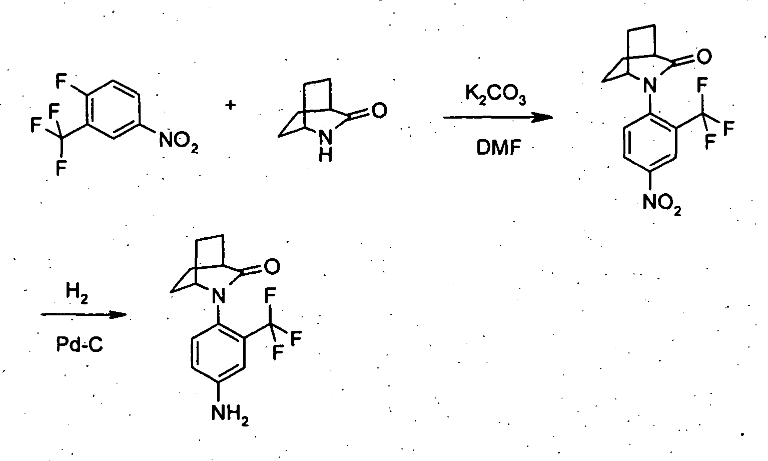
14.11 1-(4-Amino-3-chlor-phenyl)-pyrrolidin-2-on 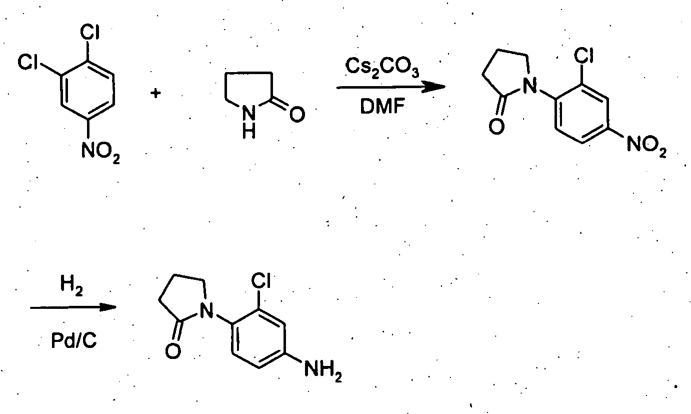
14.12 1-(4-Amino-2-trifluormethyl-phenyl)-piperidin-2-on 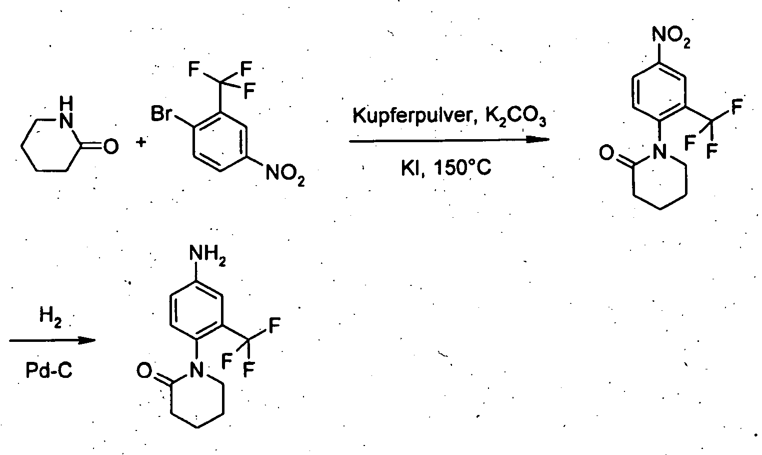
14.13 3-(4-Amino-2-methyl-phenyl)-[1,3]oxazinan-2-on 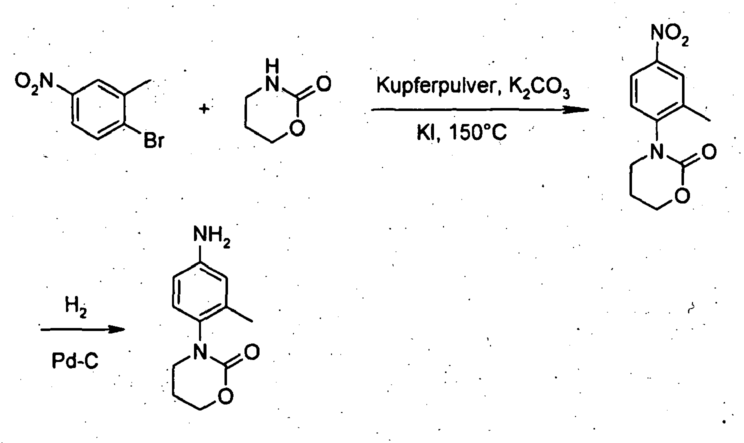
14.14 4-(4-Amino-phenyl)-morpholin-3-on 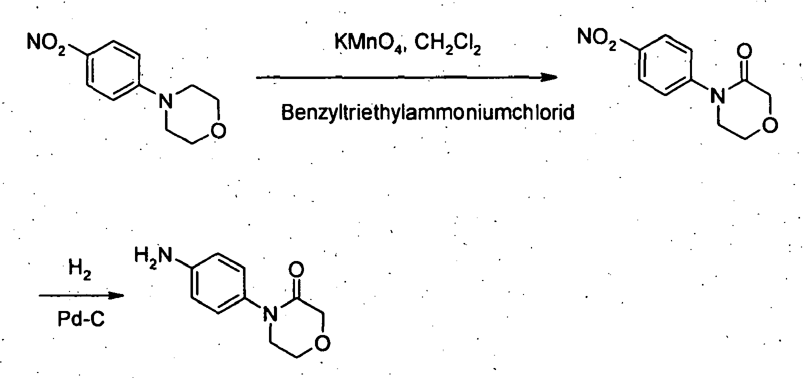
14.15 1-(4-Amino-phenyl)-pyridin-2-on 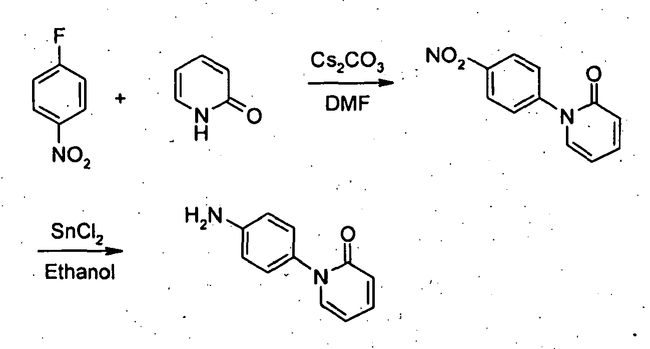
14.16 1-(4-Amino-2-methyl-phenyl)-piperidin-2-on 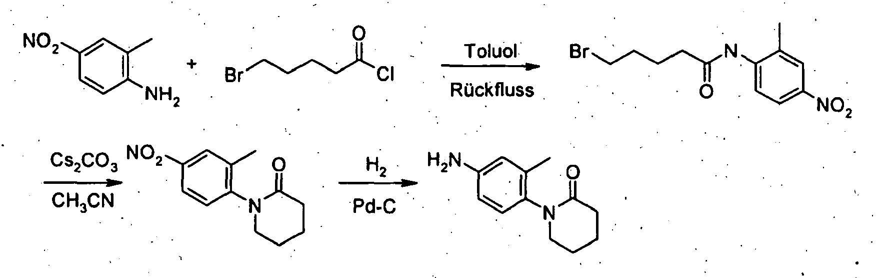
14.17 1-(4-Amino-phenyl)-1*H*-pyridin-4-on 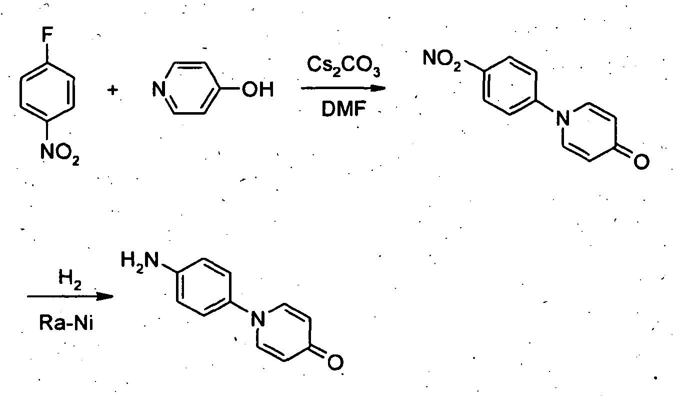
14.18 1-(4-Amino-phenyl)-4-tert.-butyloxycarbonyl-piperazin-2-on 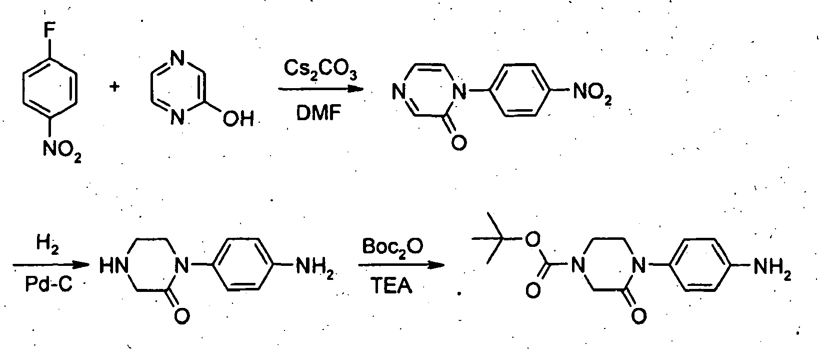
14.19 1-(3-Aminophenyl)-piperidin-2-on 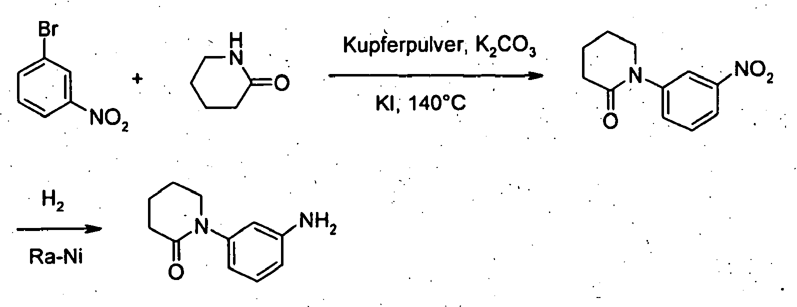
14.20 1-(4-Amino-phenyl)-2-caprolactam 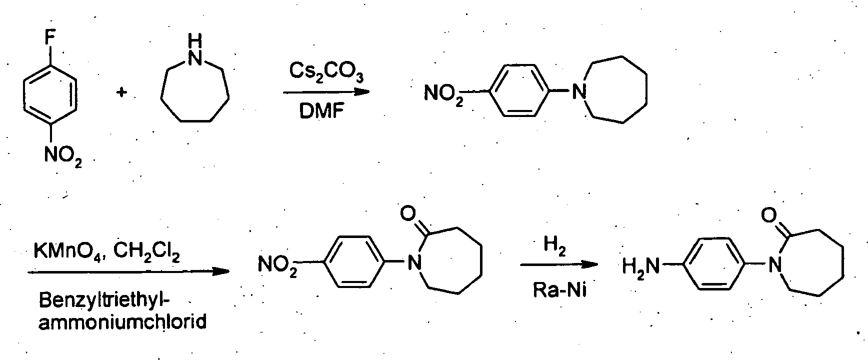
14.21 1-(4-Amino-3-fluor-phenyl)-piperidin-2-on 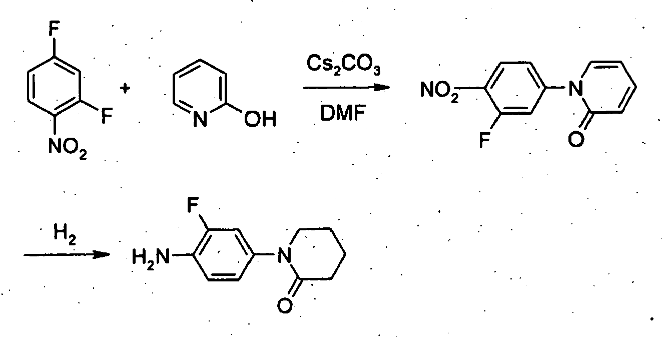
14.22 1-(4-Amino-2-fluor-phenyl)-piperidin-2-on 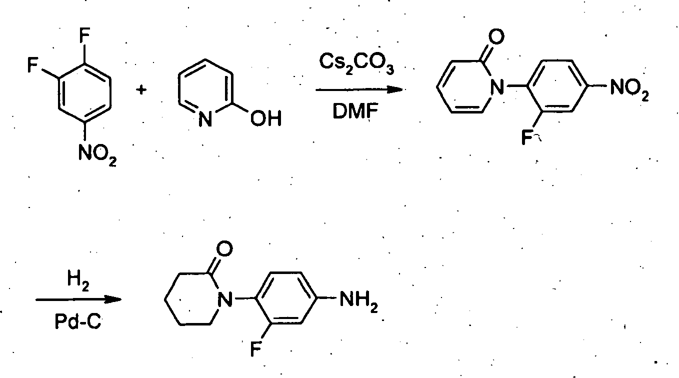
14.23 1-(4-Amino-2-fluorphenyl)-2-caprolactam 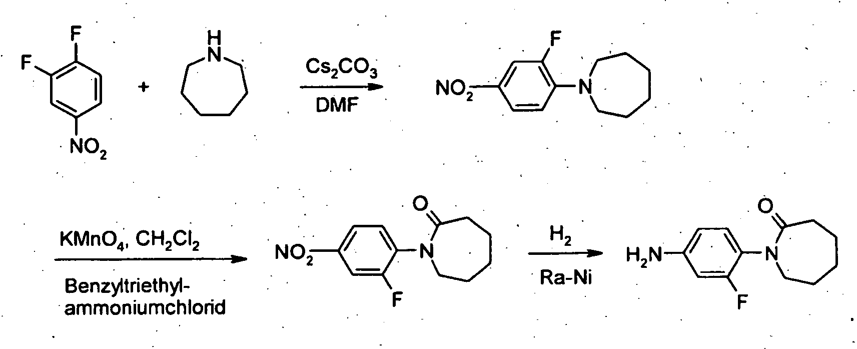
14.24 4-(4-Amino-2-fluorphenyl)-[1,4]oxazepan-5-on 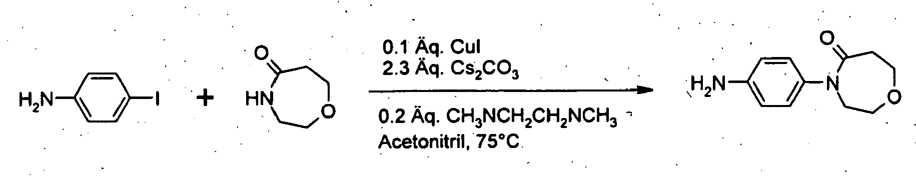
14.25 4-(4-Amino-3-phenoxy-phenyl)-morpholin-3-on 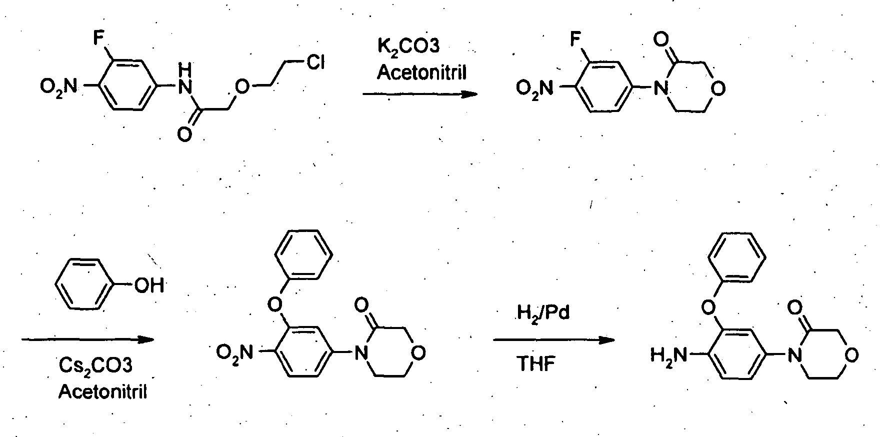
14.26 2-[3-(4-Chlorphenyl)-ureido]-cyclopentancarbonsäure 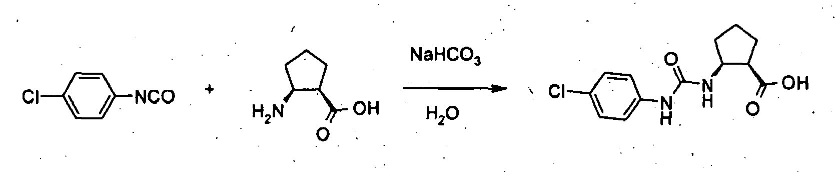
14.27 1-(4-Chlor-phenylcarbamoyl)-piperidin-3-carbonsäure 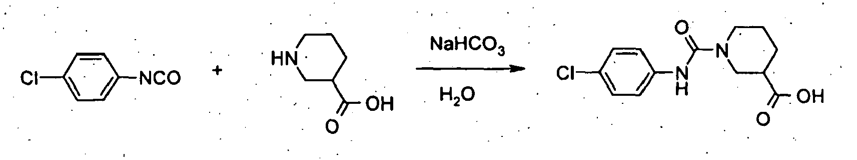
14.28 4-(4-Amino-phenyl)-[1,4]oxazepan-3-on

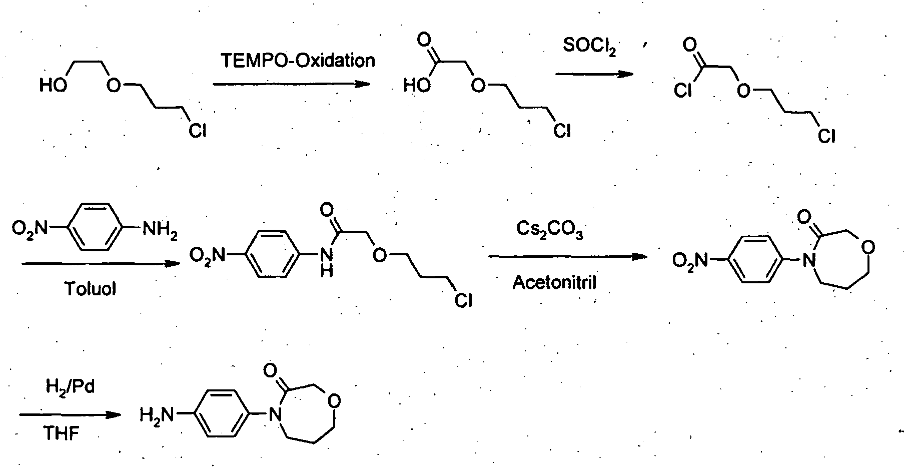

Die TEMPO-Oxidation wird nach folgender Literatur durchgeführt: L. DeLuca et al., J. Org. Chem. 68, 4999-5001 (2003).

### Pharmakologische Daten

### Affinität zu Rezeptoren

**Tabelle 1**

| Verbindung | FXa-IC₅₀ [M] | TF/FVIIa-IC₅₀ [M] |
|---|---|---|
| Nr. | | |
| "A1" | 1.8 x 10⁻⁸ | 2.3 x 10⁻⁸ |
| "A2" | 2.7 x 10⁻⁸ | |
| "AB1" | 1.8 x 10⁻⁸ | 3.9 x 10⁻⁸ |
| "A6" | 3.7 x 10⁻⁹ | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 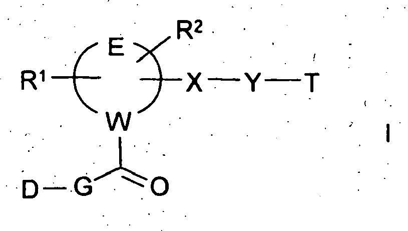 worin
D ein- oder zweifach durch Hal substituiertes Phenyl, Pyridyl oder Thienyl,
R¹ H, =O, COOR³, OH, OA, NH₂, Alkyl mit 1, 2, 3, 4, 5 oder 6 C-Atomen, N₃, Ethinyl, Vinyl, Allyloxy, -OCOR³, NHCOA oder NHSO₂A,
R² H, =O, OH, OA oder Alkyl mit 1, 2, 3, 4, 5 oder 6 C-Atomen,
R¹ und R² zusammen auch einen spirocyclisch gebundenen 3- bis 6-gliedrigen Carbocyclus,
R³ H oder A,
R⁴ H oder A, 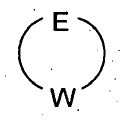 Pyrrolidin-1,2-diyl, Piperidin-1,2-diyl, Oxazolidin-3,4-oder 3,5-diyl, Thiazolidin-3,4-diyl, 2,5-Dihydro-1*H*-pyrrol-1,5-diyl, [1,3]-Dioxolan-4,5-diyl, [1,3]-Oxazinan-3,4-diyl, Piperazin-1,4-diyl, Tetrahydrofuran-3,4-diyl oder Azetidin-1,2-diyl,
G (CH₂)ₙ oder (CH₂)ₙNH-,
X CONH,
Y unsubstituiertes oder ein- oder zweifach durch Methyl, Trifluormethyl, Ethyl, Propyl, Cl oder F substituiertes 1,3- oder 1,4-Phenylen,
T ein- oder zweifach durch Carbonylsauerstoff substituiertes Morpholin-4-yl
A unverzweigtes oder verzweigtes Alkyl mit 1-10 C-Atomen und worin 1-7 H-Atome durch F ersetzt sein können,
Hal F, Cl, Br oder I,
n 0, 1 oder 2,
sowie ihre pharmazeutisch verwendbaren Solvate, Salze und Stereoisomere, einschließlich deren Mischungen in allen Verhältnissen.

2. Verbindungen gemäß Anspruch 1 ausgewählt aus der Gruppe
(R)-Pyrrolidin-1,2-dicarbonsäure-1-[(4-chlor-phenyl)-amid]-2-{[4-(3-oxo-morpholin-4-yl)-phenyl]-amid},
(R)-Pyrrolidin-1,2-dicarbonsäure-1-[(4-chlor-phenyl)-amid]-2-{[3-methyl-4-(3-oxo-morpholin-4-yl)-phenyl]-amid},
(R)-Pyrrolidin-1,2-dicarbonsäure-1-[(4-chlor-phenyl)-amid]-2-{[3-fluor-4-(3-oxo-morpholin-4-yl)-phenyl]-amid},
(R)-Pyrrolidin-1,2-dicarbonsäure-1-[(4-chlor-phenyl)-amid]-2-{[2-fluor-4-(3-oxo-morpholin-4-yl)-phenyl]-amid},
(R)-Pyrrolidin-1,2-dicarbonsäure-1-[(4-chlor-phenyl)-amid]-2-{[3-trifluormethyl-4-(3-oxo-morpholin-4-yl)-phenyl]-amid},
(R)-Piperidin-1,2-dicarbonsäure-1-[(4-[(chlor-phenyl)-amid]-2-{[3-methyl-4-(3-oxo-morpholin-4-yl)-phenyl]-amid},
(R)-2,5-Dihydro-pyrrol-1,2-dicarbonsäure-1-[(4-chlor-phenyl)-amid]-2-{[4-(3-oxo-morpholin-4-yl)-phenyl]-amid},
(R)-1-(5-Chlor-thiophen-2-carbonyl)-pyrrolidin-2-carbonsäure-*N*-[4-(3-oxo-morpholin-4-yl)-phenyl]-amid,
(R)-1-(5-Chlor-thiophen-2-carbonyl)-pyrrolidin-2-carbonsäure-*N*-[3-methyl-4-(3-oxo-morpholin-4-yl)-phenyl]-amid,
(R)-Oxazolidin-3,4-dicarbonsäure-3-[(4-chlor-phenyl)-amid]-4-{[4-(3-oxo-morpholin-4-yl)-phenyl]-amid},
(R)-Oxazolidin-3,4-dicarbonsäure-3-[(4-chlor-phenyl)-amid]-4-{[3-methyl-4-(3-oxo-morpholin-4-yl)-phenyl]-amid},
(4R,5S)- 5-Methyl-oxazolidin-3,4-dicarbonsäure-3-[(4-chlor-phenyl)-amid]-4-{[4-(3-oxo-morpholin-4-yl)-phenyl]-amid},
(4R,5S)-5-Methyl-oxazolidin-3,4-dicarbonsäure-3-[(4-chlor-phenyl)-amid-4-{[3-methyl-4-(3-oxo-morpholin-4-yl)-phenyl]-amid},
(4R,5S)-5-Methyl-oxazolidin-3,4-dicarbonsäure-3-[(4-chlor-phenyl)-amid]-4-{[3-fluor-4-(3-oxo-morpholin-4-yl)-phenyl]-amid},
(4R,5S)-5-Methyl-oxazolidin-3,4-dicarbonsäure-3-[(4-chlor-phenyl)-amid]-4-{[3-chlor-4-(3-oxo-morpholin-4-yl)-phenyl]-amid},
(4R,5R)-5-Methyl-oxazolidin-3,4-dicarbonsäure-3-[(4-chlor-phenyl)-amid]-4-{[3-methyl-4-(3-oxo-morpholin-4-yl)-phenyl]-amid},
(S)-Thiazolidin-3,4-dicarbonsäure-3-[(4-chlor-phenyl)-amid]-4-{[4-(3-oxo-morpholin-4-yl)-phenyl]-amid},
(S)-1,1-Dioxo-1λ⁶-thiazolidin-3,4-dicarbonsäure-3-[(4-chlor-phenyl)-amid] 4-{[4-(3-oxo-morpholin-4-yl)-phenyl]-amid},
(S)-Thiazolidin-3,4-dicarbonsäure-3-[(4-chlor-phenyl)-amid]-4-{[3-methyl-4-(3-oxo-morpholin-4-yl)-phenyl]-amid},
(S)-1,1-Dioxo-1λ⁶-thiazolidin-3,4-dicarbonsäure-3-[(4-chlor-phenyl)-amid]4-{[3-methyl-4-(3-oxo-morpholin-4-yl)-phenyl]-amid},
3-(5-Chlorthiophen-2-carbonyl)-oxazolidin-5-carbonsäure-[4-(3-oxo-morpholin-4-yl)-phenyl]-amid,
3-(5-Chlorthiophen-2-carbonyl)-oxazolidin-5-carbonsäure-[3-methyl-4-(3-oxo-morpholin-4-yl)-phenyl]-amid,
(2R,4R)-4-Hydroxypyrrolidin-1,2-dicarbonsäure-1-[(5-chlorpyridin-2-yl)-amid]-2-{[4-(3-oxo-morpholin-4-yl)-phenyl]-amid},
(R)-4,4-Dimethoxypyrrolidin-1,2-dicarbonsäure-1-[(5-chlorpyridin-2-yl)-amid]-2-{[4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[2-fluor-4-(3-oxo-morpholin-4-yl)-phenyl]-amid},
(2R,3R)-3-Hydroxy-pyrrolidin-1,2-dicarbonsäure-1-[(4-chlor-phenyl)-amid]-2-{[3-fluor-4-(3-oxo-morpholin-4-yl)-phenyl]-amid},
(2R,3S)-3-Hydroxy-pyrrolidin-1,2-dicarbonsäure-1-[(4-chlor-phenyl)-amid]-2-{[3-fluor-4-(3-oxo-morpholin-4-yl)-phenyl]-amid},
(2R,4S)-4-Hydroxy-pyrrolidin-1,2-dicarbonsäure-1-[(4-chlor-phenyl)-amid]-2-{[4-(3-oxo-morpholin-4-yl)-phenyl]-amid},
(2S,4R)-4-Hydroxy-pyrrolidin-1,2-dicarbonsäure-1-[(4-chlor-phenyl)-amid]-2-{[4-(3-oxo-morpholin-4-yl)-phenyl]-amid},
3,4-Dihydroxy-pyrrolidin-1,2-dicarbonsäure-1-[(4-chlor-phenyl)-amid]-2-{[4-(3-oxo-morpholin-4-yl)-phenyl]-amid},
(2R,4S)-4-Azido-pyrrolidin-1,2-dicarbonsäure-1-[(4-chlor-phenyl)-amid]-2-{[4-(3-oxo-morpholin-4-yl)-phenyl]-amid},
(2R,4S)-4-Amino-pyrrolidin-1,2-dicarbonsäure-1-[(4-chlor-phenyl)-amid]-2-{[4-(3-oxo-morpholin-4-yl)-phenyl]-amid},
(2R,4R)-4-Azido-pyrrolidin-1,2-dicarbonsäure-1-[(4-chlor-phenyl)-amid]-2-{[4-(3-oxo-morpholin-4-yl)-phenyl]-amid},
(2R,4R)-4-Amino-pyrrolidin-1,2-dicarbonsäure-1-[(4-chlor-phenyl)-amid]-2-{[4-(3-oxo-morpholin-4-yl)-phenyl]-amid},
(2R,4S)-4-Acetamino-pyrrolidin-1,2-dicarbonsäure-1-[(4-chlor-phenyl)-amid]-2-{[4-(3-oxo-morpholin-4-yl)-phenyl]-amid},
(2R,4R)-4-Acetamino-pyrrolidin-1,2-dicarbonsäure-1-[(4-chlor-phenyl)-amid]-2-{[4-(3-oxo-morpholin-4-yl)-phenyl]-amid},
(2R,4S)-4-Methylsulfonylamino-pyrrolidin-1,2-dicarbonsäure-1-[(4-chlor-phenyl)-amid]-2-{[4-(3-oxo-morpholin-4-yl)-phenyl]-amid},
(2R,4R)-4-Methylsulfonylamino-pyrrolidin-1,2-dicarbonsäure-1-[(4-chlor-phenyl)-amid]-2-{[4-(3-oxo-morpholin-4-yl)-phenyl]-amid},
(2R,4R)-4-Methoxy-pyrrolidin-1,2-dicarbonsäure-1-[(4-chlor-phenyl)-amid]-2-{[4-(3-oxo-morpholin-4-yl)-phenyl]-amid},
(2R,4R)-4-Ethoxy-pyrrolidin-1,2-dicarbonsäure-1-[(4-chlor-phenyl)-amid]-2-{[4-(3-oxo-morpholin-4-yl)-phenyl]-amid},
(2R,4R)-4-Propoxy-pyrrolidin-1,2-dicarbonsäure-1-[(4-chlor-phenyl)-amid]-2-{[4-(3-oxo-morpholin-4-yl)-phenyl]-amid},
(2R,4R)-4-Allyloxy-pyrrolidin-1,2-dicarbonsäure-1-[(4-chlor-phenyl)-amid]-2-{[4-(3-oxo-morpholin-4-yl)-phenyl]-amid},
Isobuttersäure-(3R,5R)-1-(4-chlor-phenyl-carbamoyl)-5-[4-(3-oxo-morpholin-4-yl)-phenylcarbamoyl]-pyrrolidin-3-yl-ester,
Propionsäure-(3R,5R)-1-(4-chlor-phenyl-carbamoyl)-5-[4-(3-oxo-morpholin-4-yl)-phenylcarbamoyl]-pyrrolidin-3-yl-ester,
Essigsäure-(3R,5R)-1-(4-chlor-phenyl-carbamoyl)-5-[4-(3-oxo-morpholin-4-yl)-phenylcarbamoyl]-pyrrolidin-3-yl-ester,
[1,3]Dioxolan-4,5-dicarbonsäure-4-[(4-chlorphenyl)-amid]-5-{[4-(3-oxo-morpholin-4-yl)-phenyl]-amid},
[1,3]Dioxolan-4,5-dicarbonsäure-4-[(4-chlorphenyl)-amid]-5-{[3-methyl-4-(3-oxo-morpholin-4-yl)-phenyl]-amid},
[1,3]Dioxolan-2,2-dimethyl-4,5-dicarbonsäure-4-[(4-chlorphenyl)-amid]-5-{[4-(3-oxo-morpholin-4-yl)-phenyl]-amid},
[1,3]Dioxolan-2,2-dimethyl-4,5-dicarbonsäure-4-[(4-chlorphenyl)-amid]-5-{[3-methyl-4-(3-oxo-morpholin-4-yl)-phenyl]-amid},
1-BOC-piperazin-1,2-dicarbonsäure-1-[4-chlorphenyl)-amid]-2-{[4-(3-oxo-morpholin-4-yl)-phenyl]-amid},
Piperazin-1,2-dicarbonsäure-1-[4-chlorphenyl)-amid]-2-{[4-(3-oxo-morpholin-4-yl)-phenyl]-amid},
[1,3]Oxazinan-3,4-dicarbonsäure-1-[4-chlorphenyl)-amid]-2-{[4-(3-oxo-morpholin-4-yl)-phenyl]-amid},
(2R,4S)-4-Ethinyl-4-hydroxy-pyrrolidin-1,2-dicarbonsäure-1-[(4-chlor-phenyl)-amid]-2-{[4-(3-oxo-morpholin-4-yl)-phenyl]-amid},
4-Oxa-6-aza-spiro[2.4]heptan-6,7-dicarbonsäure-6-[(4-chlorphenyl)-amid]-7-{[4-(3-oxo-morpholin-4-yl)-phenyl]-amid},
(2R,4S)-4-Acetamino-pyrrolidin-1,2-dicarbonsäure-1-[(4-chlor-phenyl)-amid]-2-{[3-methyl-4-(3-oxo-morpholin-4-yl)-phenyl]-amid},
(2R,4S)-4-Butylsulfonylamino-pyrrolidin-1,2-dicarbonsäure- 1-[(4-chlor-phenyl)-amid]-2-{[4-(3-oxo-morpholin-4-yl)-phenyl]-amid},
(R)-4-Oxo-pyrrolidin-1,2-dicarbonsäure-1-[(4-chloro-phenyl)-amid]-2-{[4-(3-oxo-morpholin-4-yl)-phenyl]-amid},
(2R,4S)-4-Amino-pyrrolidin-1,2-dicarbonsäure-1-[(4-chlor-phenyl)-amid]-2-{[3-methyl-4-(3-oxo-morpholin-4-yl)-phenyl]-amid},
(S)-Pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[2-fluor-4-(3-oxo-morpholin-4-yl)-phenyl]-amid},
(2R,4R)-1-[2-(4-Chlor-phenyl)-acetyl]-4-hydroxy-pyrrolidin-2-carbonsäure-[4-(3-oxo-morpholin-4-yl)-phenyl]-amid,
(2R,4R)-1-(4-Chlor-benzoyl)-4-hydroxy-pyrrolidin-2-carbonsäure-[4-(3-oxo-morpholin-4-yl)-phenyl]-amid,
(2R,4R)-4-Methoxy-pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Methoxy-pyrrolidin-1,2-dicarbonsäure-1-[(4-chlor-phenyl)-amid]-2-{[2-fluor-4-(3-oxo-morpholin-4-yl)-phenyl]-amid},
(2R,4S)-4-(2-Methylpropanoyl-amino)-pyrrolidin-1,2-dicarbonsäure-1-[(4-chlor-phenyl)-amid]-2-{[4-(3-oxo-morpholin-4-yl)-phenyl]-amid},
(2R,4R)-4-Ethoxy-pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Ethoxy-pyrrolidin-1,2-dicarbonsäure-1-[(4-chlor-phenyl)-amid]-2-{[2-fluor-4-(3-oxo-morpholin-4-yl)-phenyl]-amid},
(R)-Pyrrolidin-1,2-dicarbonsäure-2-[(4-chlorphenyl)-amide]-1-{[4-(3-oxo-morpholin-4-yl)-phenyl]-amid},
(S)-Pyrrolidin-1,2-dicarbonsäure-2-[(4-chlorphenyl)-amide]-1-{[4-(3-oxo-morpholin-4-yl)-phenyl]-amid},
(R)-1-{2-[4-(3-Oxo-morpholin-4-yl)-phenyl]-acetyl}-pyrrolidin-2-carbonsäure-(4-chlorphenyl)-amid,
(S)-1-{2-[4-(3-Oxo-morpholin-4-yl)-phenyl]-acetyl}-pyrrolidin-2-carbonsäure-(4-chlorphenyl)-amid,
(2R,4R)-1-{2-[4-(3-Oxo-morpholin-4-yl)-phenyl]-acetyl}-4-methoxy-pyrrolidin-2-carbonsäure-(4-chlorphenyl)-amid,
(2R,4S)-1-{2-[4-(3-Oxo-morpholin-4-yl)-phenyl]-acetyl}-4-methoxy-pyrrolidin-2-carbonsäure-(4-chlorphenyl)-amid,
(2S,4R)-1-{2-[4-(3-Oxo-morpholin-4-yl)-phenyl]-acetyl}-4-methoxy-pyrrolidin-2-carbonsäure-(4-chlorphenyl)-amid,
(2R,4R)-4-Ethoxy-pyrrolidin-1,2-dicarbonsäure-1-[(4-chlor-phenyl)-amid]-2-{[3-fluor-4-(3-oxo-morpholin-4-yl)-phenyl]-amid},
(2R,4R)-4-(Prop-2-in-yloxy)-pyrrolidin-1,2-dicarbonsäure-1-[(4-chlor-phenyl)-amid]-2-{[4-(3-oxo-morpholin-4-yl)-phenyl]-amid},
(2R,4R)-4-(But-2-in-yloxy)-pyrrolidin-1,2-dicarbonsäure-1-[(4-chlor-phenyl)-amid]-2-{[4-(3-oxo-morpholin-4-yl)-phenyl]-amid},
(2R,4R)-4-(2,3-Dihydroxypropoxy)-pyrrolidin-1,2-dicarbonsäure-1-[(4-chlor-phenyl)-amid]-2-{[4-(3-oxo-morpholin-4-yl)-phenyl]-amid},
(2R,4R)-4-(2-Hydroxy-3-pyrrolidin-1-ylpropoxy))-pyrrolidin-1,2-dicarbonsäure-1-[(4-chlor-phenyl)-amid]-2-{[4-(3-oxo-morpholin-4-yl)-phenyl]-amid},
(2R,4R)-4-(2-Oxo-oxazolidin-5-ylmethoxy)-pyrrolidin-1,2-dicarbonsäure-1-[(4-chlor-phenyl)-amid]-2-{[4-(3-oxo-morpholin-4-yl)-phenyl]-amid},
(2R,4R)-4-(3-Amino-2-hydroxy-propoxy)-pyrrolidin-1,2-dicarbonsäure-1-[(4-chlor-phenyl)-amid]-2-{[4-(3-oxo-morpholin-4-yl)-phenyl]-amid},
(R)-2,5-Dihydro-pyrrol-1,2-dicarbonsäure-1-[(4-chlor-phenyl)-amid]-2-{[3-fluor-4-(3-oxo-morpholin-4-yl)-phenyl]-amid},
(R)-2,5-Dihydro-pyrrol-1,2-dicarbonsäure-1-[(4-chlor-phenyl)-amid]-2-{[2-fluor-4-(3-oxo-morpholin-4-yl)-phenyl]-amid},
(2S,3S)-3-Hydroxy-pyrrolidin-1,2-dicarbonsäure-1-[(4-chlor-phenyl)-amid]-2-{[4-(3-oxo-morpholin-4-yl)-phenyl]-amid},
(2S,4S)-4-Hydroxy-pyrrolidin-1,2-dicarbonsäure-1-[(4-chlor-phenyl)-amid]-2-{[4-(3-oxo-morpholin-4-yl)-phenyl]-amid},
(2R,4R)-3-Hydroxy-pyrrolidin-1,2-dicarbonsäure-1-[(4-chlor-phenyl)-amid]-2-{[2-carboxy-4-(3-oxo-morpholin-4-yl)-phenyl]-amid},
(2R, 3S, 4R)-3,4-Dihydroxy-pyrrolidin-1,2-dicarbonsäure-1-[(4-chlor-phenyl)-amid]-2-{[4-(3-oxo-morpholin-4-yl)-phenyl]-amid},
(2R,4R)-4-Allyloxy-pyrrolidin-1,2-dicarbonsäure-1-[(4-chlor-phenyl)-amid]-2-{[2-fluor-4-(3-oxo-morpholin-4-yl)-phenyl]-amid},
(2R,4R)-4-(Prop-2-in-yloxy)-pyrrolidin-1,2-dicarbonsäure-1-[(4-chlor-phenyl)-amid]-2-{[2-fluor-4-(3-oxo-morpholin-4-yl)-phenyl]-amid},
(2R,4S)-4-(Prop-2-in-yloxy)-pyrrolidin-1,2-dicarbonsäure-1-[(4-chlor-phenyl)-amid]-2-{[2-fluor-4-(3-oxo-morpholin-4-yl)-phenyl]-amid},
(2R,4R)-4-(Methoxycarbonylmethoxy)-pyrrolidin-1,2-dicarbonsäure-1-[(4-chlor-phenyl)-amid]-2-{[4-(3-oxo-morpholin-4-yl)-phenyl]-amid},
(2R,4R)-4-(Carboxymethoxy)-pyrrolidin-1,2-dicarbonsäure-1-[(4-chlor-phenyl)-amid]-2-{[4-(3-oxo-morpholin-4-yl)-phehyl]-amid},
(2R,4R)-4-Methoxy-pyrrolidin-1,2-dicarbonsäure-1-[(4-brom-phenyl)-amid]-2-{[2-fluor-4-(3-oxo-morpholin-4-yl)-phenyl]-amid},
(2R,4R)-4-(2,3-Dihydroxy-propoxy)-pyrrolidin-1,2-dicarbonsäure-1-[(4-chlor-phenyl)-amid] 2-{[2-fluor-4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[2-aminocarbonyl-4-(3-oxo-morpholin-4-yl)-phenyl]-amid}, ESI 502;
(2R,4R)-4-Hydroxy-2-methyl-pyrrolidin-1,2-dicarbonsäure-1-[(4-chlor-phenyl)-amid]-2-{[4-(3-oxo-morpholin-4-yl)-Phenyl]-amid},
Piperidin-1,3-dicarbonsäure-1-[(4-chlor-phenyl)-amid]-3-{[4-(3-oxo-morpholin-4-yl)-phenyl]-amid},
Piperidin-1,3-dicarbonsäure-1-[(4-chlor-phenyl)-amid]-3-{[3-methyl-4-(3-oxo-morpholin-4-yl)-phenyl]-amid},
(2R,4R)-4-(2-Methoxy-ethoxy)-pyrrolidin-1,2-dicarbonsäure-1-[(4-chlor-phenyl)-amid]-2-{[4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[2-methyl-4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[2-(3-oxo-morpholin-4-yl)-phenyl]-amid},
sowie ihre pharmazeutisch verwendbaren Solvate, Salze und Stereoisomere, einschließlich deren Mischungen in allen Verhältnissen.

3. Verfahren zur Herstellung von Verbindungen der Formel I nach Anspruch 1 oder 2 sowie ihrer pharmazeutisch verwendbaren Solvate, Salze und Stereoisomere, **dadurch gekennzeichnet, daß** man
zur Herstellung von Verbindungen der Formel I, worin
W N und
G NH bedeuten,
eine Verbindung der Formel II 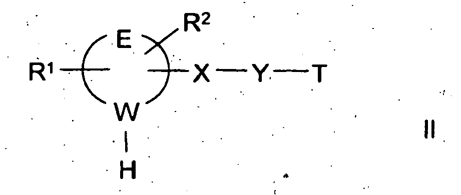 worin
R¹, R², E, X, Y und T die in Anspruch 1 angegebene Bedeutung haben,
und W N bedeutet,
mit einer Verbindung der Formel III
D-N=C=O III
worin
D die in Anspruch 1 angegebene Bedeutung hat,
umsetzt,
und/oder
eine Base oder Säure der Formel I in eines ihrer Salze umwandelt.

4. Verbindungen der Formel I nach Anspruch 1 oder 2 als Inhibitoren des Koagulationsfaktors Xa.

5. Verbindungen der Formel I nach Anspruch 1 oder 2 als Inhibitoren des Koagulationsfaktors VIIa.

6. Arzneimittel, enthaltend mindestens eine Verbindung der Formel I nach Anspruch 1 oder 2 und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

7. Arzneimittel enthaltend mindestens eine Verbindung der Formel I gemäß Anspruch 1 oder 2 und/oder ihre pharmazeutisch verwendbaren Solvate und Stereoisomere, einschließlich deren Mischungen in allen Verhältnissen, und mindestens einen weiteren Arzneimittelwirkstoff.

8. Verwendung von Verbindungen gemäß Anspruch 1 oder 2 und/oder ihre physiologisch unbedenklichen Salze, Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

9. Set (Kit), bestehend aus getrennten Packungen von
(a) einer wirksamen Menge an einer Verbindung der Formel I gemäß Anspruch 1 oder 2 und/oder ihrer pharmazeutisch verwendbaren Solvate, Salze und Stereoisomere, einschließlich deren Mischungen in allen Verhältnissen,
und
(b) einer wirksamen Menge eines weiteren Arzneimittelswirkstoffs.

10. Verwendung von Verbindungen der Formel I gemäß Anspruch 1 oder 2 und/oder ihrer pharmazeutisch verwendbaren Solvate, Salze und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

11. Zwischenverbindungen, ausgewählt aus der Gruppe
(S)-Pyrrolidin-2-carbonsäure-*N*-[4-(3-oxo-morpholin-4-yl)-phenyl]-amid,
(R)-Pyrrolidin-2-carbonsäure-*N*-[4-(3-oxo-morpholin-4-yl)-phenyl]-amid,
(2R,4R)-4-Hydroxy-pyrrolidin-2-carbonsäure-*N*-[4-(3-oxo-morpholin-4-yl)-phenyl]-amid,
4-Hydroxy-pyrrolidin-2-carbonsäure-*N*-[4-(3-oxo-morpholin-4-yl)-phenyl]-amid,
(R)-4,4-Dimethoxy-pyrrolidin-2-carbonsäure-*N*-[4-(3-oxo-morpholin-4-yl)-phenyl]-amid,
(2R,4R)-4-Methoxy-pyrrolidin-2-carbonsäure-*N*-[4-(3-oxo-morpholin-4-yl)-phenyl]-amid,
sowie deren Isomere und Salze.

12. Arzneimittel nach Anspruch 7, enthaltend (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} und/oder seine pharmazeutisch verwendbaren Solvate, Salze und Stereoisomere, einschließlich deren Mischungen in allen Verhältnissen, und Aspirin.

13. Verwendung nach Anspruch 10, enthaltend (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} und/oder seine pharmazeutisch verwendbaren Solvate, Salze und Stereoisomere, einschließlich deren Mischungen in allen Verhältnissen, in Kombination mit Aspirin.

## Claims

1. Compounds of the formula I 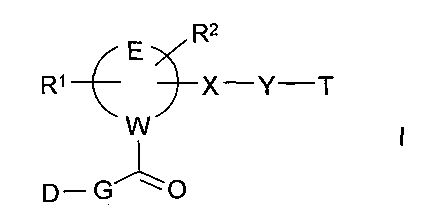 in which
D denotes phenyl, pyridyl or thienyl, each of which is mono- or disubstituted by Hal,
R¹ denotes H, =O, COOR³, OH, OA, NH₂, alkyl having 1, 2, 3, 4, 5 or 6 C atoms, N₃, ethynyl, vinyl, allyloxy, -OCOR³, NHCOA or NHSO₂A,
R² denotes H, =O, OH, OA or alkyl having 1, 2, 3, 4, 5 or 6 C atoms,
R¹ and R² together also denote a spirocyclically bonded 3- to 6-membered carbocycle,
R³ denotes H or A,
R⁴ denotes H or A, 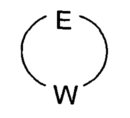 denotes pyrrolidine-1,2-diyl, piperidine-1,2-diyl, oxazolidine-3,4- or 3,5-diyl, thiazolidine-3,4-diyl, 2,5-dihydro-1*H*-pyrrole-1,5-diyl, 1,3-dioxolane-4,5-diyl, 1,3-oxazinane-3,4-diyl, piperazine-1,4-diyl, tetrahydrofuran-3,4-diyl or azetidine-1,2-diyl,
G denotes (CH₂)ₙ or (CH₂)ₙNH-,
X denotes CONH,
Y denotes 1,3- or 1,4-phenylene which is unsubstituted or mono- or disubstituted by methyl, trifluoromethyl, ethyl, propyl, Cl or F,
T denotes morpholin-4-yl which is mono- or disubstituted by carbonyl oxygen,
A denotes unbranched or branched alkyl having 1-10 C atoms and in which 1-7 H atoms may be replaced by F,
Hal denotes F, Cl, Br or I,
n denotes 0, 1 or 2,
and pharmaceutically usable solvates, salts and stereoisomers thereof, including mixtures thereof in all ratios.

2. Compounds according to Claim 1, selected from the group
1-N-(4-chlorophenyl)-2-N-[4-(3-oxomorpholin-4-yl)phenyl]-(R)-pyrrolidine-1,2-dicarboxamide,
1-N-(4-chlorophenyl)-2-N-[3-methyl-4-(3-oxomorpholin-4-yl)-phenyl]-(R)-pyrrolidine-1,2-dicarboxamide,
1-N-(4-chlorophenyl)-2-N-[3-fluoro-4-(3-oxomorpholin-4-yl)-phenyl]-(R)-pyrrolidine-1,2-dicarboxamide,
1-N-(4-chlorophenyl)-2-N-[2-fluoro-4-(3-oxomorpholin-4-yl)-phenyl]-(R)-pyrrolidine-1,2-dicarboxamide,
1-N-(4-chlorophenyl)-2-N-[3-trifluoromethyl-4-(3-oxomorpholin-4-yl)phenyl]-(R)-pyrrolidine-1,2-dicarboxamide,
1-N-(4-chlorophenyl)-2-N-[3-methyl-4-(3-oxomorpholin-4-yl)-phenyl]-(R)-piperidine-1,2-dicarboxamide,
1-N-(4-chlorophenyl)-2-N-[4-(3-oxomorpholin-4-yl)phenyl]-(R)-2,5-dihydropyrrole-1,2-dicarboxamide,
N-[4-(3-oxomorpholin-4-yl)phenyl]-(R)-1-(5-chlorothiophene-2-carbonyl)pyrrolidine-2-carboxamide,
N-[3-methyl-4-(3-oxomorpholin-4-yl)phenyl]-(R)-1-(5-chlorothiophene-2-carbonyl)pyrrolidine-2-carboxamide,
3-N-(4-chlorophenyl)-4-N-[4-(3-oxomorpholin-4-yl)phenyl]-(R)-oxazolidine-3,4-dicarboxamide,
3-N-(4-chlorophenyl)-4-N-[3-methyl-4-(3-oxomorpholin-4-yl)-phenyl]-(R)-oxazolidine-3,4-dicarboxamide,
3-N-(4-chlorophenyl)-4-N-[4-(3-oxomorpholin-4-yl)phenyl]-(4R,5S)-5-methyloxazolidine-3,4-dicarboxamide,
3-N-(4-chlorophenyl)-4-N-[3-methyl-4-(3-oxomorpholin-4-yl)-phenyl]-(4R,5S)-5-methyloxazolidine-3,4-dicarboxamide,
3-N-(4-chlorophenyl)-4-N-[3-fluoro-4-(3-oxomorpholin-4-yl)-phenyl]-(4R,5S)-5-methyloxazolidine-3,4-dicarboxamide,
3-N-(4-chlorophenyl)-4-N-[3-chloro-4-(3-oxomorpholin-4-yl)-phenyl]-(4R,5S)-5-methyloxazolidine-3,4-dicarboxamide,
3-N-(4-chlorophenyl)-4-N-[3-methyl-4-(3-oxomorpholin-4-yl)-phenyl]-(4R,5R)-5-methyloxazolidine-3,4-dicarboxamide,
3-N-(4-chlorophenyl)-4-N-[4-(3-oxomorpholin-4-yl)phenyl]-(S)-thiazolidine-3,4-dicarboxamide,
3-N-(4-chlorophenyl)-4-N-[4-(3-oxomorpholin-4-yl)phenyl]-(S)-1,1-dioxo-1λ⁶-thiazolidine-3,4-dicarboxamide,
3-N-(4-chlorophenyl)-4-N-[3-methyl-4-(3-oxomorpholin-4-yl)-phenyl]-(S)-thiazolidine-3,4-dicarboxamide,
3-N-(4-chlorophenyl)-4-N-[3-methyl-4-(3-oxomorpholin-4-yl)-phenyl]-(S)-1,1-dioxo-1λ⁶-thiazolidine-3,4-dicarboxamide,
N-[4-(3-oxomorpholin-4-yl)phenyl]-3-(5-chlorothiophene-2-carbonyl)oxazolidine-5-carboxamide,
N-[3-methyl-4-(3-oxomorpholin-4-yl)phenyl]-3-(5-chlorothiophene-2-carbonyl)oxazolidine-5-carboxamide,
1-N-(5-chloropyridin-2-yl)-2-N-[4-(3-oxomorpholin-4-yl)phenyl]-(2R,4R)-4-hydroxypyrrolidine-1,2-dicarboxamide,
1-N-(5-chloropyridin-2-yl)-2-N-[4-(3-oxomorpholin-4-yl)phenyl]-(R)-4,4-dimethoxypyrrolidine-1,2-dicarboxamide,
1-N-(4-chlorophenyl)-2-N-[4-(3-oxomorpholin-4-yl)phenyl]-(2R,4R)-4-hydroxypyrrolidine-1,2-dicarboxamide,
1-N-(4-chlorophenyl)-2-N-[3-methyl-4-(3-oxomorpholin-4-yl)-phenyl]-(2R,4R)-4-hydroxypyrrolidine-1,2-dicarboxamide,
1-N-(4-chlorophenyl)-2-N-[2-fluoro-4-(3-oxomorpholin-4-yl)-phenyl]-(2R,4R)-4-hydroxypyrrolidine-1,2-dicarboxamide,
1-N-(4-chlorophenyl)-2-N-[3-fluoro-4-(3-oxomorpholin-4-yl)-phenyl]-(2R,3R)-3-hydroxypyrrolidine-1,2-dicarboxamide,
1-N-(4-chlorophenyl)-2-N-[3-fluoro-4-(3-oxomorpholin-4-yl)-phenyl]-(2R,3S)-3-hydroxypyrrolidine-1,2-dicarboxamide,
1-N-(4-chlorophenyl)-2-N-[4-(3-oxomorpholin-4-yl)phenyl]-(2R,4S)-4-hydroxypyrrolidine-1,2-dicarboxamide,
1-N-(4-chlorophenyl)-2-N-[4-(3-oxomorpholin-4-yl)phenyl]-(2S,4R)-4-hydroxypyrrolidine-1,2-dicarboxamide,
1-N-(4-chlorophenyl)-2-N-[4-(3-oxomorpholin-4-yl)phenyl]-3,4-dihydroxypyrrolidine-1,2-dicarboxamide,
1-N-(4-chlorophenyl)-2-N-[4-(3-oxomorpholin-4-yl)phenyl]-(2R,4S)-4-azidopyrrolidine-1,2-dicarboxamide,
1-N-(4-chlorophenyl)-2-N-[4-(3-oxomorpholin-4-yl)phenyl]-(2R,4S)-4-aminopyrrolidine-1,2-dicarboxamide,
1-N-(4-chlorophenyl)-2-N-[4-(3-oxomorpholin-4-yl)phenyl]-(2R,4R)-4-azidopyrrolidine-1,2-dicarboxamide,
1-N-(4-chlorophenyl)-2-N-[4-(3-oxomorpholin-4-yl)phenyl]-(2R,4R)-4-aminopyrrolidine-1,2-dicarboxamide,
1-N-(4-chlorophenyl)-2-N-[4-(3-oxomorpholin-4-yl)phenyl]-(2R,4S)-4-acetaminopyrrolidine-1,2-dicarboxamide,
1-N-(4-chlorophenyl)-2-N-[4-(3-oxomorpholin-4-yl)phenyl]-(2R,4R)-4-acetaminopyrrolidine-1,2-dicarboxamide,
1-N-(4-chlorophenyl)-2-N-[4-(3-oxomorpholin-4-yl)phenyl]-(2R,4S)-4-methylsulfonylaminopyrrolidine-1,2-dicarboxamide,
1-N-(4-chlorophenyl)-2-N-[4-(3-oxomorpholin-4-yl)phenyl]-(2R,4R)-4-methylsulfonylaminopyrrolidine-1,2-dicarboxamide,
1-N-(4-chlorophenyl)-2-N-[4-(3-oxomorpholin-4-yl)phenyl]-(2R,4R)-4-methoxypyrrolidine-1,2-dicarboxamide,
1-N-(4-chlorophenyl)-2-N-[4-(3-oxomorpholin-4-yl)phenyl]-(2R,4R)-4-ethoxypyrrolidine-1,2-dicarboxamide,
1-N-(4-chlorophenyl)-2-N-[4-(3-oxomorpholin-4-yl)phenyl]-(2R,4R)-4-propoxypyrrolidine-1,2-dicarboxamide,
1-N-(4-chlorophenyl)-2-N-[4-(3-oxomorpholin-4-yl)phenyl]-(2R,4R)-4-allyloxypyrrolidine-1,2-dicarboxamide,
(3R,5R)-1-(4-chlorophenylcarbamoyl)-5-[4-(3-oxomorpholin-4-yl)phenylcarbamoyl]pyrrolidin-3-yl isobutyrate,
(3R,5R)-1-(4-chlorophenylcarbamoyl)-5-[4-(3-oxomorpholin-4-yl)phenylcarbamoyl]pyrrolidin-3-yl propionate,
(3R,5R)-1-(4-chlorophenylcarbamoyl)-5-[4-(3-oxomorpholin-4-yl)phenylcarbamoyl]pyrrolidin-3-yl acetate,
4-N-(4-chlorophenyl)-5-N-[4-(3-oxomorpholin-4-yl)phenyl]-1,3-dioxolane-4,5-dicarboxamide,
4-N-(4-chlorophenyl)-5-N-[3-methyl-4-(3-oxomorpholin-4-yl)-phenyl]-1,3-dioxolane-4,5-dicarboxamide,
4-N-(4-chlorophenyl)-5-N-[4-(3-oxomorpholin-4-yl)phenyl]-1,3-dioxolane-2,2-dimethyl-4,5-dicarboxamide,
4-N-(4-chlorophenyl)-5-N-[3-methyl-4-(3-oxomorpholin-4-yl)-phenyl]-1,3-dioxolane-2,2-dimethyl-4,5-dicarboxamide,
1-N-(4-chlorophenyl)-2-N-[4-(3-oxomorpholin-4-yl)phenyl]-1-BOC-piperazine-1,2-dicarboxamide,
1-N-(4-chlorophenyl)-2-N-[4-(3-oxomorpholin-4-yl)phenyl]-piperazine-1,2-dicarboxamide,
1-N-(4-chlorophenyl)-2-N-[4-(3-oxomorpholin-4-yl)phenyl]-1,3-oxazinane-3,4-dicarboxamide,
1-N-(4-chlorophenyl)-2-N-[4-(3-oxomorpholin-4-yl)phenyl]-(2R,4S)-4-ethynyl-4-hydroxypyrrolidine-1,2-dicarboxamide,
6-N-(4-chlorophenyl)-7-N-[4-(3-oxomorpholin-4-yl)phenyl]-4-oxa-6-azaspiro[2.4]heptane-6,7-dicarboxamide,
1-N-(4-chlorophenyl)-2-N-[3-methyl-4-(3-oxomorpholin-4-yl)-phenyl]-(2R,4S)-4-acetaminopyrrolidine-1,2-dicarboxamide,
1-N-(4-chlorophenyl)-2-N-[4-(3-oxomorpholin-4-yl)phenyl]-(2R,4S)-4-butylsulfonylaminopyrrolidine-1,2-dicarboxamide,
1-N-(4-chlorophenyl)-2-N-[4-(3-oxomorpholin-4-yl)phenyl]-(R)-4-oxopyrrolidine-1,2-dicarboxamide,
1-N-(4-chlorophenyl)-2-N-[3-methyl-4-(3-oxomorpholin-4-yl)-phenyl]-(2R,4S)-4-aminopyrrolidine-1,2-dicarboxamide,
1-N-(4-chlorophenyl)-2-N-[3-methyl-4-(3-oxomorpholin-4-yl)-phenyl]-(S)-pyrrolidine-1,2-dicarboxamide,
1-N-(4-chlorophenyl)-2-N-[2-fluoro-4-(3-oxomorpholin-4-yl)-phenyl]-(2R,4R)-4-hydroxypyrrolidine-1,2-dicarboxamide,
N-[4-(3-oxomorpholin-4-yl)phenyl]-(2R,4R)-1-[2-(4-chlorophenyl)acetyl]-4-hydroxypyrrolidine-2-carboxamide,
N-[4-(3-oxomorpholin-4-yl)phenyl]-(2R,4R)-1-(4-chlorobenzoyl)-4-hydroxypyrrolidine-2-carboxamide,
1-N-(4-chlorophenyl)-2-N-[3-methyl-4-(3-oxomorpholin-4-yl)-phenyl]-(2R,4R)-4-methoxypyrrolidine-1,2-dicarboxamide,
1-N-(4-chlorophenyl)-2-N-[2-fluoro-4-(3-oxomorpholin-4-yl)-phenyl]-(2R,4R)-4-methoxypyrrolidine-1,2-dicarboxamide,
1-N-(4-chlorophenyl)-2-N-[4-(3-oxomorpholin-4-yl)phenyl]-(2R,4S)-4-(2-methylpropanoylamino)pyrrolidine-1,2-dicarboxamide,
1-N-(4-chlorophenyl)-2-N-[3-methyl-4-(3-oxomorpholin-4-yl)-phenyl]-(2R,4R)-4-ethoxypyrrolidine-1,2-dicarboxamide,
1-N-(4-chlorophenyl)-2-N-[2-fluoro-4-(3-oxomorpholin-4-yl)-phenyl]-(2R,4R)-4-ethoxypyrrolidine-1,2-dicarboxamide,
2-N-(4-chlorophenyl)-1-N-[4-(3-oxomorpholin-4-yl)phenyl]-(R)-pyrrolidine-1,2-dicarboxamide,
2-N-(4-chlorophenyl)-1-N-[4-(3-oxomorpholin-4-yl)phenyl]-(S)-pyrrolidine-1,2-dicarboxamide,
N-(4-chlorophenyl)-(R)-1-{2-[4-(3-oxomorpholin-4-yl)phenyl]-acetyl}pyrrolidine-2-carboxamide,
N-(4-chlorophenyl)-(S)-1-{2-[4-(3-oxomorpholin-4-yl)phenyl]-acetyl}pyrrolidine-2-carboxamide,
N-(4-chlorophenyl)-(2R,4R)-1-{2-[4-(3-oxomorpholin-4-yl)-phenyl]acetyl}-4-methoxypyrrolidine-2-carboxamide,
N-(4-chlorophenyl)-(2R,4S)-1-{2-[4-(3-oxomorpholin-4-yl)-phenyl]acetyl}-4-methoxypyrrolidine-2-carboxamide,
N-(4-chlorophenyl)-(2S,4R)-1-{2-[4-(3-oxomorpholin-4-yl)-phenyl]acetyl}-4-methoxypyrrolidine-2-carboxamide,
1-N-(4-chlorophenyl)-2-N-[3-fluoro-4-(3-oxomorpholin-4-yl)-phenyl]-(2R,4R)-4-ethoxypyrrolidine-1,2-dicarboxamide,
1-N-(4-chlorophenyl)-2-N-[4-(3-oxomorpholin-4-yl)phenyl]-(2R,4R)-4-(prop-2-ynyloxy)pyrrolidine-1,2-dicarboxamide,
1-N-(4-chlorophenyl)-2-N-[4-(3-oxomorpholin-4-yl)phenyl]-(2R,4R)-4-(but-2-ynyloxy)pyrrolidine-1,2-dicarboxamide,
1-N-(4-chlorophenyl)-2-N-[4-(3-oxomorpholin-4-yl)phenyl]-(2R,4R)-4-(2,3-dihydroxypropoxy)pyrrolidine-1,2-dicarboxamide,
1-N-(4-chlorophenyl)-2-N-[4-(3-oxomorpholin-4-yl)phenyl]-(2R,4R)-4-(2-hydroxy-3-pyrrolidin-1-ylpropoxy)pyrrolidine-1,2-dicarboxamide,
1-N-(4-chlorophenyl)-2-N-[4-(3-oxomorpholin-4-yl)phenyl]-(2R,4R)-4-(2-oxooxazolidin-5-ylmethoxy)pyrrolidine-1,2-dicarboxamide,
1-N-(4-chlorophenyl)-2-N-[4-(3-oxomorpholin-4-yl)phenyl]-(2R,4R)-4-(3-amino-2-hydroxypropoxy)pyrrolidine-1,2-dicarboxamide,
1-N-(4-chlorophenyl)-2-N-[3-fluoro-4-(3-oxomorpholin-4-yl)-phenyl]-(R)-2,5-dihydropyrrole-1,2-dicarboxamide,
1-N-(4-chlorophenyl)-2-N-[2-fluoro-4-(3-oxomorpholin-4-yl)-phenyl]-(R)-2,5-dihydropyrrole-1,2-dicarboxamide,
1-N-(4-chlorophenyl)-2-N-[4-(3-oxomorpholin-4-yl)phenyl]-(2S,3S)-3-hydroxypyrrolidine-1,2-dicarboxamide,
1-N-(4-chlorophenyl)-2-N-[4-(3-oxomorpholin-4-yl)phenyl]-(2S,4S)-4-hydroxypyrrolidine-1,2-dicarboxamide,
1-N-(4-chlorophenyl)-2-N-[2-carboxy-4-(3-oxomorpholin-4-yl)-phenyl]-(2R,4R)-3-hydroxypyrrolidine-1,2-dicarboxamide,
1-N-(4-chlorophenyl)-2-N-[4-(3-oxomorpholin-4-yl)phenyl]-(2R,3S,4R)-3,4-dihydroxypyrrolidine-1,2-dicarboxamide,
1-N-(4-chlorophenyl)-2-N-[2-fluoro-4-(3-oxomorpholin-4-yl)-phenyl]-(2R,4R)-4-allyloxypyrrolidine-1,2-dicarboxamide,
1-N-(4-chlorophenyl)-2-N-[2-fluoro-4-(3-oxomorpholin-4-yl)-phenyl]-(2R,4R)-4-(prop-2-ynyloxy)pyrrolidine-1,2-dicarboxamide,
1-N-(4-chlorophenyl)-2-N-[2-fluoro-4-(3-oxomorpholin-4-yl)-phenyl]-(2R,4S)-4-(prop-2-ynyloxy)pyrrolidine-1,2-dicarboxamide,
1-N-(4-chlorophenyl)-2-N-[4-(3-oxomorpholin-4-yl)phenyl]-(2R,4R)-4-(methoxycarbonylmethoxy)pyrrolidine-1,2-dicarboxamide,
1-N-(4-chlorophenyl)-2-N-[4-(3-oxomorpholin-4-yl)phenyl]-(2R,4R)-4-(carboxymethoxy)pyrrolidine-1,2-dicarboxamide,
1-N-(4-bromophenyl)-2-N-[2-fluoro-4-(3-oxomorpholin-4-yl)-phenyl]-(2R,4R)-4-methoxypyrrolidine-1,2-dicarboxamide,
1-N-(4-chlorophenyl)-2-N-[2-fluoro-4-(3-oxomorpholin-4-yl)-phenyl]-(2R,4R)-4-(2,3-dihydroxypropoxy)pyrrolidine-1,2-dicarboxamide,
1-N-(4-chlorophenyl)-2-N-[2-aminocarbonyl-4-(3-oxomorpholin-4-yl)phenyl]-(2R,4R)-4-hydroxypyrrolidine-1,2-dicarboxamide, ESI 502;
1-N-(4-chlorophenyl)-2-N-[4-(3-oxomorpholin-4-yl)phenyl]-(2R,4R)-4-hydroxy-2-methylpyrrolidine-1,2-dicarboxamide,
1-N-(4-chlorophenyl)-3-N-[4-(3-oxomorpholin-4-yl)phenyl]-piperidine-1,3-dicarboxamide,
1-N-(4-chlorophenyl)-3-N-[3-methyl-4-(3-oxomorpholin-4-yl)-phenyl]piperidine-1,3-dicarboxamide,
1-N-(4-chlorophenyl)-2-N-[4-(3-oxomorpholin-4-yl)phenyl]-(2R,4R)-4-(2-methoxyethoxy)pyrrolidine-1,2-dicarboxamide,
1-N-(4-chlorophenyl)-2-N-[2-methyl-4-(3-oxomorpholin-4-yl)-phenyl]-(2R,4R)-4-hydroxypyrrolidine-1,2-dicarboxamide,
1-N-(4-chlorophenyl)-2-N-[4-(3-oxomorpholin-4-yl)phenyl]-(2R,4R)-4-hydroxypyrrolidine-1,2-dicarboxamide,
1-N-(4-chlorophenyl)-2-N-[2-(3-oxomorpholin-4-yl)phenyl]-(2R,4R)-4-hydroxypyrrolidine-1,2-dicarboxamide,
and pharmaceutically usable solvates, salts and stereoisomers thereof, including mixtures thereof in all ratios.

3. Process for the preparation of compounds of the formule I according to Claim 1 or 2 and pharmaceutically usable solvates, salts and stereoisomers thereof, **characterised in that**
for the preparation of compounds of the formula I in which
W denotes N and
G denotes NH,
a compound of the formula II 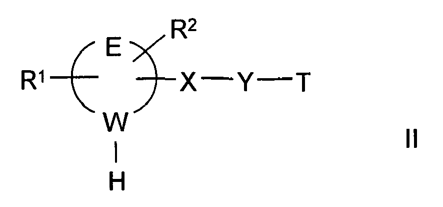 in which
R¹, R², E, X, Y and T have the meaning indicated in Claim 1,
and W denotes N,
is reacted with a compound of the formula III
D-N=C=O III
in which
D has the meaning indicated in Claim 1,
and/or
a base or acid of the formula I is converted into one of its salts.

4. Compounds of the formula I according to Claim 1 or 2 as inhibitors of coagulation factor Xa.

5. Compounds of the formula I according to Claim 1 or 2 as inhibitors of coagulation factor VIIa.

6. Medicaments comprising at least one compound of the formula I according to Claim 1 or 2 and/or pharmaceutically usable solvates, salts and stereoisomers thereof, including mixtures thereof in all ratios, and optionally excipients and/or adjuvants.

7. Medicaments comprising at least one compound of the formula I according to Claim 1 or 2 and/or pharmaceutically usable solvates and stereoisomers thereof, including mixtures thereof in all ratios, and at least one further medicament active compound.

8. Use of compounds according to Claim 1 or 2 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

9. Set (kit) consisting of separate packs of
(a) an effective amount of a compound of the formula I according to Claim 1 or 2 and/or pharmaceutically usable solvates, salts and stereoisomers thereof, including mixtures thereof in all ratios,
and
(b) an effective amount of a further medicament active compound.

10. Use of compounds of the formula I according to Claim 1 or 2 and/or pharmaceutically usable solvates, salts and stereoisomers thereof, including mixtures thereof in all ratios,
for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases, in combination with at least one further medicament active compound.

11. Intermediate compounds, selected from the group
*N*-[4-(3-oxomorpholin-4-yl)phenyl]-(S)-pyrrolidine-2-carboxamide,
*N*-[4-(3-oxomorpholin-4-yl)phenyl]-(R)-pyrrolidine-2-carboxamide,
*N*-[4-(3-oxomorpholin-4-yl)phenyl]-(2R,4R)-4-hydroxypyrrolidine-2-carboxamide,
*N*-[4-(3-oxomorpholin-4-yl)phenyl]-4-hydroxypyrrolidine-2-carboxamide,
*N*-[4-(3-oxomorpholin-4-yl)phenyl]-(R)-4,4-dimethoxypyrrolidine-2-carboxamide,
*N*-[4-(3-oxomorpholin-4-yl)phenyl]-(2R,4R)-4-methoxypyrrolidine-2-carboxamide,
and isomers and salts thereof.

12. Medicaments according to Claim 7, comprising 1-N-(4-chlorophenyl)-2-N-[4-(3-oxomorpholin-4-yl)phenyl]-(2R,4R)-4-hydroxypyrrolidine-1,2-dicarboxamide and/or pharmaceutically usable solvates, salts and stereoisomers thereof, including mixtures thereof in all ratios, and aspirin.

13. Use according to Claim 10, comprising 1-N-(4-chlorophenyl)-2-N-[4-(3-oxomorpholin-4-yl)phenyl]-(2R,4R)-4-hydroxypyrrolidine-1,2-dicarboxamide and/or pharmaceutically usable solvates, salts and stereoisomers thereof, including mixtures thereof in all ratios, in combination with aspirin.

## Revendications

1. Composés de formule I 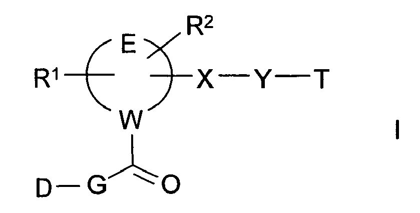 dans laquelle
D désigne phényle, pyridyle ou thiényle, chacun d'entre eux étant mono- ou disubstitué par Hal,
R¹ désigne H, =O, COOR³, OH, OA, NH₂, alkyle ayant 1, 2, 3, 4, 5 ou 6 atomes de C, N₃, éthynyle, vinyle, allyloxy, -OCOR³, NHCOA ou NHSO₂A,
R² désigne H, =O, OH, OA ou alkyle ayant 1, 2, 3, 4, 5 ou 6 atomes de C,
R¹ et R² désignent également ensemble un carbocycle de 3 à 6 chaînons lié de manière spirocyclique,
R³ désigne H ou A,
R⁴ désigne H ou A, 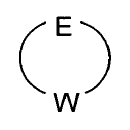 désigne pyrrolidine-1,2-diyle, pipéridine-1,2-diyle, oxazolidine-3,4- ou 3,5-diyle, thiazolidine-3,4-diyle, 2,5-dihydro-1H-pyrrole-1,5-diyle, 1,3-dioxolane-4,5-diyle, 1,3-oxazinane-3,4-diyle, pipérazine-1,4-diyle, tétrahydrofuran-3,4-diyle ou azétidine-1,2-diyle,
G désigne (CH₂)ₙ ou (CH₂)ₙNH-,
X désigne CONH,
Y désigne 1,3- ou 1,4-phénylène qui est non substitué ou mono- ou disubstitué par méthyle, trifluorométhyle, éthyle, propyle, Cl ou F,
T désigne morpholin-4-yle qui est mono- ou disubstitué par oxygène carbonyle,
A désigne alkyle non ramifié ou ramifié ayant 1-10 atomes de C et où 1-7 atomes de H peuvent être remplacés par F,
Hal désigne F, Cl, Br ou I,
n désigne 0, 1 ou 2,
et les solvats, sels et stéréoisomères pharmaceutiquement utilisables de ceux-ci, y compris des mélanges de ceux-ci en toutes proportions.

2. Composés selon la revendication 1, choisis parmi le groupe constitué par
le 1-N-(4-chlorophényl)-2-N-[4-(3-oxomorpholin-4-yl)phényl]-(R)-pyrrolidine-1,2-dicarboxamide,
le 1-N-(4-chlorophényl)-2-N-[3-méthyl-4-(3-oxomorpholin-4-yl)-phényl]-(R)-pyrrolidine-1,2-dicarboxamide,
le 1-N-(4-chlorophényl)-2-N-[3-fluoro-4-(3-oxomorpholin-4-yl)-phényl]-(R)-pyrrolidine-1,2-dicarboxamide,
le 1-N-(4-chlorophényl)-2-N-[2-fluoro-4-(3-oxomorpholin-4-yl)-phényl]-(R)-pyrrolidine-1,2-dicarboxamide,
le 1-N-(4-chlorophényl)-2-N-[3-trifluorométhyl-4-(3-oxomorpholin-4-yl)phényl]-(R)-pyrrolidine-1,2-dicarboxamide,
le 1-N-(4-chlorophényl)-2-N-[3-méthyl-4-(3-oxomorpholin-4-yl)-phényl]-(R)-pipéridine-1,2-dicarboxamide,
le 1-N-(4-chlorophényl)-2-N-[4-(3-oxomorpholin-4-yl)phényl]-(R)-2,5-dihydropyrrole-1,2-dicarboxamide,
le N-[4-(3-oxomorpholin-4-yl)phényl]-(R)-1-(5-chlorothiophène-2-carbonyl)pyrrolidine-2-carboxamide,
le N-[3-méthyl-4-(3-oxomorpholin-4-yl)phényl]-(R)-1-(5-chlorothiophène-2-carbonyl)pyrrolidine-2-carboxamide,
le 3-N-(4-chlorophényl)-4-N-[4-(3-oxomorpholin-4-yl)phényl]-(R)-oxazolidine-3,4-dicarboxamide,
le 3-N-(4-chlorophényl)-4-N-[3-méthyl-4-(3-oxomorpholin-4-yl)-phényl]-(R)-oxazolidine-3,4-dicarboxamide,
le 3-N-(4-chlorophényl)-4-N-[4-(3-oxomorpholin-4-yl)phényl]-(4R,5S)-5-méthyloxazolidine-3,4-dicarboxamide,
le 3-N-(4-chlorophényl)-4-N-[3-méthyl-4-(3-oxomorpholin-4-yl)-phényl]-(4R,5S)-5-méthyloxazolidine-3,4-dicarboxamide,
le 3-N-(4-chlorophényl)-4-N-[3-fluoro-4-(3-oxomorpholin-4-yl)phényl]-(4R,5S)-5-méthyloxazolidine-3,4-dicarboxamide,
le 3-N-(4-chlorophényl)-4-N-[3-chloro-4-(3-oxomorpholin-4-yl)-phényl]-(4R,5S)-5-méthyloxazolidine-3,4-dicarboxamide,
le 3-N-(4-chlorophényl)-4-N-[3-méthyl-4-(3-oxomorpholin-4-yl)-phényl]-(4R,5R)-5-méthyloxazolidine-3,4-dicarboxamide,
le 3-N-(4-chlorophényl)-4-N-[4-(3-oxomorpholin-4-yl)phényl]-(S)-thiazolidine-3,4-dicarboxamide,
le 3-N-(4-chlorophényl)-4-N-[4-(3-oxomorpholin-4-yl)phényl]-(S)-1,1-dioxo-1λ⁶-thiazolidine-3,4-dicarboxamide,
le 3-N-(4-chlorophényl)-4-N-[3-méthyl-4-(3-oxomorpholin-4-yl)-phényl]-(S)-thiazolidine-3,4-dicarboxamide,
le 3-N-(4-chlorophényl)-4-N-[3-méthyl-4-(3-oxomorpholin-4-yl)-phényl]-(S)-1,1-dioxo-1λ⁶-thiazolidine-3,4-dicarboxamide,
le N-[4-(3-oxomorpholin-4-yl)phényl]-3-(5-chlorothiophène-2-carbonyl)oxazolidine-5-carboxamide,
le N-[3-méthyl-4-(3-oxomorpholin-4-yl)phényl]-3-(5-chlorothiophène-2-carbonyl)oxazolidine-5-carboxamide,
le 1-N-(5-chloropyridin-2-yl)-2-N-[4-(3-oxomorpholin-4-yl)-phényl]-(2R,4R)-4-hydroxypyrrolidine-1,2-dicarboxamide,
le 1-N-(5-chloropyridin-2-yl)-2-N-[4-(3-oxomorpholin-4-yl)-phényl]-(R)-4,4-diméthoxypyrrolidine-1,2-dicarboxamide,
le 1-N-(4-chlorophényl)-2-N-[4-(3-oxomorpholin-4-yl)phényl]-(2R,4R)-4-hydroxypyrrolidine-1,2-dicarboxamide,
le 1-N-(4-chlorophényl)-2-N-[3-méthyl-4-(3-oxomorpholin-4-yl)-phényl]-(2R,4R)-4-hydroxypyrrolidine-1,2-dicarboxamide,
le 1-N-(4-chlorophényl)-2-N-[2-fluoro-4-(3-oxomorpholin-4-yl)-phényl]-(2R,4R)-4-hydroxypyrrolidine-1,2-dicarboxamide,
le 1-N-(4-chlorophényl)-2-N-[3-fluoro-4-(3-oxomorpholin-4-yl)-phényl]-(2R,3R)-3-hydroxypyrrolidine-1,2-dicarboxamide,
le 1-N-(4-chlorophényl)-2-N-[3-fluoro-4-(3-oxomorpholin-4-yl)-phényl]-(2R,3S)-3-hydroxypyrrolidine-1,2-dicarboxamide,
le 1-N-(4-chlorophényl)-2-N-[4-(3-oxomorpholin-4-yl)phényl]-(2R,4S)-4-hydroxypyrrolidine-1,2-dicarboxamide,
le 1-N-(4-chlorophényl)-2-N-[4-(3-oxomorpholin-4-yl)phényl]-(2S,4R)-4-hydroxypyrrolidine-1,2-dicarboxamide,
le 1-N-(4-chlorophényl)-2-N-[4-(3-oxomorpholin-4-yl)phényl]-3,4-dihydroxypyrrolidine-1,2-dicarboxamide,
le 1-N-(4-chlorophényl)-2-N-[4-(3-oxomorpholin-4-yl)phényl]-(2R,4S)-4-azidopyrrolidine-1,2-dicarboxamide,
le 1-N-(4-chlorophényl)-2-N-[4-(3-oxomorpholin-4-yl)phényl]-(2R,4S)-4-aminopyrrolidine-1,2-dicarboxamide,
le 1-N-(4-chlorophényl)-2-N-[4-(3-oxomorpholin-4-yl)phényl]-(2R,4R)-4-azidopyrrolidine-1,2-dicarboxamide,
le 1-N-(4-chlorophényl)-2-N-[4-(3-oxomorpholin-4-yl)phényl]-(2R,4R)-4-aminopyrrolidine-1,2-dicarboxamide,
le 1-N-(4-chlorophényl)-2-N-[4-(3-oxomorpholin-4-yl)phényl]-(2R,4S)-4-acétaminopyrrolidine-1,2-dicarboxamide,
le 1-N-(4-chlorophényl)-2-N-[4-(3-oxomorpholin-4-yl)phényl]-(2R,4R)-4-acétaminopyrrolidine-1,2-dicarboxamide,
le 1-N-(4-chlorophényl)-2-N-[4-(3-oxomorpholin-4-yl)phényl]-(2R,4S)-4-méthylsulfonylaminopyrrolidine-1,2-dicarboxamide,
le 1-N-(4-chlorophényl)-2-N-[4-(3-oxomorpholin-4-yl)phényl]-(2R,4R)-4-méthylsulfonylaminopyrrolidine-1,2-dicarboxamide,
le 1-N-(4-chlorophényl)-2-N-[4-(3-oxomorpholin-4-yl)phényl]-(2R,4R)-4-méthoxypyrrolidine-1,2-dicarboxamide,
le 1-N-(4-chlorophényl)-2-N-[4-(3-oxomorpholin-4-yl)phényl]-(2R,4R)-4-éthoxypyrrolidine-1,2-dicarboxamide,
le 1-N-(4-chlorophényl)-2-N-[4-(3-oxomorpholin-4-yl)phényl]-(2R,4R)-4-propoxypyrrolidine-1,2-dicarboxamide,
le 1-N-(4-chlorophényl)-2-N-[4-(3-oxomorpholin-4-yl)phényl]-(2R,4R)-4-allyloxypyrrolidine-1,2-dicarboxamide,
l'isobutyrate de (3R,5R)-1-(4-chlorophénylcarbamoyl)-5-[4-(3-oxomorpholin-4-yl)phénylcarbamoyl]pyrrolidin-3-yle,
le propionate de (3R,5R)-1-(4-chlorophénylcarbamoyl)-5-[4-(3-oxomorpholin-4-yl)phénylcarbamoyl]pyrrolidin-3-yle,
l'acétate de (3R,5R)-1-(4-chlorophénylcarbamoyl)-5-[4-(3-oxomorpholin-4-yl)phénylcarbamoyl]pyrrolidin-3-yle,
le 4-N-(4-chlorophényl)-5-N-[4-(3-oxomorpholin-4-yl)phényl]-1,3-dioxolane-4,5-dicarboxamide,
le 4-N-(4-chlorophényl)-5-N-[3-méthyl-4-(3-oxomorpholin-4-yl)-phényl]-1,3-dioxolane-4,5-dicarboxamide,
le 4-N-(4-chlorophényl)-5-N-[4-(3-oxomorpholin-4-yl)phényl]-1,3-dioxolane-2,2-diméthyl-4,5-dicarboxamide,
le 4-N-(4-chlorophényl)-5-N-[3-méthyl-4-(3-oxomorpholin-4-yl)-phényl]-1,3-dioxolane-2,2-diméthyl-4,5-dicarboxamide,
le 1-N-(4-chlorophényl)-2-N-[4-(3-oxomorpholin-4-yl)phényl]-1-BOC-pipérazine-1,2-dicarboxamide,
le 1-N-(4-chlorophényl)-2-N-[4-(3-oxomorpholin-4-yl)phényl]-pipérazine-1,2-dicarboxamide,
le 1-N-(4-chlorophényl)-2-N-[4-(3-oxomorpholin-4-yl)phényl]-1,3-oxazinane-3,4-dicarboxamide,
le 1-N-(4-chlorophényl)-2-N-[4-(3-oxomorpholin-4-yl)phényl]-(2R,4S)-4-éthynyl-4-hydroxypyrrolidine-1,2-dicarboxamide,
le 6-N-(4-chlorophényl)-7-N-[4-(3-oxomorpholin-4-yl)phényl]-4-oxa-6-azaspiro[2.4]heptane-6,7-dicarboxamide,
le 1-N-(4-chlorophényl)-2-N-[3-méthyl-4-(3-oxomorpholin-4-yl)-phényl]-(2R,4S)-4-acétaminopyrrolidine-1,2-dicarboxamide,
le 1-N-(4-chlorophényl)-2-N-[4-(3-oxomorpholin-4-yl)phényl]-(2R,4S)-4-butylsulfonylaminopyrrolidine-1,2-dicarboxamide,
le 1-N-(4-chlorophényl)-2-N-[4-(3-oxomorpholin-4-yl)phényl]-(R)-4-oxopyrrolidine-1,2-dicarboxamide,
le 1-N-(4-chlorophényl)-2-N-[3-méthyl-4-(3-oxomorpholin-4-yl)-phényl]-(2R,4S)-4-aminopyrrolidine-1,2-dicarboxamide,
le 1-N-(4-chlorophényl)-2-N-[3-méthyl-4-(3-oxomorpholin-4-yl)-phényl]-(S)-pyrrolidine-1,2-dicarboxamide,
le 1-N-(4-chlorophényl)-2-N-[2-fluoro-4-(3-oxomorpholin-4-yl)phényl]-(2R,4R)-4-hydroxypyrrolidine-1,2-dicarboxamide,
le N-[4-(3-oxomorpholin-4-yl)phényl]-(2R,4R)-1-[2-(4-chlorophényl)acétyl]-4-hydroxypyrrolidine-2-carboxamide,
le N-[4-(3-oxomorpholin-4-yl)phényl]-(2R,4R)-1-(4-chlorobenzoyl)-4-hydroxypyrrolidine-2-carboxamide,
le 1-N-(4-chlorophényl)-2-N-[3-méthyl-4-(3-oxomorpholin-4-yl)-phényl]-(2R,4R)-4-méthoxypyrrolidine-1,2-dicarboxamide,
le 1-N-(4-chlorophényl)-2-N-[2-fluoro-4-(3-oxomorpholin-4-yl)-phényl]-(2R,4R)-4-méthoxypyrrolidine-1,2-dicarboxamide,
le 1-N-(4-chlorophényl)-2-N-[4-(3-oxomorpholin-4-yl)phényl]-(2R,4S)-4-(2-méthylpropanoylamino)pyrrolidine-1,2-dicarboxamide,
le 1-N-(4-chlorophényl)-2-N-[3-méthyl-4-(3-oxomorpholin-4-yl)phényl]-(2R,4R)-4-éthoxypyrrolidine-1,2-dicarboxamide,
le 1-N-(4-chlorophényl)-2-N-[2-fluoro-4-(3-oxomorpholin-4-yl)-phényl]-(2R,4R)-4-éthoxypyrrolidine-1,2-dicarboxamide,
le 2-N-(4-chlorophényl)-1-N-[4-(3-oxomorpholin-4-yl)phényl]-(R)-pyrrolidine-1,2-dicarboxamide,
le 2-N-(4-chlorophényl)-1-N-[4-(3-oxomorpholin-4-yl)phényl]-(S)-pyrrolidine-1,2-dicarboxamide,
le N-(4-chlorophényl)-(R)-1-{2-[4-(3-oxomorpholin-4-yl)phényl]-acétyl}pyrrolidine-2-carboxamide,
le N-(4-chlorophényl)-(S)-1-{2-[4-(3-oxomorpholin-4-yl)phényl]-acétyl}pyrrolidine-2-carboxamide,
le N-(4-chlorophényl)-(2R,4R)-1-{2-[4-(3-oxomorpholin-4-yl)-phényl]acétyl}-4-méthoxypyrrolidine-2-carboxamide,
le N-(4-chlorophényl)-(2R,4S)-1-{2-[4-(3-oxomorpholin-4-yl)-phényl]acétyl}-4-méthoxypyrrolidine-2-carboxamide,
le N-(4-chlorophényl)-(2S,4R)-1-{2-[4-(3-oxomorpholin-4-yl)-phényl]acétyl}-4-méthoxypyrrolidine-2-carboxamide,
le 1-N-(4-chlorophényl)-2-N-[3-fluoro-4-(3-oxomorpholin-4-yl)-phényl]-(2R,4R)-4-éthoxypyrrolidine-1,2-dicarboxamide,
le 1-N-(4-chlorophényl)-2-N-[4-(3-oxomorpholin-4-yl)phényl]-(2R,4R)-4-(prop-2-ynyloxy)pyrrolidine-1,2-dicarboxamide,
le 1-N-(4-chlorophényl)-2-N-[4-(3-oxomorpholin-4-yl)phényl]-(2R,4R)-4-(but-2-ynyloxy)pyrrolidine-1,2-dicarboxamide,
le 1-N-(4-chlorophényl)-2-N-[4-(3-oxomorpholin-4-yl)phényl]-(2R,4R)-4-(2,3-dihydroxypropoxy)pyrrolidine-1,2-dicarboxamide,
le 1-N-(4-chlorophényl)-2-N-[4-(3-oxomorpholin-4-yl)phényl]-(2R,4R)-4-(2-hydroxy-3-pyrrolidin-1-ylpropoxy)pyrrolidine-1,2-dicarboxamide,
le 1-N-(4-chlorophényl)-2-N-[4-(3-oxomorpholin-4-yl)phényl]-(2R,4R)-4-(2-oxooxazolidin-5-ylméthoxy)pyrrolidine-1,2-dicarboxamide,
le 1-N-(4-chlorophényl)-2-N-[4-(3-oxomorpholin-4-yl)phényl]-(2R,4R)-4-(3-amino-2-hydroxypropoxy)pyrrolidine-1,2-dicarboxamide,
le 1-N-(4-chlorophényl)-2-N-[3-fluoro-4-(3-oxomorpholin-4-yl)-phényl]-(R)-2,5-dihydropyrrole-1,2-dicarboxamide,
le 1-N-(4-chlorophényl)-2-N-[2-fluoro-4-(3-oxomorpholin-4-yl)-phényl]-(R)-2,5-dihydropyrrole-1,2-dicarboxamide,
le 1-N-(4-chlorophényl)-2-N-[4-(3-oxomorpholin-4-yl)phényl]-(2S,3S)-3-hydroxypyrrolidine-1,2-dicarboxamide,
le 1-N-(4-chlorophényl)-2-N-[4-(3-oxomorpholin-4-yl)phényl]-(2S,4S)-4-hydroxypyrrolidine-1,2-dicarboxamide,
le 1-N-(4-chlorophényl)-2-N-[2-carboxy-4-(3-oxomorpholin-4-yl)-phényl]-(2R,4R)-3-hydroxypyrrolidine-1,2-dicarboxamide,
le 1-N-(4-chlorophényl)-2-N-[4-(3-oxomorpholin-4-yl)phényl]-(2R,3S,4R)-3,4-dihydroxypyrrolidine-1,2-dicarboxamide,
le 1-N-(4-chlorophényl)-2-N-[2-fluoro-4-(3-oxomorpholin-4-yl)-phényl]-(2R,4R)-4-allyloxypyrrolidine-1,2-dicarboxamide,
le 1-N-(4-chlorophényl)-2-N-[2-fluoro-4-(3-oxomorpholin-4-yl)-phényl]-(2R,4R)-4-(prop-2-ynyloxy)pyrrolidine-1,2-dicarboxamide,
le 1-N-(4-chlorophényl)-2-N-[2-fluoro-4-(3-oxomorpholin-4-yl)-phényl]-(2R,4S)-4-(prop-2-ynyloxy)pyrrolidine-1,2-dicarboxamide,
le 1-N-(4-chlorophényl)-2-N-[4-(3-oxomorpholin-4-yl)phényl]-(2R,4R)-4-(méthoxycarbonylméthoxy)pyrrolidine-1,2-dicarboxamide,
le 1-N-(4-chlorophényl)-2-N-[4-(3-oxomorpholin-4-yl)phényl]-(2R,4R)-4-(carboxyméthoxy)pyrrolidine-1,2-dicarboxamide,
le 1-N-(4-bromophényl)-2-N-[2-fluoro-4-(3-oxomorpholin-4-yl)-phényl]-(2R,4R)-4-méthoxypyrrolidine-1,2-dicarboxamide,
le 1-N-(4-chlorophényl)-2-N-[2-fluoro-4-(3-oxomorpholin-4-yl)-phényl]-(2R,4R)-4-(2,3-dihydroxypropoxy)pyrrolidine-1,2-dicarboxamide,
le 1-N-(4-chlorophényl)-2-N-[2-aminocarbonyl-4-(3-oxomorpholin-4-yl)phényl]-(2R,4R)-4-hydroxypyrrolidine-1,2-dicarboxamide, ESI 502 ;
le 1-N-(4-chlorophényl)-2-N-[4-(3-oxomorpholin-4-yl)phényl]-(2R,4R)-4-hydroxy-2-méthylpyrrolidine-1,2-dicarboxamide,
le 1-N-(4-chlorophényl)-3-N-[4-(3-oxomorpholin-4-yl)phényl]-pipéridine-1,3-dicarboxamide,
le 1-N-(4-chlorophényl)-3-N-[3-méthyl-4-(3-oxomorpholin-4-yl)-phényl]pipéridine-1,3-dicarboxamide;
le 1-N-(4-chlorophényl)-2-N-[4-(3-oxomorpholin-4-yl)phényl]-(2R,4R)-4-(2-méthoxyéthoxy)pyrrolidine-1,2-dicarboxamide,
le 1-N-(4-chlorophényl)-2-N-[2-méthyl-4-(3-oxomorpholin-4-yl)-phényl]-(2R,4R)-4-hydroxypyrrolidine-1,2-dicarboxamide,
le 1-N-(4-chlorophényl)-2-N-[4-(3-oxomorpholin-4-yl)phényl]-(2R,4R)-4-hydroxypyrrolidine-1,2-dicarboxamide,
le 1-N-(4-chlorophényl)-2-N-[2-(3-oxomorpholin-4-yl)phényl]-(2R,4R)-4-hydroxypyrrolidine-1,2-dicarboxamide,
et les solvats, sels et stéréoisomères pharmaceutiquement utilisables de ceux-ci, y compris des mélanges de ceux-ci en toutes proportions.

3. Procédé de préparation de composés de formule I selon la revendication 1 ou 2, et de solvats, sels et stéréoisomères pharmaceutiquement utilisables de ceux-ci, **caractérisé en ce que**
pour la préparation de composés de formule I dans laquelle
W désigne N et
G désigne NH,
un composé de formule II 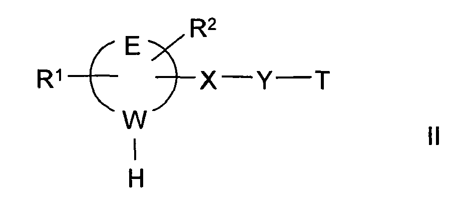 dans laquelle
R¹, R², E, X, Y et T ont la signification indiquée selon la revendication 1,
et W désigne N,
est réagi avec un composé de formule III
D-N=C=O III
dans laquelle
D a la signification indiquée selon la revendication 1,
et/ou
une base ou un acide de formule I est converti(e) en l'un de ses sels.

4. Composés de formule I selon la revendication 1 ou 2, comme inhibiteurs du facteur de coagulation Xa.

5. Composés de formule I selon la revendication 1 ou 2, comme inhibiteurs du facteur de coagulation VIIa.

6. Médicaments comprenant au moins un composé de formule I selon la revendication 1 ou 2, et/ou des solvats, sels et stéréoisomères pharmaceutiquement utilisables de ceux-ci, y compris des mélanges de ceux-ci en toutes proportions, et éventuellement des excipients et/ou adjuvants.

7. Médicaments comprenant au moins un composé de formule I selon la revendication 1 ou 2, et/ou des solvats et stéréoisomères pharmaceutiquement utilisables de ceux-ci, y compris des mélanges de ceux-ci en toutes proportions, et au moins un autre composé actif médicamenteux.

8. Utilisation de composés selon la revendication 1 ou 2 et/ou de sels et solvats physiologiquement acceptables de ceux-ci, pour la préparation d'un médicament destiné au traitement des thromboses, de l'infarctus du myocarde, de l'artériosclérose, des inflammations, de l'apoplexie, de l'angine de poitrine, de la resténose après angioplastie, de la boiterie intermittente, de la migraine, des tumeurs, des maladies tumorales et/ou des métastases tumorales.

9. Ensemble (kit) constitué de conditionnements séparés
(a) d'une quantité efficace d'un composé de formule I selon la revendication 1 ou 2 et/ou de solvats, sels et stéréoisomères pharmaceutiquement utilisables de ceux-ci, y compris des mélanges de ceux-ci en toutes proportions,
et
(b) d'une quantité efficace d'un autre composé actif médicamenteux.

10. Utilisation de composés de formule I selon la revendication 1 ou 2 et/ou de solvats, sels et stéréoisomères pharmaceutiquement utilisables de ceux-ci, y compris des mélanges de ceux-ci en toutes proportions,
pour la préparation d'un médicament destiné au traitement des thromboses, de l'infarctus du myocarde, de l'artériosclérose, des inflammations, de l'apoplexie, de l'angine de poitrine, de la resténose après angioplastie, de la boiterie intermittente, de la migraine, des tumeurs, des maladies tumorales et/ou des métastases tumorales, en association avec au moins un autre ingrédient actif médicamenteux.

11. Composés intermédiaires, choisis parmi le groupe constitué par
le *N*-[4-(3-oxomorpholin-4-yl)phényl]-(S)-pyrrolidine-2-carboxamide,
le *N*-[4-(3-oxomorpholin-4-yl)phényl]-(R)-pyrrolidine-2-carboxamide,
le *N*-[4-(3-oxomorpholin-4-yl)phényl]-(2R,4R)-4-hydroxypyrrolidine-2-carboxamide,
le *N*-[4-(3-oxomorpholin-4-yl)phényl]-4-hydroxypyrrolidine-2-carboxamide,
le *N*-[4-(3-oxomorpholin-4-yl)phényl]-(R)-4,4-diméthoxypyrrolidine-2-carboxamide,
le *N*-[4-(3-oxomorpholin-4-yl)phényl]-(2R,4R)-4-méthoxypyrrolidine-2-carboxamide,
et des isomères et sels de ceux-ci.

12. Médicaments selon la revendication 7, comprenant du 1-N-(4-chlorophényl)-2-N-[4-(3-oxomorpholin-4-yl)phényl]-(2R,4R)-4-hydroxypyrrolidine-1,2-dicarboxamide et/ou des solvats, sels et stéréoisomères pharmaceutiquement utilisables de celui-ci, y compris des mélanges de ceux-ci en toutes proportions, et de l'aspirine.

13. Utilisation selon la revendication 10, comprenant du 1-N-(4-chloro-phényl)-2-N-[4-(3-oxomorpholin-4-yl)phényl]-(2R,4R)-4-hydroxypyrrolidine-1,2-dicarboxamide et/ou des solvats, sels et stéréoisomères pharmaceutiquement utilisables de celui-ci, y compris des mélanges de ceux-ci en toutes proportions, en association avec de l'aspirine.
